# EUROPÄISCHE PATENTSCHRIFT

(11) **EP 1 866 417 B1**
(45) Veröffentlichungstag und Bekanntmachung des Hinweises auf die Patenterteilung: **26.04.2017**
(21) Anmeldenummer: 06725200.7
(22) Anmeldetag: 21.03.2006
(51) Int. Cl.: C12N 15/82, C12P 7/64

(54) **VERFAHREN ZUR HERSTELLUNG VON MEHRFACH UNGESÄTTIGTEN C20- UND C22-FETTSÄUREN MIT MINDESTENS VIER DOPPELBINDUNGEN IN TRANSGENEN PFLANZEN**
METHOD FOR PRODUCING POLYUNSATURATED C20 AND C22FATTY ACIDS WITH AT LEAST FOUR DOUBLE BONDS IN TRANSGENIC PLANTS
PROCEDE DE PRODUCTION D'ACIDES GRAS C20 ET C22 POLYINSATURES AVEC AU MOINS QUATRE LIAISONS DOUBLES DANS DES PLANTES TRANSGENIQUES

(30) Priorität: 22.03.2005 DE 102005013779
(43) Veröffentlichungstag der Anmeldung: 19.12.2007
(73) Patentinhaber: BASF Plant Science GmbH, 67056 Ludwigshafen (DE)
(72) Erfinder: CIRPUS, Petra, 68163 Mannheim (DE); BAUER, Jörg, 67061 Ludwigshafen (DE)
(74) Vertreter: BASF IP Association
(86) Internationale Anmeldenummer: PCT/EP2006/060913
(87) Internationale Veröffentlichungsnummer: WO 2006/100241

(56) Entgegenhaltungen:
- WO-A-02/26946
- WO-A-03/012092
- WO-A-03/064638
- WO-A-03/093482
- WO-A-2005/007845
- WO-A-2005/012316
- ZANK THORSTEN K ET AL: "Cloning and functional characterisation of an enzyme involved in the elongation of DELTA6-polyunsaturated fatty acids from the moss Physcomitrella patens" PLANT JOURNAL, Bd. 31, Nr. 3, August 2002 (2002-08), Seiten 255-268, XP002393086 ISSN: 0960-7412
- DATABASE UniProt Felta-12-Desaturase from Saprolegnia diclina 4. Juli 2004 (2004-07-04), PEREIRA S.ET AL: "Q6UB74_9STRA" XP002393088 gefunden im EBI Database accession no. Q6UB74 & PEREIRA S L ET AL: "A novel omega3-fatty acid desaturase involved in the biosynthesis of eicosapentaenoic acid" BIOCHEMICAL JOURNAL, THE BIOCHEMICAL SOCIETY, LONDON, GB, Bd. 378, Nr. 2, 2004, Seiten 665-671, XP002334258 ISSN: 0264-6021

## Beschreibung

Die vorliegende Erfindung betrifft allgemein die Herstellung von mehrfach ungesättigten Fettsäuren in transgenen Organismen, in dem Nukleinsäuren den Organismus eingebracht werden, die für Polypeptide mit Δ-6-Desaturase-, Δ-6-Elongase-, eine Δ-5-Desaturase-, Δ-5-Elongase-, Δ-4-Desaturase-, Δ-12-Desaturase- und/oder ω-3-Desaturaseaktivität codieren. Vorteilhaft stammen diese Desaturasen und Elongasen aus Phytophtora sojae.

Die Erfindung betrifft weiterhin die Nukleinsäuresequenzen, Nukleinsäurekonstrukte, Vektoren und Organismen enthaltend die erfindungsgemäßen Nukleinsäuresequenzen, Vektoren enthaltend die Nukleinsäuresequenzen und/oder die Nukleinsäurekonstrukte sowie transgene Pflanzen enthalten die vorgenannten Nukleinsäuresequenzen, Nukleinsäurekonstrukte und/oder Vektoren.

Ein weiterer Teil der Erfindung betrifft Fettsäurezusammensetzungen hergestellt nach dem hier beschriebenen Verfahren und deren Verwendung.

Fettsäuren und Triacylglyceride haben eine Vielzahl von Anwendungen in der Lebensmittelindustrie, der Tierernährung, der Kosmetik und im Pharmabereich. Je nachdem, ob es sich um freie gesättigte und ungesättigte Fettsäuren oder um Triacylglyceride mit einem erhöhten Gehalt an gesättigten oder ungesättigten Fettsäuren handelt, sind sie für die unterschiedlichsten Anwendungen geeignet. Mehrfachungesättigte Fettsäuren wie Linol- und Linolensäure sind für Säugetiere essentiell, da sie nicht von diesen selbst hergestellt werden können. Deshalb stellen mehrfach ungesättigte ω-3-Fettsäuren und ω-6-Fettsäuren einen wichtigen Bestandteil der tierischen und menschlichen Nahrung dar.

Mehrfach ungesättigte langkettige ω-3-Fettsäuren wie Eicosapentaensäure (= EPA, C20:5^{Δ5,8,11,14,17}) oder Docosahexaensäure (= DHA, C22:6^{Δ4,7,10,13,16,19}) sind wichtige Komponenten der menschlichen Ernährung aufgrund ihrer verschiedenen Rollen in der Gesundheit, die Aspekte wie die Entwicklung des kindlichen Gehirns, der Funktionalität des Auges, der Synthese von Hormonen und anderer Signalstoffe, sowie die Vorbeugung von Herz-Kreislauf-Beschwerden, Krebs und Diabetes umfassen (Poulos, A Lipids 30:1-14, 1995; Horrocks, LA und Yeo YK Pharmacol Res 40:211-225, 1999). Es besteht aus diesem Grund ein Bedarf an der Produktion mehrfach ungesättigter langkettiger Fettsäuren.

Aufgrund der Heute üblichen Zusammensetzung der menschlichen Nahrung ist ein Zusatz von mehrfach ungesättigten ω-3-Fettsäuren, die bevorzugt in Fischölen vorkommen, zur Nahrung besonders wichtig. So werden beispielsweise mehrfach ungesättigte Fettsäuren wie Docosahexaensäure (= DHA, C22:6^{Δ4,7,10,13,16,19}) oder Eisosapentaensäure (= EPA, C20:5^{Δ5,8,11,14,17}) Babynahrung zur Erhöhung des Nährwertes zugesetzt. Der ungesättigten Fettsäure DHA wird dabei ein positiver Effekt auf die Entwicklung und Aufrechterhaltung von Gehirnfunktionen zugeschrieben.

Im folgenden werden mehrfach ungesättigte Fettsäuren als PUFA, PUFAs, LCPUFA oder LCPUFAs bezeichnet (**p**oly **u**nsaturated **f**atty **a**cids, **PUFA,** mehrfach ungesättigte Fettsäuren; **l**ong **c**hain **p**oly **u**nsaturated **f**atty **a**cids, **LCPUFA,** langkettige mehrfach ungesättigte Fettsäuren).

Hauptsächlich werden die verschiedenen Fettsäuren und Triglyceride aus Mikroorganismen wie Mortierella oder Schizochytrium oder aus Öl-produzierenden Pflanzen wie Soja, Raps, Algen wie Crypthecodinium oder Phaeodactylum und weiteren gewonnen, wobei sie in der Regel in Form ihrer Triacylglyceride (= Triglyceride = Triglycerole) anfallen. Sie können aber auch aus Tieren wie z.B. Fischen gewonnen werden. Die freien Fettsäuren werden vorteilhaft durch Verseifung hergestellt. Sehr langkettige mehrfach ungesättigte Fettsäuren wie DHA, EPA, Arachidonsäure (= ARA, C20:4^{Δ5,8,11,14}), Dihomo-γ-linolensäure (C20:3^{Δ8,11,14}) oder Docosapentaensäure (DPA, C22:5^{Δ7,10,13,16,19}) werden in Ölfruchtpflanzen wie Raps, Soja, Sonnenblume, Färbersaflor nicht synthetisiert. Übliche natürliche Quellen für diese Fettsäuren sind Fische wie Hering, Lachs, Sardine, Goldbarsch, Aal, Karpfen, Forelle, Heilbutt, Makrele, Zander oder Thunfisch oder Algen.

Je nach Anwendungszweck werden Öle mit gesättigten oder ungesättigten Fettsäuren bevorzugt. So werden z.B. in der humanen Ernährung Lipide mit ungesättigten Fettsäuren speziell mehrfach ungesättigten Fettsäuren bevorzugt. Den mehrfach ungesättigten ω-3-Fettsäuren wird dabei ein positiver Effekt auf den Cholesterinspiegel im Blut und damit auf die Möglichkeit der Prävention einer Herzerkrankung zugeschrieben. Durch Zugabe dieser ω-3-Fettsäuren zur Nahrung kann das Risiko einer Herzerkrankung, eines Schlaganfalls oder von Bluthochdruck deutlich verringert werden. Auch entzündliche speziell chronisch entzündliche Prozesse im Rahmen immunologischer Erkrankungen wie rheumatroider Arthritis lassen sich durch ω-3-Fettsäuren positiv beeinflussen. Sie werden deshalb Lebensmitteln speziell diätischen Lebensmitteln zugegeben oder finden in Medikamenten Anwendung. ω-6-Fettsäuren wie Arachidonsäure haben bei diesen rheumatischen Erkrankungen aufgrund unserer üblichen Nahrungsmittelzusammensetzung eher einen negativen Effekt auf diese Krankheiten.

ω-3- und ω-6-Fettsäuren sind Vorläufer von Gewebshormonen, den sogenannten Eicosanoiden wie den Prostaglandinen, die sich von der Dihomo-γ-linolensäure, der Arachidonsäure und der Eicosapentaensäure ableiten, den Thromoxanen und Leukotrienen, die sich von der Arachidonsäure und der Eicosapentaensäure ableiten. Eicosanoide (sog. PG₂-Serie), die aus ω-6-Fettsäuren gebildet werden fördern in der Regel Entzündungsreaktionen, während Eicosanoide (sog. PG₃-Serie) aus ω-3-Fettsäuren geringe oder keine entzündungsfördernde Wirkung haben.

Aufgrund ihrer positiven Eigenschaften hat es in der Vergangenheit nicht an Ansätzen gefehlt, Gene, die an der Synthese von Fettsäuren bzw. Triglyceriden beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine Δ-15-Desaturase in WO 94/11516 wird eine Δ-12-Desaturase beansprucht. Weitere Desaturasen werden beispielsweise in EP-A-0 550 162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 oder Huang et al., Lipids 34, 1999: 649-659 beschrieben. Die biochemische Charakterisierung der verschiedenen Desaturasen ist jedoch bisher nur unzureichend erfolgt, da die Enzyme als membrangebundene Proteine nur sehr schwer zu isolieren und zu charakterisieren sind (McKeon et al., Methods in Enzymol. 71, 1981: 12141-12147, Wang et al., Plant Physiol. Biochem., 26, 1988: 777-792). In der Regel erfolgt die Charakterisierung membrangebundener Desaturasen durch Einbringung in einen geeigneten Organismus, der anschließend auf Enzymaktivität mittels Edukt- und Produktanalyse untersucht wird. Δ-6-Desaturasen werden in WO 93/06712, US 5,614,393, US5614393, WO 96/21022, WO00/21557 und WO 99/27111 beschrieben und auch die Anwendung zur Produktion in transgenen Organismen beschrieben wie in WO98/46763 WO98/46764, WO9846765. Dabei wird auch die Expression verschiedener Desaturasen wie in WO99/64616 oder WO98/46776 und Bildung polyungesättigter Fettsäuren beschrieben und beansprucht. Bzgl. der Effektivität der Expression von Desaturasen und ihren Einfluss auf die Bildung polyungesättigter Fettsäuren ist anzumerken, dass durch Expression einer einzelnen Desaturase wie bisher beschrieben lediglich geringe Gehalte an ungesättigten Fettsäuren/Lipiden wie z.B. γ-Linolensäure und Stearidonsäure erreicht wurden. Weiterhin wurde in der Regel ein Gemisch aus ω-3- und ω-6-Fettsäuren erhalten.

Auch wurden in der Vergangenheit wurden zahlreiche Versuche unternommen, Elongase Gene zu erhalten. Millar and Kunst, 1997 (Plant Journal 12:121-131) und Millar et al. 1999, (Plant Cell 11:825-838) beschreiben die Charakterisierung von pflanzlichen Elongasen zur Synthese von einfachungesättigten langkettigen Fettsäuren (C22:1) bzw. zur Synthese von sehr langkettigen Fettsäuren für die Wachsbildung in Pflanzen (C₂₈-C₃₂). Beschreibungen zur Synthese von Arachidonsäure und EPA finden sich beispielsweise in WO0159128, WO0012720, WO02077213 und WO0208401. Die Synthese von mehrfachungesättigter C24 Fettsäuren ist beispielsweise in Tvrdik et al 2000, JCB 149:707-717 oder WO0244320 beschrieben.

Besonders geeignete Mikroorganismen zur Herstellung von PUFAs sind Mikroorganismen wie Mikroalgen wie Phaeodactylum tricornutum, Porphiridium-Arten, Thraustochytrien-Arten, Schizochytrien-Arten oder Crypthecodinium-Arten, Ciliaten, wie Stylonychia oder Colpidium, Pilze, wie Mortierella, Entomophthora oder Mucor und/oder Moosen wie Physcomitrella, Ceratodon und Marchantia (R. Vazhappilly & F. Chen (1998) Botanica Marina 41: 553-558; K. Totani & K. Oba (1987) Lipids 22: 1060-1062; M. Akimoto et al. (1998) Appl. Biochemistry and Biotechnology 73: 269-278). Durch Stammselektion ist eine Anzahl von Mutantenstämmen der entsprechenden Mikroorganismen entwickelt worden, die eine Reihe wünschenswerter Verbindungen, einschließlich PUFAs, produzieren. Die Mutation und Selektion von Stämmen mit verbesserter Produktion eines bestimmten Moleküls wie den mehrfach ungesättigten Fettsäuren ist jedoch ein zeitraubendes und schwieriges Verfahren. Deshalb werden, wann immer möglich wie oben beschrieben gentechnologische Verfahren bevorzugt. Mit Hilfe der vorgenannten Mikroorganismen lassen sich jedoch nur begrenzte Mengen der gewünschten mehrfach ungesättigten Fettsäuren wie DPA, EPA oder ARA herstellen. Wobei diese in der Regel je nach verwendeten Mikroorganismus als Fettsäuregemische aus beispielsweise EPA, DPA und ARA anfallen.

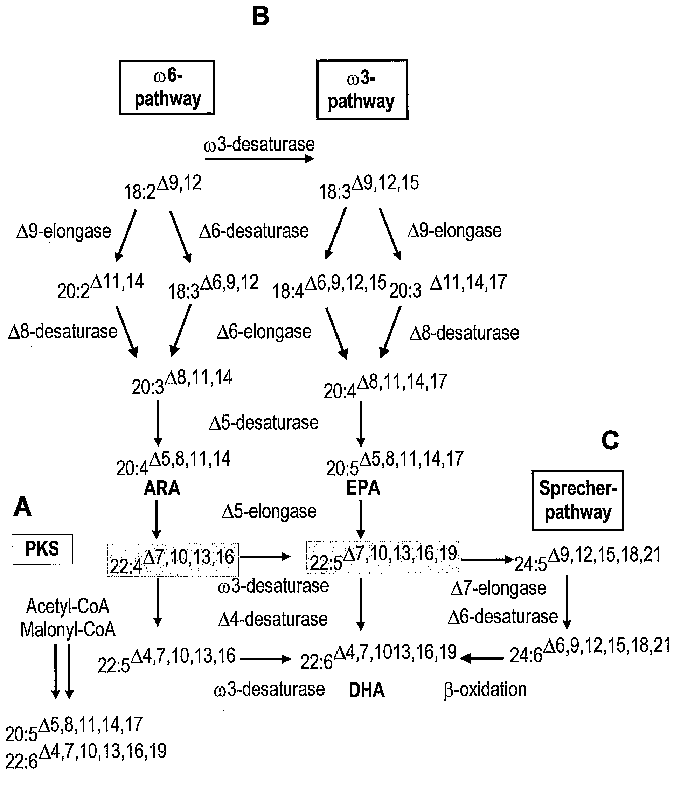

Für die Synthese von Arachidonsäure, Eicosapentaensäure (EPA) und Docosahexaensäure (DHA) werden verschiedene Synthesewege diskutiert (Figur. 1). So erfolgt die Produktion von EPA bzw. DHA in marinen Bakterien wie Vibrio sp. oder Shewanella sp. nach dem Polyketid-Weg (Yu, R. et al. Lipids 35:1061-1064, 2000; Takeyama, H. et al. Microbiology 143:2725-2731, 1997).

Ein alternative Strategie verläuft über die wechselnde Aktivität von Desaturasen und Elongasen (Zank, T.K. et al. Plant Journal 31:255-268, 2002; Sakuradani, E. et al. Gene 238:445-453, 1999). Eine Modifikation des beschriebenen Weges über Δ6-Desaturase, Δ6-Elongase, Δ5-Desaturase, Δ5-Elongase, Δ4-Desaturase ist der Sprecher-Syntheseweg (Sprecher 2000, Biochim. Biophys. Acta 1486:219-231) in Säugetieren. Anstelle der Δ4-Desaturierung erfolgt hier ein weiterer Elongationsschritt auf C₂₄, eine weitere Δ6-Desaturierung und abschliessend eine β-Oxidation auf die C₂₂-Kettenlänge. Für die Herstellung in Pflanzen und Mikroorganismen ist der sogenannte Sprecher-Syntheseweg (siehe Figur 1) allerdings nicht geeignet, da die Regulationsmechanismen nicht bekannt sind.

Für die Herstellung längerkettiger mehrfach ungesättigter Fettsäuren wie Arachidonsäure, EPA oder besonders DHA (C22:6 n-3) sind, wie Figur 1 zu entnehmen ist, neben den Desaturasen auch Elongasen erforderlich, die ungesättigte Fettsäuren mit Doppelbindungen beispielsweise in delta-9-, delta-6- oder delta-5-Postion um mindestens zwei Kohlenstoffatome verlängern. Ihrer Funktion nach werden dabei zwei verschiedene Elongasetypen unterschieden. Die Typ I-Elongasen, die im Tierreich weit verbreitet sind, haben nur eine geringe Substratspezifität, das heißt sie verlängern eine Reihe verschiedener ungestättigter Fettsäuren. Typ II-Elongasen zeichnen sich durch eine weit höhere Substratspezifität aus. Durch sie werden nur wenige Fettsäuren mit Doppelbindungen in spezifischen Positionen umgesetzt.

In WO 2005/012316 werden einige der vorgenannten Desaturasen und Elongasen beschrieben. Speziell werden in WO 2005/012316 erste Typ II-Elongasen offenbart, die spezifisch Fettsäuren mit einer Doppelbindung in delta-5-Position elongieren.

Die polyungesättigten Fettsäuren können entsprechend ihrem Desaturierungsmuster in zwei große Klassen, in ω-6- oder ω-3-Fettsäuren eingeteilt werden, die metabolisch und funktionell unterschiedlich Aktivitäten haben (Fig. 1).

Als Ausgangsprodukt für den ω-6-Stoffwechselweg fungiert die Fettsäure Linolsäure (18:2^{Δ9,12}), während der ω-3-Weg über Linolensäure (18:3^{Δ9,12,15}) abläuft. Linolensäure wird dabei durch Aktivität einer ω-3-Desaturase gebildet (Tocher et al. 1998, Prog. Lipid Res. 37, 73-117 ; Domergue et al. 2002, Eur. J. Biochem. 269, 4105-4113).

Säugetiere und damit auch der Mensch verfügen über keine entsprechende Desaturaseaktivität (Δ-12- und ω-3-Desaturase) und müssen diese Fettsäuren (essentielle Fettsäuren) über die Nahrung aufnehmen. Über die Abfolge von Desaturase- und Elongase-Reaktionen werden dann aus diesen Vorstufen die physiologisch wichtigen polyungesättigten Fettsäuren Arachidonsäure (= ARA, 20:4^{Δ5,8,11,14}), eine ω-6-Fettsäure und die beiden ω-3-Fettsäuren Eicosapentaen- (= EPA, 20:5^{Δ5,8,11,14,17}) und Docosahexaensäure (DHA, 22:6^{Δ4,7,10,13,17,19}) synthetisiert. Die Applikation von ω-3-Fettsäuren zeigt dabei die wie oben beschrieben therapeutische Wirkung bei der Behandlung von Herz-Kreislaufkrankheiten (Shimikawa 2001, World Rev. Nutr. Diet. 88, 100-108), Entzündungen (Calder 2002, Proc. Nutr. Soc. 61, 345-358) und Arthridis (Cleland und James 2000, J. Rheumatol. 27, 2305-2307).

Höhere Pflanzen enthalten mehrfach ungesättigte Fettsäuren wie Linolsäure (C18:2) und Linolensäure (C18:3). ARA, EPA und DHA kommen im Samenöl höherer Pflanzen gar nicht oder nur in Spuren vor (E. Ucciani: Nouveau Dictionnaire des Huiles Vegetales. Technique & Documentation - Lavoisier, 1995. ISBN: 2-7430-0009-0). Es wäre jedoch vorteilhaft, in höheren Pflanzen, bevorzugt in Ölsaaten wie Raps, Lein, Sonnenblume und Soja, LCPUFAs herzustellen, da auf diese Weise große Mengen qualitativ hochwertiger LCPUFAs für die Lebensmittelindustrie, die Tierernährung und für pharmazeutische Zwecke kostengünstig gewonnen werden können. Hierzu müssen vorteilhaft über gentechnische Methoden die Gene kodierend für Enzyme der Biosynthese von LCPUFAs in Ölsaaten eingeführt und exprimiert werden. WO 2005/012316 ist ein derartiger Ansatz zu entnehmen. Von Nachteil bei dem in WO 2005/012316 beschriebenen Weg ist jedoch noch, dass die Ausbeute an den gewünschten Fettsäuren für eine technische Nutzung noch zu gering ist. Außerdem entstehen neben den gewünschten Fettsäuren wie ARA, EPA oder DHA noch durch unerwünschte Nebenreaktionen Fettsäuren, die die Ausbeute weiter veringern und das entstehende Produkt verunreinigen.

Ein vorteilhaftes Verfahren zur Herstellung mehrfach ungesättigter Fettsäuren sollte deshalb möglichst viele der folgenden Eigenschaften auf sich vereinigen:
- hohe Spezifität der für die Herstellung von mehrfach ungesättigten Fettsäuren verwendeten Desaturasen und Elongasen,
- hohe Syntheseleistung der verwendeten Desaturasen und Elongasen,
- Synthese möglichst nur einer mehrfach ungesättigten Fettsäuren, wie ARA, EPA oder DHA,
- hohe Ausbeute an mehrfach ungesättigten Fettsäuren wie ARA, EPA oder DHA oder deren Mischungen
- möglichst keine oder nur sehr geringe Mengen an unerwünschten Nebenprodukten,
- keine Herstellung unnatürlicher Fettsäuren, das heißt von Fettsäuren, die nicht in der Natur vorkommen,
- Synthese der mehrfach ungesättigten Fettsäuren vorteilhaft nur in den Triglyceriden.

Um eine Anreicherung der Nahrung und des Futters mit diesen mehrfach ungesättigten Fettsäuren zu ermöglichen, besteht daher ein großer Bedarf an einem einfachen, kostengünstigen Verfahren zur Herstellung dieser mehrfach ungesättigten Fettsäuren speziell in eukaryontischen Systemen.

Es bestand daher die Aufgabe ein einfaches, kostengünstiges Verfahren zu entwickeln, das möglichst viele der oben genannten vorteilhaften Eigenschaften aufweist. Aufgabe der Erfindung war ferner, im Zusammenhang mit der Herstellung mehrfach ungesättigter Fettsäuren nützliche Nukleinsäuren, Proteine, Genkonstrukte, Vektoren und transgene Organismen, vorzugsweise Pflanzen und deren Teile, anzugeben.

Die Aufgabe wird gelöst durch die Gegenstände der Patentansprüche.

Ferner wird ein Verfahren angegeben zur Herstellung von mehrfach ungesättigten C₂₀-oder C₂₂-Fettsäuren mit mindestens vier Doppelbindungen in transgenen Pflanzen mit einem Gehalt von mindestens 15 Gew.-% bezogen auf den Gesamttriglyceridgehalt der transgenen Pflanze, dadurch gekennzeichnet, dass es folgende Verfahrensschritte umfasst:
a) Einbringen eines Nukleinsäurekonstrukts in die transgene Pflanze, das Nukleinsäuresequenzen enthält, die für eine Δ-6-Desaturase, eine Δ-6-Elongase und eine Δ-5-Desaturase codiert, oder
b) Einbringen eines Nukleinsäurekonstrukts in die transgene Pflanze, das Nukleinsäuresequenzen enthält, die für eine Δ-6-Desaturase, eine Δ-6-Elongase, eine Δ-5-Desaturase, eine Δ-12-Desaturase und ω-3-Desaturase codiert, oder
c) Einbringen eines Nukleinsäurekonstrukts in die transgene Pflanze, das Nukleinsäuresequenzen enthält, die für eine Δ-6-Desaturase, eine Δ-6-Elongase, eine Δ-5-Desaturase, eine Δ-5-Elongase und Δ-4-Desaturase codiert, oder
d) Einbringen eines Nukleinsäurekonstrukts in die transgene Pflanze, das Nukleinsäuresequenzen enthält, die für eine Δ-6-Desaturase, eine Δ-6-Elongase, eine Δ-5-Desaturase, eine Δ-5-Elongase, Δ-4-Desaturase, eine Δ-12-Desaturase und ω-3-Desaturase codiert, und
e) Gewinnung der Öle oder Lipide aus den Pflanzen, gelöst.

Vorteilhaft enthalten die im Verfahren hergestellten mehrfach ungesättigten C₂₀- oder C₂₂-Fettsäuren mindestens vier, fünf oder sechs Doppelbindungen. Besonders vorteilhaft enthalten die Fettsäuren fünf oder sechs Doppelbindungen. Vorteilhaft werden gesättigte Fettsäuren mit den im Verfahren verwendeten Nukleinsäuren wenig oder gar nicht umgesetzt. Unter wenig ist zu verstehen, das im Vergleich zu mehrfach ungesättigten Fettsäuren die gesättigten Fettsäuren mit weniger als 5 % der Aktivität, vorteilhaft weniger als 3 %, besonders vorteilhaft mit weniger als 2 %, ganz besonders bevorzugt mit weniger als 1; 0,5; 0,25 oder 0,125 % umgesetzt werden. Diese hergestellten Fettsäuren können als einziges Produkt im Verfahren hergestellt werden oder in einem Fettsäuregemisch vorliegen.

Bei den im Verfahren verwendeten Nukleinsäuresequenzen handelt es sich um isolierte Nukleinsäuresequenzen, die für Polypeptide mit Δ-6-Desaturase-, Δ-6-Elongase-, Δ-5-Desaturase-, Δ-5-Elongase-, Δ-4-Desaturase-, Δ-12-Desaturase-und/oder ω-3-Desaturaseaktivität codieren.

Vorteilhaft werden im Verfahren Nukleinsäuresequenzen, die für Polypeptide mit Δ-6-Desaturase-, Δ-6-Elongase-, Δ-5-Desaturase-, Δ-5-Elongase-, Δ-4-Desaturase-, Δ-12-Desaturase- und/oder ω-3-Desaturaseaktivität codieren, verwendet ausgewählt aus der Gruppe bestehend aus:
SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25.

Besonders vorteilhaft handelt es sich bei den im Verfahren hergestellten mehrfach ungesättigten C₂₀- oder C₂₂-Fettsäuren mit mindestens vier Doppelbindungen um die Fettsäuren Arachidonsäure (= ARA), Eicosapentaensäure (= EPA) oder Docosahexaensäure (= DHA). In einer vorteilhaften Ausführungsform des Verfahrens handelts es sich bei der mehrfach ungesättigten C₂₀- oder C₂₂-Fettsäuren mit mindestens vier Doppelbindungen um Arachidonsäure handelt. In einer anderen vorteilhaften Ausführungsform werden als mehrfach ungesättigten C₂₀- oder C₂₂-Fettsäuren mit mindestens vier Doppelbindungen Eicosapentaensäure oder Docosahexaensäure oder deren Mischungen hergestellt. Auch Mischungen von ARA, EPA und DHA sind nach dem Verfahren herstellbar.

Vorteilhaft werden im Verfahren Arachidonsäure oder die Eicosapentaensäure mit einem Gehalt von mindestens 15, 16, 17, 18, 19 oder 20 Gew.-%, bevorzugt von 25, 30, 35 oder 40 Gew.-%, besonders bevorzugt von 45, 50, 55 oder 60 Gew.-% bezogen auf den Gesamttriglyceridgehalt in der transgenen Pflanze hergestellt.

In einer anderen vorteilhaften Ausführungsform des Verfahrens wird Docosahexaensäure mit einem Gehalt von mindestens 4, 5 oder 6 Gew.-%, vorteilhaft mit einem Gehalt von 7, 8, 9 oder 10 Gew.-% bezogen auf den Gesamttriglyceridgehalt in der transgenen Pflanze hergestellt.

Besonders vorteilhaft sollten im Verfahren in den Triglyceriden weniger als 0,5; 0,4; 0,3; 0,2 oder 0,1 Gew.-%, vorteilhaft weniger als 0,09; 0,08; 0,07; 0,06 oder 0,05 Gew.-%, besonders vorteilhaft weniger als 0,04; 0,03; 0,02 oder 0,01 Gew.-% bezogen auf den Gesamtfettsäuregehalt der Triglyceride einer mehrfach ungesättigten Fettsäure ausgewählt aus der Gruppe der C22:4^{Δ7,10,13,16}-, C22:5^{Δ4,7,10,13,16}- oder C22:5^{Δ7,10,13,16,19}-Fettsäure vorhanden sein.

Die im Verfahren hergestellten mehrfach ungesättigten Fettsäuren sind vorteilhaft in Triacylglyceriden gebunden, können aber auch als freie Fettsäuren oder aber gebunden in Form anderer Fettsäureester in den Organismen vorkommen. Dabei können sie als "Reinprodukte" oder aber vorteilhaft in Form von Mischungen verschiedener Fettsäuren oder Mischungen unterschiedlicher Glyceride vorliegen.

Im Verfahren werden vorteilhaft Fettsäureester mit mehrfach ungesättigten C₂₀- und/oder C₂₂-Fettsäuremolekülen mit mindestens vier Doppelbindungen im Fettsäureester, vorteilhaft mit mindestens fünf oder sechs Doppelbindungen im Fettsäureester hergestellt. Dies führt zur Synthese von ω-3-Eicosatetraensäure (= ETA, C20:4 ^{Δ5,8,11,14}), Arachidonsäure (ARA, C20:4^{Δ5,8,11,14}), Eicosapentaensäure (EPA, C20:5^{Δ5,8,11,14,17}), ω-6-Docosapentaensäure (C22:5^{Δ4,7,10,13,16}), ω-6-Docosatetraensäure (C22:4^{Δ7,10,13,16}), ω-3-Docosapentaensäure (= DPA, C22:5^{Δ7,10,13,16,19}), Docosahexaensäure (= DHA, C22:6^{Δ4,7,10,13,16,19}) oder deren Mischungen, bevorzugt werden ARA, EPA und/oder DHA hergestellt. In einer besonders bevorzugten Ausführungsform des Verfahrens werden ω-6-Fettsäuren vorteilhaft Arachidonsäure hergestellt. In einer anderen besonders bevorzugten Ausführungsform werden, ω-3-Fettsäuren wie EPA und/oder DHA hergestellt.

Besonders vorteilhaft werden im Verfahren, wie oben beschrieben, mehrfach ungesättigte Fettsäuren ausgewählt aus der Gruppe ω-6-Docosatetraensäure C22:4^{Δ7,10,13,16}, ω-6-Docosapentaensäure C22:5^{Δ4,7,10,13,16} oder ω-3-Docosapentaensäure C22:5^{Δ7,10,13,16,19} in den Triglyceriden mit weniger als 0,5 Gew.-% bezogen auf den Gesamtfettsäuregehalt der Triglyceride hergestellt.

Die Fettsäureester mit mehrfach ungesättigten C₂₀- und/oder C₂₂-Fettsäuremolekülen können aus den Organismen, die für die Herstellung der Fettsäureester verwendet wurden, in Form eines Öls oder Lipids beispielsweise in Form von Verbindungen wie Sphingolipide, Phosphoglyceride, Lipide, Glycolipide wie Glycosphingolipide, Phospholipide wie Phosphatidylethanolamin, Phosphatidylcholin, Phosphatidylserin, Phosphatidylglycerol, Phosphatidylinositol oder Diphosphatidylglycerol, Monoacylglyceride, Diacylglyceride, Triacylglyceride oder sonstige Fettsäureester wie die AcetylCoenzymA-Ester, die die mehrfach ungesättigten Fettsäuren mit mindestens vier, fünf oder sechs bevorzugt fünf oder sechs Doppelbindungen enthalten, isoliert werden, vorteilhaft werden sie in der Form ihrer Diacylglyceride, Triacylglyceride und/oder in Form des Phosphatidylcholin isoliert, besonders bevorzugt in der Form der Triacylglyceride. Neben diesen Estern sind die mehrfach ungesättigten Fettsäuren auch als freie Fettsäuren oder gebunden in anderen Verbindungen in den Organismen vorteilhaft den Pflanzen enthalten. In der Regel liegen die verschiedenen vorgenannten Verbindungen (Fettsäureester und frei Fettsäuren) in den Organismen in einer ungefähren Verteilung von 80 bis 90 Gew.-% Triglyceride, 2 bis 5 Gew.-% Diglyceride, 5 bis 10 Gew.-% Monoglyceride, 1 bis 5 Gew.-% freie Fettsäuren, 2 bis 8 Gew.-% Phospholipide vor, wobei sich die Summe der verschiedenen Verbindungen zu 100 Gew.-% ergänzt.

Im Verfahren werden die hergestellten LCPUFAs wie Arachidonsäure und Eicosapentaensäure mit einem Gehalt von mindestens 15 Gew.-%, vorteilhaft von mindestens 16 oder 17 Gew.-%, bevorzugt von mindestens 18, 19 oder 20 Gew.-%, besonders bevorzugt von mindestens 21, 22, 23, 24 oder 25 Gew.-%, ganz besonders bevorzugt von mindestens 26, 27, 28, 29 oder 30 Gew.-% bezogen auf die gesamten Fettsäuren in den transgenen Organismen vorteilhaft in einer transgenen Pflanze hergestellt. Dabei werden vorteilhaft C₁₈- und/oder C₂₀-Fettsäuren, die in den Wirtsorganismen vorhanden sind, zu mindestens 10 %, vorteilhaft zu mindestens 20 %, besonders vorteilhaft zu mindestens 30 %, ganz besonders vorteilhaft zu mindestens 40 % in die entsprechenden Produkte wie ARA, EPA und/oder DHA umgesetzt. Vorteilhaft werden die Fettsäuren in gebundener Form hergestellt. Mit Hilfe der im Verfahren verwendeten Nukleinsäuren lassen sich diese ungesättigten Fettsäuren an sn1-, sn2- und/oder sn3-Position der vorteilhaft hergestellten Triglyceride bringen. Da im Verfahren von den Ausgangsverbindungen Linolsäure (C18:2) bzw. Linolensäure (C18:3) mehrere Reaktionsschritte durchlaufen werden, fallen die Endprodukte des Verfahrens wie beispielsweise Arachidonsäure (ARA), Eicosapentaensäure (EPA) oder Docosapentaensäure (DHA) nicht als absolute Reinprodukte an, es sind immer auch geringe Spuren der Vorstufen im Endprodukt enthalten. Sind in dem Ausgangsorganismus bzw. in der Ausgangspflanze beispielsweise sowohl Linolsäure als auch Linolensäure vorhanden, so liegen die Endprodukte wie ARA, EPA oder DHA in der Regel als Mischungen vor. Die Vorstufen Linolsäure und/oder Linolensäure sollten vorteilhaft nicht mehr als 20 Gew.-%, bevorzugt nicht mehr als 15 Gew.-%, besonders bevorzugt nicht als 10 Gew.-%, ganz besonders bevorzugt nicht mehr als 5 Gew.-% bezogen auf die Menge des jeweilige Endproduktes betragen. Vorteilhaft werden in einer transgenen Pflanze als Endprodukte nur ARA, ARA und EPA, EPA und DHA oder nur DHA im Verfahren gebunden oder als freie Säuren hergestellt. Werden die Verbindungen ARA, EPA und DHA gleichzeitig hergestellt, werden sie vorteilhaft in einem Verhältnis von mindesten 1:1:2 (EPA:ARA:DHA), vorteilhaft von mindestens 1:1:3, bevorzugt von 1:1:4, besonders bevorzugt von 1:1:5 hergestellt. Werden ARA und EPA im Verfahren hergestellt, so werden sie vorteilhaft in einem Verhältnis von mindestens 5:1 bis mindestens 1:5 hergestellt. Werden im Verfahren EPA und DHA hergestellt, so werden sie vorteilhaft in einem Verhältnis von mindestens 5:1 bis mindestens 1:5 hergestellt.

Fettsäureester bzw. Fettsäuregemische, die nach dem Verfahren hergestellt wurden, enthalten vorteilhaft 6 bis 15 % Palmitinsäure, 1 bis 6 % Stearinsäure; 7 - 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85 % mehrfach ungesättigte Fettsäuren jeweils bezogen auf 100 % und auf den Gesamtfettsäuregehalt der Organismen. Als vorteilhafte mehrfach ungesättigte Fettsäure sind in den Fettsäureester bzw. Fettsäuregemische bevorzugt mindestens 0,1; 0,2; 0,3; 0,4; 0,5; 0,6; 0,7; 0,8; 0,9 oder 1 % bezogen auf den Gesamtfettsäuregehalt an Arachidonsäure enthalten. Weiterhin enthalten die Fettsäureester bzw. Fettsäuregemische, die nach dem Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopenten-dodecansäure), Furan-Fettsäure (9,12-Epoxy-octadeca-9,11-dienonsäure), Vernonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11t13c-Octadecatriensäure), Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem Verfahren hergestellten Fettsäureester bzw. Fettsäuregemischen in der Regel vorteilhaft nur in geringen Mengen vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. Vorteilhaft enthalten die nach dem Verfahren hergestellten Fettsäureester bzw. Fettsäuregemische weniger als 0,1 % bezogen auf die Gesamtfettsäuren oder keine Buttersäure, kein Cholesterin, keine Clupanodonsäure (= Docosapentaensäure, C22:5^{Δ4,8,12,15,21}) sowie keine Nisinsäure (Tetracosahexaensäure, C23:6^{Δ3,8,12,15,18,21}).

Durch die erfindungsgemäßen Nukleinsäuresequenzen bzw. im Verfahren verwendeten Nukleinsäuresequenzen kann eine Steigerung der Ausbeute an mehrfach ungesättigten Fettsäuren von mindestens 50 %, vorteilhaft von mindestens 80 %, besonders vorteilhaft von mindestens 100 %, ganz besonders vorteilhaft von mindestens 150 % gegenüber der nicht transgenen Pflanze wie Arabidopsis, Lein, Raps, Soja oder Camelina beim Vergleich in der GC-Analyse erreicht werden.

Auch chemisch reine mehrfach ungesättigte Fettsäuren oder Fettsäurezusammensetzungen sind nach den vorbeschriebenen Verfahren darstellbar. Dazu werden die Fettsäuren oder die Fettsäurezusammensetzungen aus der Pflanze in bekannter Weise beispielsweise über Extraktion, Destillation, Kristallisation, Chromatographie oder Kombinationen dieser Methoden isoliert. Diese chemisch reinen Fettsäuren oder Fettsäurezusammensetzungen sind für Anwendungen im Bereich der Lebensmittelindustrie, der Kosmetikindustrie und besonders der Pharmaindustrie vorteilhaft.

Als Organismus für die Herstellung im Verfahren kommen prinzipiell alle Pflanzen in Frage. Vorteilhaft werden Ölfruchtpflanzen oder Nutzpflanzen im Verfahren verwendet. Als Pflanzen kommen prinzipiell alle Pflanzen in Frage, die in der Lage sind Fettsäuren zu synthetisieren wie alle dicotylen oder monokotylen Pflanzen, Algen oder Moose. Vorteilhaft Pflanzen sind ausgewählt aus der Gruppe der Pflanzenfamilien Adelotheciaceae, Anacardiaceae, Asteraceae, Apiaceae, Betulaceae, Boraginaceae, Brassicaceae, Bromeliaceae, Caricaceae, Cannabaceae, Convolvulaceae, Chenopodiaceae, Crypthecodiniaceae, Cucurbitaceae, Ditrichaceae, Elaeagnaceae, Ericaceae, Euphorbiaceae, Fabaceae, Geraniaceae, Gramineae, Juglandaceae, Lauraceae, Leguminosae, Linaceae, Prasinophyceae oder Gemüsepflanzen oder Zierpflanzen wie Tagetes in Betracht.

Beispielhaft seien die folgenden Pflanzen genannt ausgewählt aus der Gruppe: Adelotheciaceae wie die Gattungen Physcomitrella z.B. die Gattung und Arten *Physcomitrella patens,* Anacardiaceae wie die Gattungen Pistacia, Mangifera, Anacardium z.B. die Gattung und Arten *Pistacia vera* [Pistazie], *Mangiferindica* [Mango] oder *Anacardium occidentale* [Cashew], Asteraceae wie die Gattungen Calendula, Carthamus, Centaurea, Cichorium, Cynara, Helianthus, Lactuca, Locusta, Tagetes, Valeriana z.B. die Gattung und Arten Calendula officinalis [Garten-Ringelblume], Carthamus tinctorius [Färberdistel, safflower], *Centaurea cyanus* [Kornblume], *Cichorium intybus* [Wegwarte], Cynara scolymus [Artichoke], *Helianthus annus* [Sonnenblume], *Lactuca sativa, Lactuca crispa, Lactuca esculenta, Lactuca scariola L. ssp. sativa, Lactuca scariola L. var. integrata, Lactuca scariola L. var. integrifolia, Lactuca sativa subsp. romana, Locusta communis, Valeriana locusta* [Salat], *Tagetes lucida, Tagetes erecta* oder *Tagetes tenuifolia* [Studentenblume], Apiaceae wie die Gattung Daucus z.B. die Gattung und Art *Daucus carota* [Karotte], Betulaceae wie die Gattung Corylus z.B. die Gattungen und Arten *Corylus avellana* oder *Corylus colurna* [Haselnuss], Boraginaceae wie die Gattung Borago z.B. die Gattung und Art *Borago officinalis* [Borretsch], Brassicaceae wie die Gattungen Brassica, Camelina, Melanosinapis, Sinapis, Arabadopsis z.B. die Gattungen und Arten *Brassica napus, Brassica rapa* ssp. [Raps], *Sinapis arvensis Brassica juncea, Brassica juncea var. juncea, Brassica juncea var. crispifolia, Brassica juncea var. foliosa, Brassica nigra, Brassica sinapioides,* Camelina sativa, *Melanosinapis communis* [Senf], *Brassica oleracea* [Futterrübe] oder Arabidopsis thaliana, Bromeliaceae wie die Gattungen Anana, Bromelia (Ananas) z.B. die Gattungen und Arten *Anana comosus, Ananas ananas* oder *Bromelia comosa* [Ananas], Caricaceae wie die Gattung Carica wie die Gattung und Art *Carica papaya* [Papaya], Cannabaceae wie die Gattung Cannabis wie die Gattung und Art *Cannabis sative* [Hanf], Convolvulaceae wie die Gattungen Ipomea, Convolvulus z.B. die Gattungen und Arten *Ipomoea batatus, Ipomoea pandurata, Convolvulus batatas, Convolvulus tiliaceus, Ipomoea fastigiata, Ipomoea tiliacea, Ipomoea triloba* oder *Convolvulus panduratus* [Süßkartoffel, Batate], Chenopodiaceae wie die Gattung Beta wie die Gattungen und Arten *Beta vulgaris, Beta vulgaris var. altissima, Beta vulgaris var. Vulgaris, Beta maritima, Beta vulgaris var. perennis, Beta vulgaris var. conditiva* oder *Beta vulgaris var. esculenta* [Zuckerrübe], Crypthecodiniaceae wie die Gattung Crypthecodinium z.B. die Gattung und Art *Cryptecodinium cohnii,* Cucurbitaceae wie die Gattung Cucubita z.B. die Gattungen und Arten *Cucurbita maxima, Cucurbita mixta, Cucurbita pepo* oder *Cucurbita moschata* [Kürbis], Cymbellaceae wie die Gattungen Amphora, Cymbella, Okedenia, Phaeodactylum, Reimeria z.B. die Gattung und Art *Phaeodactylum tricornutum,* Ditrichaceae wie die Gattungen Ditrichaceae, Astomiopsis, Ceratodon, Chrysoblastella, Ditrichum, Distichium, Eccremidium, Lophidion, Philibertiella, Pleuridium, Saelania, Trichodon, Skottsbergia z.B. die Gattungen und Arten *Ceratodon antarcticus, Ceratodon columbiae, Ceratodon heterophyllus, Ceratodon purpurascens, Ceratodon purpureus, Ceratodon purpureus ssp. convolutus, Ceratodon purpureus ssp. stenocarpus, Ceratodon purpureus var. rotundifolius, Ceratodon ratodon, Ceratodon stenocarpus, Chrysoblastella chilensis, Ditrichum ambiguum, Ditrichum brevisetum, Ditrichum crispatissimum, Ditrichum difficile, Ditrichum falcifolium, Ditrichum flexicaule, Ditrichum giganteum, Ditrichum heteromallum, Ditrichum lineare, Ditrichum lineare, Ditrichum montanum, Ditrichum montanum, Ditrichum pallidum, Ditrichum punctulatum, Ditrichum pusillum, Ditrichum pusillum var. tortile, Ditrichum rhynchostegium, Ditrichum schimperi, Ditrichum tortile, Distichium capillaceum, Distichium hagenii, Distichium inclinatum, Distichium macounii, Eccremidium floridanum, Eccremidium whiteleggei, Lophidion strictus, Pleuridium acuminatum, Pleuridium alternifolium, Pleuridium holdridgei, Pleuridium mexicanum, Pleuridium ravenelii, Pleuridium subulatum, Saelania glaucescens, Trichodon borealis, Trichodon cylindricus oder Trichodon cylindricus var. oblongus,* Elaeagnaceae wie die Gattung Elaeagnus z.B. die Gattung und Art *Olea europaea* [Olive], Ericaceae wie die Gattung Kalmia z.B. die Gattungen und Arten *Kalmia latifolia, Kalmia angustifolia, Kalmia microphylla, Kalmia polifolia, Kalmia occidentalis, Cistus chamaerhodendros* oder *Kalmia lucida* [Berglorbeer], Euphorbiaceae wie die Gattungen Manihot, Janipha, Jatropha, Ricinus z.B. die Gattungen und Arten *Manihot utilissima, Janipha manihot" Jatropha manihot., Manihot aipil, Manihot dulcis, Manihot manihot, Manihot melanobasis, Manihot esculenta* [Manihot] oder *Ricinus communis* [Rizinus], Fabaceae wie die Gattungen Pisum, Albizia, Cathormion, Feuillea, Inga, Pithecolobium, Acacia, Mimosa, Medicajo, Glycine, Dolichos, Phaseolus, Soja z.B. die Gattungen und Arten *Pisum sativum, Pisum arvense, Pisum humile* [Erbse], *Albizia berteriana, Albizia julibrissin, Albizia lebbeck, Acacia berteriana, Acacia littoralis, Albizia berteriana, Albizzia berteriana, Cathormion berteriana, Feuillea berteriana, Inga fragrans, Pithecellobium berterianum, Pithecellobium fragrans, Pithecolobium berterianum, Pseudalbizzia berteriana, Acacia julibrissin, Acacia nemu, Albizia nemu, Feuilleea julibrissin, Mimosa julibrissin, Mimosa speciosa, Sericanrda julibrissin, Acacia lebbeck, Acacia macrophylla, Albizia lebbek, Feuilleea lebbeck, Mimosa lebbeck, Mimosa speciosa* [Seidenbaum], *Medicago sativa, Medicago falcata, Medicago varia* [Alfalfa] *Glycine max Dolichos soja, Glycine gracilis, Glycine hispida, Phaseolus max, Soja hispida* oder *Soja max* [Sojabohne], Funariaceae wie die Gattungen Aphanorrhegma, Entosthodon, Funaria, Physcomitrella, Physcomitrium z.B. die Gattungen und Arten *Aphanorrhegma serratum, Entosthodon attenuatus, Entosthodon bolanderi, Entosthodon bonplandii, Entosthodon californicus, Entosthodon drummondii, Entosthodon jamesonii*, *Entosthodon leibergii, Entosthodon neoscoticus, Entosthodon rubrisetus, Entosthodon spathulifolius, Entosthodon tucsoni, Funaria americana, Funaria bolanderi, Funaria calcarea, Funaria californica, Funaria calvescens, Funaria convoluta, Funaria flavicans, Funaria groutiana, Funaria hygrometrica, Funaria hygrometrica var. arctica, Funaria hygrometrica var. calvescens, Funaria hygrometrica var. convoluta, Funaria hygrometrica var. muralis, Funaria hygrometrica var. utahensis, Funaria microstoma, Funaria microstoma var. obtusifolia, Funaria muhlenbergii, Funaria orcuttii, Funaria plano-convexa, Funaria polaris, Funaria ravenelii, Funaria rubriseta, Funaria serrata, Funaria sonorae, Funaria sublimbatus, Funaria tucsoni, Physcomitrella californica, Physcomitrella patens, Physcomitrella readeri, Physcomitrium australe, Physcomitrium californicum, Physcomitrium collenchymatum, Physcomitrium coloradense, Physcomitrium cupuliferum, Physcomitrium drummondii, Physcomitrium eurystomum, Physcomitrium flexifolium, Physcomitrium hookeri, Physcomitrium hookeri var. serratum, Physcomitrium immersum, Physcomitrium kellermanii, Physcomitrium megalocarpum, Physcomitrium pyriforme, Physcomitrium pyriforme var. serratum, Physcomitrium rufipes, Physcomitrium sandbergii, Physcomitrium subsphaericum, Physcomitrium washingtoniense,* Geraniaceae wie die Gattungen Pelargonium, Cocos, Oleum z.B. die Gattungen und Arten *Cocos nucifera, Pelargonium grossularioides* oder *Oleum cocois* [Kokusnuss], Gramineae wie die Gattung Saccharum z.B. die Gattung und Art Saccharum officinarum, Juglandaceae wie die Gattungen Juglans, Wallia z.B. die Gattungen und Arten *Juglans regia, Juglans ailanthifolia, Juglans sieboldiana, Juglans cinerea, Wallia cinerea, Juglans bixbyi, Juglans californica, Juglans hindsii, Juglans intermedia, Juglans jamaicensis, Juglans major, Juglans microcarpa, Juglans nigra* oder *Wallia nigra* [Walnuss], Lauraceae Wie die Gattungen Persea, Laurus z.B. die Gattungen und Arten *Laurus nobilis* [Lorbeer], *Persea americana, Persea gratissima* oder *Persea persea* [Avocado], Leguminosae wie die Gattung Arachis z.B. die Gattung und Art *Arachis hypogaea* [Erdnuss], Linaceae wie die Gattungen Linum, Adenolinum z.B. die Gattungen und Arten *Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense* oder *Linum trigynum* [Lein], Lythrarieae wie die Gattung Punica z.B. die Gattung und Art *Punica granatum* [Granatapfel], Malvaceae wie die Gattung Gossypium z.B. die Gattungen und Arten *Gossypium hirsutum, Gossypium arboreum, Gossypium barbadense, Gossypium herbaceum* oder *Gossypium thurberi* [Baumwolle], Marchantiaceae wie die Gattung Marchantia z.B. die Gattungen und Arten *Marchantia berteroana, Marchantia foliacea, Marchantia macropora,* Musaceae wie die Gattung Musa z.B. die Gattungen und Arten *Musa nana, Musa acuminata, Musa paradisiaca, Musa* spp. [Banane], Onagraceae wie die Gattungen Camissonia, Oenothera z.B. die Gattungen und Arten *Oenothera biennis* oder *Camissonia brevipes* [Nachtkerze], Palmae wie die Gattung Elacis z.B. die Gattung und Art *Elaeis guineensis* [Ölpalme], Papaveraceae wie die Gattung Papaver z.B. die Gattungen und Arten *Papaver orientale, Papaver rhoeas, Papaver dubium* [Mohn], Pedaliaceae wie die Gattung Sesamum z.B. die Gattung und Art *Sesamum indicum* [Sesam], Piperaceae wie die Gattungen Piper, Artanthe, Peperomia, Steffensia z.B. die Gattungen und Arten *Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata.* [Cayennepfeffer], Poaceae wie die Gattungen Hordeum, Secale, Avena, Sorghum, Andropogon, Holcus, Panicum, Oryza, Zea (Mais), Triticum z.B. die Gattungen und Arten *Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum* [Gerste], *Secale cereale* [Roggen], Avena sativa, *Avena fatua, Avena byzantina, Avena fatua var. sativa, Avena hybrida* [Hafer], *Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum, Panicum militaceum [Hirse], Oryza sativa, Oryza latifolia* [Reis], *Zea mays* [Mais] *Triticum aestivum, Triticum durum, Triticum turgidum, Triticum hybernum, Triticum macha, Triticum sativum* oder *Triticum vulgare* [Weizen], Porphyridiaceae wie die Gattungen Chroothece, Flintiella, Petrovanella, Porphyridium, Rhodella, Rhodosorus, Vanhoeffenia z.B. die Gattung und Art *Porphyridium cruentum,* Proteaceae wie die Gattung Macadamia z.B. die Gattung und Art *Macadamia intergrifolia* [Macadamia], Prasinophyceae wie die Gattungen Nephroselmis, Prasinococcus, Scherffelia, Tetraselmis, Mantoniella, Ostreococcus z.B. die Gattungen und Arten *Nephroselmis olivacea, Prasinococcus capsulatus, Scherffelia dubia, Tetraselmis chui, Tetraselmis suecica,* Mantoniella squamata, Ostreococcus tauri, Rubiaceae wie die Gattung Coffea z.B. die Gattungen und Arten Cofea spp., *Coffea arabica, Coffea canephora* oder *Coffea liberica* [Kaffee], Scrophulariaceae wie die Gattung Verbascum z.B. die Gattungen und Arten *Verbascum blattaria, Verbascum chaixii, Verbascum densiflorum, Verbascum lagurus, Verbascum longifolium, Verbascum lychnitis, Verbascum nigrum, Verbascum olympicum, Verbascum phlomoides, Verbascum phoenicum, Verbascum pulverulentum* oder *Verbascum thapsus* [*Königskerze*], Solanaceae wie die Gattungen Capsicum, Nicotiana, Solanum, Lycopersicon z.B. die Gattungen und Arten *Capsicum annuum, Capsicum annuum var. glabriusculum, Capsicum frutescens* [Pfeffer], *Capsicum annuum* [Paprika], *Nicotiana tabacum, Nicotiana alata, Nicotiana attenuata, Nicotiana glauca, Nicotiana langsdorffii, Nicotiana obtusifolia, Nicotiana quadrivalvis, Nicotiana repanda, Nicotiana rustica, Nicotiana sylvestris* [Tabak], *Solanum tuberosum* [Kartoffel], *Solanum melongena* [Aubergine] *Lycopersicon esculentum, Lycopersicon lycopersicum., Lycopersicon pyriforme, Solanum integrifolium* oder *Solanum lycopersicum* [Tomate], Sterculiaceae wie die Gattung Theobroma z.B. die Gattung und Art *Theobroma cacao* [Kakao] oder Theaceae wie die Gattung Camellia z.B. die Gattung und Art *Camellia sinensis* [Tee].

Im Verfahren werden transgene Pflanzen wie zweikeimblättrige oder einkeimblättrige Pflanzen verwendet. Besonders vorteilhaft werden im Verfahren Ölfruchtpflanzen verwendet, die große Mengen an Lipidverbindungen enthalten, wie Erdnuss, Raps, Canola, Sonnenblume, Saflor (Carthamus tinctoria), Mohn, Senf, Hanf, Rizinus, Olive, Sesam, Calendula, Punica, Nachtkerze, Königskerze, Distel, Wildrosen, Haselnuss, Mandel, Macadamia, Avocado, Lorbeer, Kürbis, Lein, Soja, Pistazien, Borretsch, Bäume (Ölpalme, Kokosnuss oder Walnuss), oder es werden Nutzpflanzen, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa oder Buschpflanzen (Kaffee, Kakao, Tee), Salix-Arten verwendet. Bevorzugte erfindungsgemäße Pflanzen sind Ölfruchtpflanzen, wie Erdnuss, Raps, Canola, Sonnenblume, Saflor, Mohn, Senf, Hanf, Rhizinus, Olive, Calendula, Punica, Nachtkerze, Kürbis, Lein, Soja, Borretsch, Bäume (Ölpalme, Kokosnuss). Besonders bevorzugt sind C18:2- und/oder C18:3-Fettsäure reiche Pflanzen wie Sonnenblume, Färberdistel, Tabak, Königskerze, Sesam, Baumwolle, Kürbis, Mohn, Nachtkerze, Walnuss, Lein, Hanf, Distel oder Färberdistel. Ganz besonders bevorzugt sind Pflanzen wie Färberdistel, Sonnenblume, Mohn, Nachtkerze, Walnuss, Lein oder Hanf.

Im Prinzip können alle Gene des Fettsäure- oder Lipidstoffwechsels vorteilhaft in Kombination mit der(den) erfinderischen Δ-6-Desaturase(n), Δ-6-Elongase(n), Δ-5-Desaturase(n), Δ-5-Elongase(n), Δ-4-Desaturase(n), Δ-12-Desaturase(n) und/oder ω-3-Desaturase(n) [im Sinne dieser Anmeldung soll der Plural den Singular und umgekehrt beinhalten] im Verfahren zur Herstellung mehrfach ungesättigter Fettsäuren verwendet werden. Vorteilhaft werden Gene des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferasen, Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) in Kombination mit der Δ-6-Desaturase(n), Δ-6-Elongase(n), Δ-5-Desaturase(n), Δ-5-Elongase(n), Δ-4-Desaturase(n), Δ-12-Desaturase(n) und/oder ω-3-Desaturase(n) verwendet, wobei einzelne Gene oder mehrere Gene in Kombination verwendet werden können.

Neben der Produktion der Ausgangsfettsäuren für die im Verfahren verwendeten Δ-6-Desaturase, Δ-6-Elongase, Δ-5-Desaturase, Δ-5-Elongase, Δ-4-Desaturase, Δ-12-Desaturase und eine ω-3-Desaturase direkt im Organismus können die Fettsäuren auch von außen gefüttert werden. Aus kostengründen ist die Produktion im Organismus bevorzugt. Bevorzugte Substrate sind die Linolsäure (C18:2^{Δ9,12}), und/oder die γ-Linolensäure (C18:3^{Δ6,9,12}).

Zur Steigerung der Ausbeute im beschriebenen Verfahren zur Herstellung von Ölen und/oder Triglyceriden mit einem vorteilhaft erhöhten Gehalt an mehrfach ungesättigten Fettsäuren ist es vorteilhaft die Menge an Ausgangsprodukt für die Fettsäuresynthese zu steigern, dies kann beispielsweise durch das Einbringen einer Nukleinsäure in den Organismus, die für ein Polypeptid mit Δ-12-Desaturase codiert, erreicht werden. Vorteilhaft setzt die erfingungsgemäßen Verfahren verwendete Δ-12-Desaturase Ölsaure (C18:1^{Δ9}) zu Linolsäure (C18:2^{Δ9,12}) oder C18:2^{Δ6,9} zu C18:3^{Δ6,9,12} (= GLA) um. Dadurch werden die Ausgangsprodukte Linolsäure (C18:2^{Δ9,12}) und γ-Linolensäure (C18:3^{Δ6,9,12}) für die Synthese der mehrfach ungesättigten Fettsäuren verstärkt zu Verfügung gestellt. Dies ist besonders vorteilhaft in Öl-produzierenden Organismen wie der Familie der Brassicaceae wie der Gattung Brassica z.B. Raps; der Familie der Elaeagnaceae wie die Gattung Elaeagnus z.B. die Gattung und Art *Olea europaea* oder der Familie Fabaceae wie der Gattung Glycine z.B. die Gattung und Art *Glycine max,* die einen hohen Ölsäuregehalt aufweisen. Da diese Organismen nur einen geringen Gehalt an Linolsäure aufweisen (Mikoklajczak et al., Journal of the American Oil Chemical Society, 38, 1961, 678 - 681) ist die Verwendung der genannten Δ-12-Desaturasen zur Herstellung des Ausgangsprodukts Linolsäure vorteilhaft.

Im Verfahren verwendeten Nukleinsäuren stammen vorteilhaft aus Pflanzen wie Algen beispielsweise Algen der Familie der Prasinophyceae wie aus den Gattungen Heteromastix, Mammella, Mantoniella, Micromonas, Nephroselmis, Ostreococcus, Prasinocladus, Prasinococcus, Pseudoscourfielda, Pycnococcus, Pyramimonas, Scherffelia oder Tetraselmis wie den Gattungen und Arten Heteromastix longifillis, Mamiella gilva, Mantoniella squamata, Micromonas pusilla, Nephroselmis olivacea, Nephroselmis pyriformis, Nephroselmis rotunda, Ostreococcus tauri, Ostreococcus sp. Prasinocladus ascus, Prasinocladus lubricus, Pycnococcus provasolii, Pyramimonas amylifera, Pyramimonas disomata, Pyramimonas obovata, Pyramimonas orientalis, Pyramimonas parkeae, Pyramimonas spinifera, Pyramimonas sp., Tetraselmis apiculata, Tetraselmis carteriaformis, Tetraselmis chui, Tetraselmis convolutae, Tetraselmis desikacharyi, Tetraselmis gracilis, Tetraselmis hazeni, Tetraselmis impellucida, Tetraselmis inconspicua, Tetraselmis levis, Tetraselmis maculata, Tetraselmis marina, Tetraselmis striata, Tetraselmis subcordiformis, Tetraselmis suecica, Tetraselmis tetrabrachia, Tetraselmis tetrathele, Tetraselmis verrucosa, Tetraselmis verrucosa fo. Rubens oder Tetraselmis sp. vorteilhaft stammen die verwendeten Nukleinsäuren aus Algen der Gattung Ostreococcus. Weitere vorteilhafte Organismen sind Diatomeen wie Thalassiosira, Phaeodactylum oder Thraustochytrium oder Pilze wie Thraustochytrium oder Phytophthora.

Vorteilhaft werden im Verfahren die vorgenannten Nukleinsäuresequenzen oder deren Derivat oder Homologe, die für Polypeptide codieren, die noch die enzymatische Aktivität der durch Nukleinsäuresequenzen codierten Proteine besitzen. Diese Sequenzen werden einzeln oder in Kombination mit den für die A-6-Desaturase, Δ-6-Elongase, Δ-5-Desaturase, Δ-5-Elongase, Δ-4-Desaturase, Δ-12-Desaturase und/oder ω-3-Desaturase codierenden Nukleinsäuresquenzen in Expressionskonstrukte cloniert und zum Einbringen und zur Expression in den Pflanzen verwendet. Diese Expressionskonstrukte ermöglichen durch ihre Konstruktion eine vorteilhafte optimale Synthese der im Verfahren produzierten mehrfach ungesättigten Fettsäuren.

Bei einer bevorzugten Ausführungsform umfasst das Verfahren ferner den Schritt des Gewinnens einer Zelle oder einer ganzen Pflanze, die die im Verfahren verwendeten Nukleinsäuresequenzen enthält, wobei die Zelle und/oder die Pflanze mit einer Nukleinsäuresequenz, die für die Δ-6-Desaturase, Δ-6-Elongase, Δ-5-Desaturase, Δ-5-Elongase, Δ-4-Desaturase, Δ-12-Desaturase und/oder ω-3-Desaturase codiert, einem Genkonstrukt oder einem Vektor wie nachfolgend beschrieben, allein oder in Kombination mit weiteren Nukleinsäuresequenzen, die für Proteine des Fettsäure- oder Lipidsstoffwechsels codieren, transformiert wird. Bei einer weiteren bevorzugten Ausführungsform umfasst dieses Verfahren ferner den Schritt des Gewinnens der Öle, Lipide oder freien Fettsäuren aus der Zelle oder Pflanze. Bei der Kultur kann es sich beispielsweise um eine Fermentationskultur beispielsweise im Falle der Kultivierung von Pflanzenzellen oder um eine Treibhaus oder Feldkultur einer Pflanze handeln. Die so hergestellte Zelle oder die so hergestellte Pflanze ist vorteilhaft eine Zelle eines Ölproduzierenden Organismus wie einer Ölfruchtpflanze wie beispielsweise Erdnuss, Raps, Canola, Lein, Hanf, Erdnuss, Soja, Safflower, Hanf, Sonnenblumen oder Borretsch.

Unter Anzucht ist beispielsweise die Kultivierung im Falle von Pflanzenzellen, -gewebe oder -organe auf oder in einem Nährmedium oder der ganzen Pflanze auf bzw. in einem Substrat beispielsweise in Hydrokultur, Blumentopferde oder auf einem Ackerboden zu verstehen.

"Transgen" bzw. "Rekombinant" im Sinne der Erfindung bedeutet bezüglich zum Beispiel einer Nukleinsäuresequenz, einer Expressionskassette (= Genkonstrukt) oder einem Vektor enthaltend die erfindungsgemäße Nukleinsäuresequenz oder einem Organismus transformiert mit den erfindungsgemäßen Nukleinsäuresequenzen, Expressionskassette oder Vektor alle solche durch gentechnische Methoden zustandegekommenen Konstruktionen, in denen sich entweder
a) die erfindungsgemäße Nukleinsäuresequenz, oder
b) eine mit der erfindungsgemäßen Nukleinsäuresequenz funktionell verknüpfte genetische Kontrollsequenz, zum Beispiel ein Promotor, oder
c) (a) und (b)
sich nicht in ihrer natürlichen, genetischen Umgebung befinden oder durch gentechnische Methoden modifiziert wurden, wobei die Modifikation beispielhaft eine Substitution, Addition, Deletion, Inversion oder Insertion eines oder mehrerer Nukleotidreste sein kann. Natürliche genetische Umgebung meint den natürlichen genomischen bzw. chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des natürlichen Promotors der erfindungsgemäßen Nukleinsäuresequenzen mit den entsprechenden Δ-6-Desaturase-, Δ-6-Elongase-, Δ-5-Desaturase-, Δ-5-Eiongase-, Δ-4-Desaturase-, Δ-12-Desaturase- und/oder ω-3-Desaturasegenen - wird zu einer transgenen Expressionskassette, wenn diese durch nicht-natürliche, synthetische ("künstliche") Verfahren wie beispielsweise einer Mutagenisierung geändert wird. Entsprechende Verfahren sind beispielsweise beschrieben in US 5,565,350 oder WO 00/15815.

Unter transgenen Pflanzen im Sinne der Erfindung ist wie vorgenannt zu verstehen, dass die im Verfahren verwendeten Nukleinsäuren nicht an ihrer natürlichen Stelle im Genom der Pflanze sind, dabei können die Nukleinsäuren homolog oder heterolog exprimiert werden. Transgen bedeutet aber auch wie genannt, dass die erfindungsgemäßen Nukleinsäuren an ihrem natürlichen Platz im Genom der Pflanze sind, dass jedoch die Sequenz gegenüber der natürlichen Sequenz verändert wurde und/oder das die Regulationssequenzen, der natürlichen Sequenzen verändert wurden. Bevorzugt ist unter transgen die Expression der erfindungsgemäßen Nukleinsäuren an nicht natürlicher Stelle im Genom zu verstehen, das heißt eine homologe oder bevorzugt heterologe Expression der Nukleinsäuren liegt vor.

Als Wirtsorganismen für die im Verfahren verwendeten Nukleinsäuren, die Expressionskassette oder den Vektor eignen sich prinzipiell vorteilhaft alle Pflanzen, die in der Lage sind Fettsäuren speziell ungesättigte Fettsäuren zu synthetisieren bzw. für die Expression rekombinanter Gene geeignet sind. Beispielhaft seien Pflanzen wie Arabidopsis, Asteraceae wie Calendula oder Kulturpflanzen wie Soja, Erdnuss, Rizinus, Sonnenblume, Mais, Baumwolle, Flachs, Raps, Kokosnuss, Ölpalme, FärberSaflor (Carthamus tinctorius) oder Kakaobohne. Bevorzugt werden Pflanzen, die natürlicherweise Öle in größeren Mengen synthetisieren können wie Soja, Raps, Kokosnuss, Ölpalme, FärberSaflor, Flachs, Hanf, Rizinus, Calendula, Erdnuss, Kakaobohne oder Sonnenblume.

Zu den Pflanzen im Sinne der Erfindung gehören Pflanzenzellen und bestimmte Gewebe, Organe und Teile von Pflanzen in all ihren Erscheinungsformen, wie Antheren, Fasern, Wurzelhaare, Stängel, Embryos, Kalli, Kotelydonen, Petiolen, Erntematerial, pflanzliches Gewebe, reproduktives Gewebe und Zellkulturen, das von der eigentlichen transgenen Pflanze abgeleitet ist und/oder dazu verwendet werden kann, die transgene Pflanze hervorzubringen.

Transgene Pflanzen, die die im Verfahren synthetisierten mehrfach ungesättigten Fettsäuren enthalten, können vorteilhaft direkt vermarktet werden ohne dass die synthetisierten Öle, Lipide oder Fettsäuren isoliert werden müssen. Unter Pflanzen im Verfahren sind ganze Pflanzen sowie alle Pflanzenteile, Pflanzenorgane oder Pflanzenteile wie Blatt, Stiel, Samen, Wurzel, Knollen, Antheren, Fasern, Wurzelhaare, Stängel, Embryos, Kalli, Kotelydonen, Petiolen, Erntematerial, pflanzliches Gewebe, reproduktives Gewebe, Zellkulturen, die sich von der transgenen Pflanze abgeleiten und/oder dazu verwendet werden können, die transgene Pflanze hervorzubringen. Der Samen umfasst dabei alle Samenteile wie die Samenhüllen, Epidermis- und Samenzellen, Endosperm oder Embyrogewebe. Die im Verfahren hergestellten Verbindungen können aber auch aus den Organismen vorteilhaft Pflanzen in Form ihrer Öle, Fett, Lipide und/oder freien Fettsäuren isoliert werden. Durch dieses Verfahren hergestellte mehrfach ungesättigten Fettsäuren lassen sich durch Ernten der Organismen entweder aus der Kultur, in der sie wachsen, oder vom Feld ernten. Dies kann über Pressen oder Extraktion der Pflanzenteile bevorzugt der Pflanzensamen erfolgen. Dabei können die Öle, Fette, Lipide und/oder freien Fettsäuren durch sogenanntes kalt schlagen oder kalt pressen ohne Zuführung von Wärme durch Pressen gewonnen werden. Damit sich die Pflanzenteile speziell die Samen leichter aufschließen lassen, werden sie vorher zerkleinert, gedämpft oder geröstet. Die so vorbehandelten Samen können anschließend gepresst werden oder mit Lösungsmittel wie warmen Hexan extrahiert werden. Anschließend wird das Lösungsmittel wieder entfernt. Auf diese Weise können mehr als 96 % der im Verfahren hergestellten Verbindungen isoliert werden. Anschließend werden die so erhaltenen Produkte weiter bearbeitet, das heißt raffiniert. Dabei werden zunächst beispielsweise die Pflanzenschleime und Trübstoffe entfernt. Die sogenannte Entschleimung kann enzymatisch oder beispielsweise chemisch/physikalisch durch Zugabe von Säure wie Phosphorsäure erfolgen. Anschließend werden die freien Fettsäuren durch Behandlung mit einer Base beispielsweise Natronlauge entfernt. Das erhaltene Produkt wird zur Entfernung der im Produkt verbliebenen Lauge mit Wasser gründlich gewaschen und getrocknet. Um die noch im Produkt enthaltenen Farbstoffe zu entfernen werden die Produkte einer Bleichung mit beispielsweise Bleicherde oder Aktivkohle unterzogen. Zum Schluss wird das Produkt noch beispielsweise mit Wasserdampf noch desodoriert.

Eine Ausführungsform ist die Verwendung der Öle, Lipide, der Fettsäuren und/oder der Fettsäurezusammensetzung, die nach dem Verfahren hergestellt wurden oder die durch Mischung dieser Öle, Lipide und/oder Fettsäuren mit mit tierischen, mikrobiellen oder pflanzlichen Ölen, Lipiden oder Fettsäuren erhalten wurden, in Futtermitteln, Nahrungsmitteln, Kosmetika oder Pharmazeutika. Die im Verfahren hergestellten Öle, Lipide, Fettsäuren können in der dem Fachmann bekannten Weise zur Abmischung mit anderen Ölen, Lipiden, Fettsäuren oder Fettsäuregemischen tierischen Ursprungs wie z.B. Fischölen verwendet werden.

Unter dem Begriff "Öl", "Lipid" oder "Fett" wird ein Fettsäuregemisch verstanden, das ungesättigte, gesättigte, vorzugsweise veresterte Fettsäure(n), vorteilhaft in Triglyceride gebunden, enthält. Bevorzugt ist, dass das Öl, Lipid oder Fett einen hohen Anteil an mehrfach ungesättigten freien oder vorteilhaft veresterten Fettsäure(n), insbesondere Linolsäure, γ-Linolensäure, Dihomo-γ-linolensäure, Arachidonsäure, α-Linolensäure, Stearidonsäure, Eicosatetraensäure, Eicosapentaensäure, Docosapentaensäure oder Docosahexaensäure, besonders vorteilhaft Arachidonsäure, Eicosapentaensäure oder Docosahexaensäure hat. Vorzugsweise ist der Anteil an ungesättigten veresterten Fettsäuren ungefähr 30 %, mehr bevorzugt ist ein Anteil von 50 %, noch mehr bevorzugt ist ein Anteil von 60 %, 70 %, 80 % oder mehr. Zur Bestimmung kann z.B. der Anteil an Fettsäure nach Überführung der Fettsäuren in die Methylestern durch Umesterung gaschromatographisch bestimmt werden. Das Öl, Lipid oder Fett kann verschiedene andere gesättigte oder ungesättigte Fettsäuren, z.B. Calendulasäure, Palmitin-, Palmitolein-, Stearin-, Ölsäure etc., enthalten. Insbesondere kann je nach Ausgangsorganismus der Anteil der verschiedenen Fettsäuren in dem Öl oder Fett schwanken.

Bei den im Verfahren hergestellten mehrfach ungesättigte Fettsäuren mit vorteilhaft mindestens vier Doppelbindungen handelt es sich wie oben beschrieben beispielsweise um Sphingolipide, Phosphoglyceride, Lipide, Glycolipide, Phospholipide, Monoacylglyceride, Diacylglyceride, Triacylglyceride oder sonstige Fettsäureester, bevorzugt um Triacylglyceride.

Aus den so im Verfahren hergestellten mehrfach ungesättigte Fettsäuren mit vorteilhaft mindestens fünf oder sechs Doppelbindungen lassen sich die enthaltenden mehrfach ungesättigten Fettsäuren beispielsweise über eine Alkalibehandlung beispielsweise wäßrige KOH oder NaOH oder saure Hydrolyse vorteilhaft in Gegenwart eines Alkohols wie Methanol oder Ethanol oder über eine enzymatische Abspaltung freisetzen und isolieren über beispielsweise Phasentrennung und anschließender Ansäuerung über z.B. H₂SO₄. Die Freisetzung der Fettsäuren kann auch direkt ohne die vorhergehend beschriebene Aufarbeitung erfolgen.

Die im Verfahren verwendeten Nukleinsäuren können nach Einbringung in einem Organismus vorteilhaft einer Pflanzenzelle bzw. Pflanze entweder auf einem separaten Plasmid liegen oder vorteilhaft in das Genom der Wirtszelle integriert sein. Bei Integration in das Genom kann die Integration zufallsgemäß sein oder durch derartige Rekombination erfolgen, dass das native Gen durch die eingebrachte Kopie ersetzt wird, wodurch die Produktion der gewünschten Verbindung durch die Zelle moduliert wird, oder durch Verwendung eines Gens in trans, so dass das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist. Vorteilhaft werden die Nukleinsäuren über Multiexpressionskassetten oder Konstrukte zur multiparallelen Expression in die Organismen vorteilhaft zur multiparallelen samenspezifischen Expression von Genen in die Pflanzen gebracht.

Moose und Algen sind die einzigen bekannten Pflanzensysteme, die erhebliche Mengen an mehrfach ungesättigten Fettsäuren, wie Arachidonsäure (ARA) und/oder Eicosapentaensäure (EPA) und/oder Docosahexaensäure (DHA) herstellen. Moose enthalten PUFAs in Membranlipiden während Algen, algenverwandte Organismen und einige Pilze auch nennenswerte Mengen an PUFAs in der Triacylglycerolfraktion akkumulieren. Daher eignen sich Nukleinsäuremoleküle, die aus solchen Stämmen isoliert werden, die PUFAs auch in der Triacylglycerolfraktion akkumulieren, besonders vorteilhaft für das Verfahren und damit zur Modifikation des Lipid- und PUFA-Produktionssystems in einem Wirt, insbesondere Pflanzen, wie Ölfruchtpflanzen, beispielsweise Raps, Canola, Lein, Hanf, Soja, Sonnenblumen, Borretsch. Sie sind deshalb vorteilhaft im Verfahren verwendbar.

Als Substrate der im Verfahren verwendeten Nukleinsäuren, die für Polypeptide mit Δ-6-Desaturase-, Δ-6-Elongase, Δ-5-Desaturase-, Δ-5-Elongase-, Δ-4-Desaturase-, Δ-12-Desaturase- und ω-3-Desaturase-Aktivität codieren, und/oder den weiteren verwendeten Nukleinsäuren wie den Nukleinsäuren, die für Polypeptide des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) codieren eignen sich vorteilhaft C₁₆-, C₁₈- oder C₂₀-Fettsäuren. Bevorzugt werden die im Verfahren als Substrate umgesetzten Fettsäuren in Form ihrer Acyl-CoA-Ester und/oder ihrer Phospholipid-Ester umgesetzt.

Zur Herstellung der langkettigen PUFAs müssen die mehrfach ungesättigten C₁₈-Fettsäuren zunächst durch die enzymatische Aktivität einer Desaturase zunächst desaturiert und anschließend über eine Elongase um mindestens zwei Kohlenstoffatome verlängert werden. Nach einer Elongationsrunde führt diese Enzymaktivität zu C₂₀-Fettsäuren, und nach zwei Elongationsrunden zu C₂₂-Fettsäuren. Die Aktivität der Verfahren verwendeten Desaturasen und Elongasen führt zu C₂₀- und/oder C₂₂-Fettsäuren mit mindestens vier Doppelbindungen im Fettsäuremolekül, bevorzugt zu C₂₀-Fettsäuren mit mindestens vier Doppelbindungen, besonders bevorzugt zu C₂₀-und/oder C₂₂-Fettsäuren mit mindestens fünf oder sechs Doppelbindungen, ganz besonders bevorzugt mit sechs Doppelbindungen im Molekül. Besonders bevorzugt als Produkte des Verfahrens sind Arachidonsäure, Eicosapentaensäure und/oder Docosahesaensäure. Die C₂₀- und/oder C₂₂-Fettsäuren mit mindestens vier Doppelbindungen in der Fettsäure können durch die erfindungsgemäße enzymatische Aktivität in Form der freien Fettsäure oder in Form der Ester, wie Phospholipide, Glycolipide, Sphingolipide, Phosphoglyceride, Monoacylglycerin, Diacylglycerin oder Triacylglycerin, verlängert werden.

Der bevorzugte Biosyntheseort von Fettsäuren, Ölen, Lipiden oder Fette in den vorteilhaft verwendeten Pflanzen ist beispielsweise im allgemeinen der Samen oder Zellschichten des Samens, so dass eine samenspezifische Expression der im Verfahren verwendeten Nukleinsäuren sinnvoll ist. Es ist jedoch naheliegend, dass die Biosynthese von Fettsäuren, Ölen oder Lipiden nicht auf das Samengewebe beschränkt sein muss, sondern auch in allen übrigen Teilen der Pflanze - beispielsweise in Epidermiszellen oder in den Knollen - gewebespezifisch erfolgen kann.

Durch die Verwendung der erfindungsgemäßen Nukleinsäuren, die für eine Δ-6-Desaturase, eine Δ-6-Elongase, eine Δ-5-Desaturase, eine Δ-12-Desaturase und eine ω-3-Desaturase codieren, können im Verfahren die hergestellten mehrfach ungesättigten Fettsäuren mindestens um 5 %, bevorzugt mindestens um 10 %, besonders bevorzugt mindestens um 20 %, ganz besonders bevorzugt um mindestens 50 % gegenüber der Wildtypflanze, die die Nukleinsäuren nicht rekombinant enthalten, erhöht werden.

Durch das Verfahren können die hergestellten mehrfach ungesättigten Fettsäuren in den im Verfahren verwendeten Pflanzen prinzipiell auf zwei Arten erhöht werden. Es kann vorteilhaft der Pool an freien mehrfach ungesättigten Fettsäuren und/oder der Anteil der über das Verfahren hergestellten veresterten mehrfach ungesättigten Fettsäuren erhöht werden. Vorteilhaft wird durch das Verfahren der Pool an veresterten mehrfach ungesättigten Fettsäuren in den transgenen Pflanzne erhöht.

Die im Verfahren gewonnenen Fettsäuren eignen sich auch als Ausgangsmaterial für die chemische Synthese von weiteren Wertprodukten. Sie können beispielsweise in Kombination miteinander oder allein zur Herstellung von Pharmaka, Nahrungsmittel, Tierfutter oder Kosmetika verwendet werden.

Ein weiterer Gegenstand sind isolierte Nukleinsäuresequenzen, die für Polypeptide mit Δ-6-Desaturaseaktivität codieren, ausgewählt aus der Gruppe:
a) einer Nukleinsäuresequenz mit der in SEQ ID NO: 1 dargestellten Sequenz,
b) Nukleinsäuresequenzen, die für ein Protein mit der in SEQ ID NO: 2 dargestellten Sequenz codieren, oder
c) Derivate der in SEQ ID NO: 1 dargestellten Nukleinsäuresequenz, die für Polypeptide mit mindestens 70 % Identität auf Aminosäureebene mit SEQ ID NO: 2 codieren und eine Δ-6-Desaturaseaktivität aufweisen.

Ein weiterer Gegenstand sind isolierte Nukleinsäuresequenzen, die für Polypeptide mit Δ-6-Elongaseaktivität codiert, ausgewählt aus der Gruppe:
a) einer Nukleinsäuresequenz mit der in SEQ ID NO: 3, SEQ ID NO: 5 oder SEQ ID NO: 7 dargestellten Sequenz,
b) Nukleinsäuresequenzen, die für ein Protein mit der in SEQ ID NO: 4, SEQ ID NO: 6 oder SEQ ID NO: 8 dargestellten Sequenz codieren, oder
c) Derivate der in SEQ ID NO: 3, SEQ ID NO: 5 oder SEQ ID NO: 7 dargestellten Nukleinsäuresequenz, die für Polypeptide mit mindestens 70 % Identität auf Aminosäureebene mit SEQ ID NO: 4, SEQ ID NO: 6 oder SEQ ID NO: 8 codieren und eine Δ-6-Elongaseaktivität aufweisen.

Ein weiterer Gegenstand sind isolierte Nukleinsäuresequenzen, die für Polypeptide mit Δ-5-Desaturaseaktivität codieren, ausgewählt aus der Gruppe:
a) einer Nukleinsäuresequenz mit der in SEQ ID NO: 9 dargestellten Sequenz,
b) Nukleinsäuresequenzen, die für ein Protein mit der in SEQ ID NO: 10 dargestellten Sequenz codieren, oder
c) Derivate der in SEQ ID NO: 9 dargestellten Nukleinsäuresequenz, die für Polypeptide mit mindestens 40 % Identität auf Aminosäureebene mit SEQ ID NO: 10 codieren und eine Δ-5-Desaturaseaktivität aufweisen.

Ein weiterer Gegenstand sind isolierte Nukleinsäuresequenzen, die für Polypeptide mit ω-3-Desaturaseaktivität codieren, ausgewählt aus der Gruppe:
a) einer Nukleinsäuresequenz mit der in SEQ ID NO: 23 dargestellten Sequenz,
b) Nukleinsäuresequenzen, die für ein Protein mit der in SEQ ID NO: 24 dargestellten Sequenz codieren, oder
c) Derivate der in SEQ ID NO: 23 dargestellten Nukleinsäuresequenz, die für Polypeptide mit mindestens 40 % Identität auf Aminosäureebene mit SEQ ID NO: 24 codieren und eine ω-3-Desaturaseaktivität aufweisen.

Ein weiterer Gegenstand sind isolierte Nukleinsäuresequenzen, die für Polypeptide mit Δ-12-Desaturaseaktivität codieren, ausgewählt aus der Gruppe:
a) einer Nukleinsäuresequenz mit der in SEQ ID NO: 25 dargestellten Sequenz, oder
b) Nukleinsäuresequenzen, die für ein Protein mit der in SEQ ID NO: 26 dargestellten Sequenz codieren, oder
c) Derivate der in SEQ ID NO: 25 dargestellten Nukleinsäuresequenz, die für Polypeptide mit mindestens 40 % Identität auf Aminosäureebene mit SEQ ID NO: 26 codieren und eine Δ-12-Desaturasaktivität aufweisen.

Ein weiterer Erfindungsgegenstand sind Genkonstrukte, die die erfindungsgemäßen Nukleinsäuresequenzen SEQ ID NO: 25 enthalten, wobei die Nukleinsäure funktionsfähig mit einem oder mehreren Regulationssignalen verbunden ist. Zusätzlich können weitere Biosynthesegene des Fettsäure- oder Lipidstoffwechsels enthält ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) im Genkonstrukt enthalten sein. Vorteilhaft sind zusätzlich Biosynthesegene des Fettsäure- oder Lipidstoffwechsels ausgewählt aus der Gruppe der Δ-4-Desaturase, Δ-8-Desaturase, Δ-9-Elongase oder Δ-5-Elongase enthalten.

Vorteilhaft stammen alle die im Verfahren verwendeten Nukleinsäuresequenzen aus einem Organismus wie einer Pflanze oder einem Mikroorganismus. Bevorzugt stammen die Nukleinsäuresequenzen aus Algen, wie Ostreococcus, Pilzen wie Phytophtora oder Diatomeen wie Thalassiosira.

Die im Verfahren verwendeten Nukleinsäuresequenzen, die für Proteine mit Δ-6-Desaturase-, Δ-6-Elongase-, Δ-5-Desaturase-, Δ-5-Elongase-, Δ-4-Desaturase-, Δ-12-Desaturase- und/oder ω-3-Desaturaseaktivität codieren, werden vorteilhaft allein oder bevorzugt in Kombination in einer Expressionskassette (= Nukleinsäurekonstrukt), die die Expression der Nukleinsäuren in einer Pflanze ermöglicht, eingebracht. Es kann im Nukleinsäurekonstrukt mehr als eine Nukleinsäuresequenz einer enzymatischen Aktivität wie z.B. einer Δ-6-Desaturase, einer Δ-6-Elongase, einer Δ-5-Desaturase, einer Δ-5-Elongase, einer Δ-4-Desaturase, einer Δ-12-Desaturase und/oder einer ω-3-Desaturase enthalten sein.

Zum Einbringen werden die im Verfahren verwendeten Nukleinsäuren vorteilhaft einer Amplifikation und Ligation in bekannter Weise unterworfen. Vorzugsweise geht man in Anlehnung an das Protokoll der Pfu-DNA-Polymerase oder eines Pfu/Taq-DNA-Polymerasegemisches vor. Die Primer werden in Anlehnung an die zu amplifizierende Sequenz gewählt. Zweckmäßigerweise sollten der/die Primer so gewählt werden, dass das Amplifikat die gesamte kodogene Sequenz vom Start- bis zum Stop-Kodon umfasst. Im Anschluss an die Amplifikation wird das Amplifikat zweckmäßigerweise analysiert. Beispielsweise kann die Analyse nach gelelektrophoretischer Auftrennung hinsichtlich Qualität und Quantität erfolgen. Im Anschluss kann das Amplifikat nach einem Standardprotokoll gereinigt werden (z.B. Qiagen). Ein Aliquot des gereinigten Amplifikats steht dann für die nachfolgende Klonierung zur Verfügung. Geeignete Klonierungsvektoren sind dem Fachmann allgemein bekannt. Hierzu gehören insbesondere Vektoren, die in mikrobiellen Systemen replizierbar sind, also vor allem Vektoren, die eine effiziente Klonierung in Hefen oder Pilze gewährleisten, und die stabile Transformation von Pflanzen ermöglichen. Zu nennen sind insbesondere verschiedene für die T-DNA-vermittelte Transformation geeignete, binäre und cointegrierte Vektorsysteme. Derartige Vektorsysteme sind in der Regel dadurch gekennzeichnet, dass sie zumindest die für die Agrobakterium-vermittelte Transformation benötigten vir-Gene sowie die T-DNA begrenzenden Sequenzen (T-DNA-Border) beinhalten. Vorzugsweise umfassen diese Vektorsysteme auch weitere cisregulatorische Regionen wie Promotoren und Terminatoren und/oder Selektionsmarker, mit denen entsprechend transformierte Organismen identifiziert werden können. Während bei co-integrierten Vektorsystemen vir-Gene und T-DNA-Sequenzen auf demselben Vektor angeordnet sind, basieren binäre Systeme auf wenigstens zwei Vektoren, von denen einer vir-Gene, aber keine T-DNA und ein zweiter T-DNA, jedoch kein vir-Gen trägt. Dadurch sind letztere Vektoren relativ klein, leicht zu manipulieren und sowohl in E.-coli als auch in Agrobacterium zu replizieren. Zu diesen binären Vektoren gehören Vektoren der Serien pBIB-HYG, pPZP, pBecks, pGreen. Erfindungsgemäß bevorzugt verwendet werden Bin19, pBI101, pBinAR, pGPTV und pCAMBIA. Eine Übersicht über binäre Vektoren und ihre Verwendung gibt Hellens et al, Trends in Plant Science (2000) 5, 446-451. Für die Vektorpräparation können die Vektoren zunächst mit Restriktionsendonuklease(n) linearisiert und dann in geeigneter Weise enzymatisch modifiziert werden. Im Anschluss wird der Vektor gereinigt und ein Aliquot für die Klonierung eingesetzt. Bei der Klonierung wird das enzymatisch geschnittenen und erforderlichenfalls gereinigten Amplifikat mit ähnlich präparierten Vektorfragmenten mit Einsatz von Ligase kloniert. Dabei kann ein bestimmtes Nukleinsäurekonstrukt bzw. Vektor- oder Plasmidkonstrukt einen oder auch mehrere kodogene Genabschnitte aufweisen. Vorzugsweise sind die kodogenen Genabschnitte in diesen Konstrukten mit regulatorischen Sequenzen funktional verknüpft. Zu den regulatorischen Sequenzen gehören insbesondere pflanzliche Sequenzen wie die oben beschriebenen Promotoren und Terminatoren. Die Konstrukte lassen sich vorteilhafterweise in Mikroorganismen, insbesondere Escherichia coli und Agrobacterium tumefaciens, unter selektiven Bedingungen stabil propagieren und ermöglichen einen Transfer von heterologer DNA in Pflanzen oder Mikroorganismen.

Unter der vorteilhaften Verwendung von Klonierungsvektoren können die im Verfahren verwendeten Nukleinsäuren, die erfinderischen Nukleinsäuren und Nukleinsäurekonstrukte in Organismen wie Mikroorganismen oder vorteilhaft Pflanzen eingebracht werden und damit bei der Pflanzentransformation verwendet werden, wie denjenigen, die veröffentlicht sind in und dort zitiert sind: Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus, Annu. Rev. Plant Physiol. Plant Molec. Biol. 42 (1991), 205-225)). Die im Verfahren verwendeten Nukleinsäuren, die erfinderischen Nukleinsäuren und Nukleinsäurekonstrukte und/oder Vektoren lassen sich damit zur gentechnologischen Veränderung eines breiten Spektrums an Organismen vorteilhaft an Pflanzen verwenden, so dass diese bessere und/oder effizientere Produzenten von PUFAs werden.

Es gibt eine Reihe von Mechanismen, durch die eine Veränderung des Δ-6-Desaturase-, Δ-6-Elongase-, Δ-5-Desaturase-, Δ-12-Desaturase- und ω-3-Desaturase-Proteins sowie der weiteren im Verfahren verwendeten Proteine wie die Δ-5-Elongase-oder Δ-4-Desaturase-Proteine möglich ist, so dass die Ausbeute, Produktion und/oder Effizienz der Produktion der vorteilhaft mehrfach ungesättigten Fettsäuren in einer Pflanze bevorzugt in einer Ölfruchtpflanze aufgrund dieses veränderten Proteins direkt beeinflusst werden kann. Die Anzahl oder Aktivität der Δ-6-Desaturase-, Δ-6-Elongase-, Δ-5-Desaturase-, Δ-5-Elongase-, Δ-4-Desaturase-, Δ-12-Desaturase- und ω-3-Desaturase-Proteine oder -Gene kann erhöht werden, so dass größere Mengen der Genprodukte und damit letztlich größere Mengen der im Verfahren hergestellten mehrfach ungesättigten Fettsäuren mit mindestens vier Doppelbindungen hergestellt werden. Auch eine de novo Synthese in einer Pflanze, der die Aktivität und Fähigkeit zur Biosynthese der Verbindungen vor dem Einbringen des/der entsprechenden Gens/Gene fehlte, ist möglich. Entsprechendes gilt für die Kombination mit weiteren Desaturasen oder Elongasen oder weiteren Enzymen aus dem Fettsäure- und Lipidstoffwechsel. Auch die Verwendung verschiedener divergenter, d.h. auf DNA-Sequenzebene unterschiedlicher Sequenzen kann dabei vorteilhaft sein bzw. die Verwendung von Promotoren zur Genexpression, die eine andere zeitliche Genexpression z.B. abhängig vom Reifegrad eines Samens oder Öl-speichernden Gewebes ermöglicht.

Durch das Einbringen eines Δ-6-Desaturase-, eines Δ-6-Elongase-, eines Δ-5-Desaturase-, eines Δ-5-Elongase-, eines Δ-4-Desaturase-, eines Δ-12-Desaturase-und/oder eines Δ-3-Desaturase-Genes in einer Pflanze allein oder in Kombination mit anderen Genen in eine Zelle kann nicht nur den Biosynthesefluss zum Endprodukt erhöht, sondern auch die entsprechende Triacylglycerin-Zusammensetzung erhöht oder de novo geschaffen werden. Ebenso kann die Anzahl oder Aktivität anderer Gene, die am Import von Nährstoffen, die zur Biosynthese einer oder mehrerer Fettsäuren, Ölen, polaren und/oder neutralen Lipiden nötig sind, erhöht sein, so dass die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PUFAs, wie im folgenden beschrieben, weiter gesteigert wird. Durch Optimierung der Aktivität oder Erhöhung der Anzahl einer oder mehrerer Δ-6-Desaturase-, Δ-6-Elongase-, Δ-5-Desaturase-, Δ-5-Elongase-, Δ-4-Desaturase-, Δ-12-Desaturase- und/oder ω-3-Desaturase-Gene, die an der Biosynthese dieser Verbindungen beteiligt sind, oder durch Zerstören der Aktivität einer oder mehrerer Gene, die am Abbau dieser Verbindungen beteiligt sind, kann es möglich sein, die Ausbeute, Produktion und/oder Effizienz der Produktion von Fettsäure- und Lipidmolekülen aus Pflanzen zu steigern.

Die im Verfahren verwendeten isolierten Nukleinsäuremoleküle codieren für Proteine oder Teile von diesen, wobei die Proteine oder das einzelne Protein oder Teile davon eine Aminosäuresequenz enthält, die ausreichend homolog zu einer Aminosäuresequenz ist, die in den Sequenzen SEQ ID NO: 2, SEQ ID NO: 4, SEQ ID NO:6, SEQ ID NO: 8, SEQ ID NO:10, SEQ ID NO: 12, SEQ ID NO: 14, SEQ ID NO: 16, SEQ ID NO: 18, SEQ ID NO: 20, SEQ ID NO: 22, SEQ ID NO: 24 oder SEQ ID NO: 26 dargestellt ist, so dass die Proteine oder Teile davon noch eine Δ-6-Desaturase-, Δ-6-Elongase-, Δ-5-Desaturase-, Δ-5-Elongase-, Δ-4-Desaturase-, Δ-12-Desaturase- und/oder ω-3-Desaturase-Aktivität aufweisen. Vorzugsweise haben die Proteine oder Teile davon, die von dem Nukleinsäuremolekül/den Nukleinsäuremolekülen kodiert wird/werden, noch seine wesentliche enzymatische Aktivität und die Fähigkeit, am Stoffwechsel von zum Aufbau von Zellmembranen oder Lipidkörperchen in Pflanzen notwendigen Verbindungen oder am Transport von Molekülen über diese Membranen teilzunehmen. Vorteilhaft sind die von den Nukleinsäuremolekülen kodierten Proteine zu mindestens etwa 40 %, vorzugsweise mindestens etwa 50% oder 60 % und stärker bevorzugt mindestens etwa 70 %, 80 % oder 90 % und am stärksten bevorzugt mindestens etwa 85 %, 86 %, 87 %, 88 %, 89 %, 90 %, 91 %, 92 %, 93 %, 94 %, 95 %, 96 %, 97 %, 98 %, 99 % oder mehr identisch zu den in SEQ ID NO: 2, SEQ ID NO: 4, SEQ ID NO:6, SEQ ID NO: 8, SEQ ID NO:10, SEQ ID NO: 12, SEQ ID NO: 14, SEQ ID NO: 16, SEQ ID NO: 18, SEQ ID NO: 20, SEQ ID NO: 22, SEQ ID NO: 24 oder SEQ ID NO: 26 dargestellten Aminosäuresequenzen. Im Sinne der Erfindung ist unter Homologie oder homolog, Identität oder identisch zu verstehen.

Die Homologie wurde über den gesamten Aminosäure- bzw. Nukleinsäuresequenzbereich berechnet. Für das Vergleichen verschiedener Sequenzen stehen dem Fachmann eine Reihe von Programmen, die auf verschiedenen Algorithmen beruhen zur Verfügung. Dabei liefern die Algorithmen von Needleman und Wunsch oder Smith und Waterman besonders zuverlässige Ergebnisse. Für die Sequenzvergleiche wurde das Programm PileUp verwendet (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm GAP über den gesamten Sequenzbereich mit folgenden Einstellungen ermittelt: Gap Weight: 50, Length Weight: 3, Average Match: 10.000 und Average Mismatch: 0.000. Diese Einstellungen wurden, falls nicht anders angegeben, immer als Standardeinstellungen für Sequenzvergleiche verwendet wurden.

Unter wesentlicher enzymatischer Aktivität der im Verfahren verwendeten Δ-6-Desaturase, Δ-6-Elongase, Δ-5-Desaturasen, Δ-5-Elongase, Δ-4-Desaturasen, Δ-12-Desaturase und/oder ω-3-Desaturase ist zu verstehen, dass sie gegenüber den durch die Sequenz mit SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 und deren Derivate codierten Proteinen/Enzymen im Vergleich noch mindestens eine enzymatische Aktivität von mindestens 10 %, bevorzugt 20 %, besonders bevorzugt 30 % und ganz besonders 40 % aufweisen und damit am Stoffwechsel von zum Aufbau von Fettsäuren, Fettsäureester wie Diacylglyceride und/oder Triacylglyceride in einer Pflanze oder Pflanzenzel-le notwendigen Verbindungen oder am Transport von Molekülen über Membranen teilnehmen können, wobei C₂₀- oder C₂₂-Kohlenstoffketten im Fettsäuremolekül mit Doppelbindungen an mindestens vier, fünf oder sechs Stellen gemeint sind.

Vorteilhaft im Verfahren verwendbare Nukleinsäuren stammen aus Bakterien, Pilzen, Diatomeen, Tieren wie Caenorhabditis oder Oncorhynchus oder Pflanzen wie Algen oder Moosen wie den Gattungen Shewanella, Physcomitrella, Thraustochytrium, Fusarium, Phytophthora, Ceratodon, Mantoniella, Ostreococcus, Isochrysis, Aleurita, Muscarioides, Mortierella, Borago, Phaeodactylum, Crypthecodinium, speziell aus den Gattungen und Arten Oncorhynchus mykiss, Thalassiosira pseudonona, Mantoniella squamata, Ostreococcus sp., Ostreococcus tauri, Euglena gracilis, Physcomitrella patens, Phytophtora infestans, Phytophtora sojae, Fusarium graminaeum, Cryptocodinium cohnii, Ceratodon purpureus, Isochrysis galbana, Aleurita farinosa, Thraustochytrium sp., Muscarioides viallii, Mortierella alpina, Borago officinalis, Phaeodactylum tricornutum, Caenorhabditis elegans oder besonders vorteilhaft aus Oncorhynchus mykiss, Thalassiosira pseudonona oder Crypthecodinium cohnii.

Alternativ können im Verfahren Nukleotidsequenzen verwendet werden, die für eine Δ-6-Desaturase, Δ-6-Elongase, Δ-5-Desaturase, Δ-5-Elongase, Δ-4-Desaturase, Δ-12-Desaturase und/oder ω-3-Desaturase codieren und die an eine Nukleotidsequenz, wie in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 dargestellt, vorteilhaft unter stringenten Bedingungen hybridisieren.

Die im Verfahren verwendeten Nukleinsäuresequenzen werden vorteilhaft in einer Expressionskassette, die die Expression der Nukleinsäuren in Organismen wie Mikroorganismen oder Pflanzen ermöglicht, eingebracht.

Dabei werden die Nukleinsäuresequenzen, die für die Δ-6-Desaturase, Δ-6-Elongase, Δ-5-Desaturase, Δ-5-Elongase, Δ-4-Desaturase, Δ-12-Desaturase und/oder ω-3-Desaturase codieren, mit einem oder mehreren Regulationssignalen vorteilhafterweise zur Erhöhung der Genexpression funktionell verknüpft. Diese regulatorischen Sequenzen sollen die gezielte Expression der Gene und der Proteinexpression ermöglichen. Dies kann beispielsweise je nach Wirtspflanze bedeuten, dass das Gen erst nach Induktion exprimiert und/oder überexprimiert wird, oder dass es sofort exprimiert und/oder überexprimiert wird. Beispielsweise handelt es sich bei diesen regulatorischen Sequenzen um Sequenzen an die Induktoren oder Repressoren binden und so die Expression der Nukleinsäure regulieren. Zusätzlich zu diesen neuen Regulationssequenzen oder anstelle dieser Sequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Die Expressionskassette (= Expressionskonstrukt = Genkonstrukt) kann aber auch einfacher aufgebaut sein, das heißt es wurden keine zusätzlichen Regulationssignale vor die Nukleinsäuresequenz oder dessen Derivate inseriert und der natürliche Promotor mit seiner Regulation wurde nicht entfernt. Stattdessen wurde die natürliche Regulationssequenz so mutiert, dass keine Regulation mehr erfolgt und/oder die Genexpression gesteigert wird. Diese veränderten Promotoren können in Form von Teilsequenzen (= Promotor mit Teilen der erfindungsgemäßen Nukleinsäuresequenzen) auch allein vor das natürliche Gen zur Steigerung der Aktivität gebracht werden. Das Genkonstrukt kann außerdem vorteilhafterweise auch eine oder mehrere sogenannte "enhancer Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden wie weitere regulatorische Elemente oder Terminatoren. Die Δ-6-Desaturase-, Δ-6-Elongase-, Δ-5-Desaturase-, Δ-5-Elongase-, Δ-4-Desaturase-, Δ-12-Desaturase- und/oder ω-3-Desaturase-Gene können in einer oder mehreren Kopien in der Expressionskassette (= Genkonstrukt) enthalten sein. Vorteilhaft liegt nur jeweils eine Kopie der Gene in der Expressionskassette vor. Dieses Genkonstrukt oder die Genkonstrukte können zusammen in der Wirtspflanze exprimiert werden. Dabei kann das Genkonstrukt oder die Genkonstrukte in einem oder mehreren Vektoren inseriert sein und frei in der Zelle vorliegen oder aber im Genom inseriert sein. Es ist vorteilhaft für die Insertion weiterer Gene im Wirtsgenom, wenn die zu exprimierenden Gene zusammen in einem Genkonstrukt vorliegen.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Eine weitere Ausführungsform der Erfindung sind ein oder mehrere Genkonstrukte. Genkonstrukte können eine oder mehrere Sequenzen enthalten, die durch SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 oder dessen Derivate definiert sind und für Polypeptide gemäß SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24 oder SEQ ID NO: 26 kodieren. Die genannten Δ-6-Desaturase-, Δ-6-Elongase-, Δ-5-Desaturase-, Δ-5-Elongase-, Δ-4-Desaturase-, Δ-12-Desaturase- und/oder ω-3-Desaturase-Proteine führen dabei vorteilhaft zu einer Desaturierung oder Elongierung von Fettsäuren, wobei das Substrat vorteilhaft ein, zwei, drei, vier, fünf oder sechs Doppelbindungen aufweist und vorteilhaft 18, 20 oder 22 Kohlenstoffatome im Fettsäuremolekül aufweist. Gleiches gilt für ihre Homologen, Derivate oder Analoga, die funktionsfähig mit einem oder mehreren Regulationssignalen, vorteilhafterweise zur Steigerung der Genexpression, verbunden sind.

Vorteilhafte Regulationssequenzen, die für die Herstellung der im Verfahren verwendeten Nukleinsäuresequenzen und ihr anschließendes Einbringen in Pflanzen verwendet werden können, liegen beispielsweise in Promotoren vor, wie dem cos-, tac-, trp-, tet-, trp-tet-, Ipp-, lac-, Ipp-lac-, laclq-, T7-, T5-, T3-, gal-, trc-, ara-, SP6-, λ-PR- oder λ-PL-Promotor und werden vorteilhafterweise in Gram-negativen Bakterien angewendet. Weitere vorteilhafte Regulationssequenzen liegen beispielsweise in den Grampositiven Promotoren amy und SPO2, in den Hefe- oder Pilzpromotoren ADC1, MFα, AC, P-60, CYC1, GAPDH, TEF, rp28, ADH oder in den Pflanzenpromotoren CaMV/35S [Franck et al., Cell 21 (1980) 285-294], PRP1 [Ward et al., Plant. Mol. Biol. 22 (1993)], SSU, OCS, lib4, usp, STLS1, B33, nos oder im Ubiquitin- oder Phaseolin-Promotor vor. In diesem Zusammenhang vorteilhaft sind ebenfalls induzierbare Promotoren, wie die in EP-A-0 388 186 (Benzylsulfonamid-induzierbar), Plant J. 2, 1992:397-404 (Gatz et al., Tetracyclin-induzierbar), EP-A-0 335 528 (Abzisinsäureinduzierbar) oder WO 93/21334 (Ethanol- oder Cyclohexenol-induzierbar) beschriebenen Promotoren. Weitere geeignete Pflanzenpromotoren sind der Promotor von cytosolischer FBPase oder der ST-LSI-Promotor der Kartoffel (Stockhaus et al., EMBO J. 8, 1989, 2445), der Phosphoribosylpyrophosphatamidotransferase-Promotor aus Glycine max (Genbank-Zugangsnr. U87999) oder der in EP-A-0 249 676 beschriebene nodienspezifische Promotor. Besonders vorteilhafte Promotoren sind Promotoren, welche die Expression in Geweben ermöglichen, die an der Fettsäurebiosynthese beteiligt sind. Ganz besonders vorteilhaft sind samenspezifische Promotoren, wie der ausführungsgemäße USP Promotor aber auch andere Promotoren wie der LeB4-, DC3, Phaseolin- oder Napin-Promotor. Weitere besonders vorteilhafte Promotoren sind samenspezifische Promotoren, die für monokotyle oder dikotyle Pflanzen verwendet werden können und in US 5,608,152 (Napin-Promotor aus Raps), WO 98/45461 (Oleosin-Promotor aus Arobidopsis), US 5,504,200 (Phaseolin-Promotor aus Phaseolus vulgaris), WO 91/13980 (Bce4-Promotor aus Brassica), von Baeumlein et al., Plant J., 2, 2, 1992:233-239 (LeB4-Promotor aus einer Leguminose) beschrieben sind, wobei sich diese Promotoren für Dikotyledonen eignen. Die folgenden Promotoren eignen sich beispielsweise für Monokotyledonen Ipt-2- oder Ipt-1-Promotor aus Gerste (WO 95/15389 und WO 95/23230), Hordein-Promotor aus Gerste und andere, in WO 99/16890 beschriebene geeignete Promotoren.

Es ist im Prinzip möglich, alle natürlichen Promotoren mit ihren Regulationssequenzen, wie die oben genannten, für das neue Verfahren zu verwenden. Es ist ebenfalls möglich und vorteilhaft, zusätzlich oder alleine synthetische Promotoren zu verwenden, besonders wenn sie eine Samen-spezifische Expression vermitteln, wie z.B. beschrieben in WO 99/16890.

Um einen besonders hohen Gehalt an PUFAs vor in transgenen Pflanzen zu erzielen, sollten die PUFA-Biosynthesegene vorteilhaft samenspezifisch in Ölsaaten exprimiert werden. Hierzu können Samen-spezifische Promotoren verwendet werden, bzw. solche Promotoren die im Embryo und/oder im Endosperm aktiv sind. Samenspezifische Promotoren können prinzipiell sowohl aus dikotolydonen als auch aus monokotolydonen Pflanzen isoliert werden. Im folgenden sind vorteilhafte bevorzugte Promotoren aufgeführt: USP (= unknown seed protein) und Vicilin (Vicia faba) [Bäumlein et al., Mol. Gen Genet., 1991, 225(3)], Napin (Raps) [US 5,608,152], Acyl-Carrier Protein (Raps) [US 5,315,001 und WO 92/18634], Oleosin (Arabidopsis thaliana) [WO 98/45461 und WO 93/20216], Phaseolin (Phaseolus vulgaris) [US 5,504,200], Bce4 [WO 91/13980], Leguminosen B4 (LegB4-Promotor) [Bäumlein et al., Plant J., 2,2, 1992], Lpt2 und Ipt1 (Gerste) [WO 95/15389 u. WO95/23230], Samen-spezifische Promotoren aus Reis, Mais u. Weizen [WO 99/16890], Amy32b, Amy 6-6 und Aleurain [US 5,677,474], Bce4 (Raps) [US 5,530,149], Glycinin (Soja) [EP 571 741], Phosphoenol-Pyruvatcarboxylase (Soja) [JP 06/62870], ADR12-2 (Soja) [WO 98/08962], Isocitratlyase (Raps) [US 5,689,040] oder α-Amylase (Gerste) [EP 781 849].

Die Pflanzengenexpression lässt sich auch über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Um eine stabile Integration der Biosynthesegene in die transgene Pflanze über mehrere Generation sicherzustellen, sollte jede der im Verfahren verwendeten Nukleinsäuren, die für die Δ-6-Desaturase, Δ-6-Elongase, Δ-5-Desaturase, Δ-5-Elongase, Δ-4-Desaturase, Δ-12-Desaturase und/oder ω-3-Desaturase codieren, unter der Kontrolle eines eigenen bevorzugt eines unterschiedlichen Promotors exprimiert werden, da sich wiederholende Sequenzmotive zu Instabilität der T-DNA bzw. zu Rekombinationsereignissen führen können. Die Expressionskassette ist dabei vorteilhaft so aufgebaut, dass einem Promotor eine geeignete Schnittstelle zur Insertion der zu exprimierenden Nukleinsäure folgt vorteilhaft in einem Polylinker anschließend gegebenenfalls ein Terminator hinter dem Polylinker liegt. Diese Abfolge wiederholt sich mehrfach bevorzugt drei-, vier- oder fünfmal, so dass bis zu fünf Gene in einem Konstrukt zusammengeführt werden und so zur Expression in die transgene Pflanze eingebracht werden können. Vorteilhaft wiederholt sich die Abfolge bis zu dreimal oder falls erfoderlich noch mehrmals. Die Nukleinsäuresequenzen werden zur Expression über die geeignete Schnittstelle beispielsweise im Polylinker hinter den Promotor inseriert. Vorteilhaft hat jede Nukleinsäuresequenz ihren eigenen Promotor und gegebenenfalls ihren eigenen Terminator. Derartige vorteilhafte Konstrukte werden beispielsweise in DE 10102337 oder DE 10102338 offenbart. Es ist aber auch möglich mehrere Nukleinsäuresequenzen hinter einem Promotor und ggf. vor einem Terminator zu inserieren. Dabei ist die Insertionsstelle bzw. die Abfolge der inserierten Nukleinsäuren in der Expressionskassette nicht von entscheidender Bedeutung, das heißt eine Nukleinsäuresequenz kann an erster oder letzter Stelle in der Kassette inseriert sein, ohne dass dadurch die Expression wesentlich beeinflusst wird. Es können in der Expressionskassette vorteilhaft unterschiedliche Promotoren wie beispielsweise der USP-, LegB4 oder DC3-Promotor und unterschiedliche Terminatoren verwendet werden. Es ist aber auch möglich nur einen Promotortyp in der Kassette zu verwenden. Dies kann jedoch zu unerwünschten Rekombinationsereignissen führen.

Wie oben beschrieben sollte die Transkription der eingebrachten Gene vorteilhaft durch geeignete Terminatoren am 3'-Ende der eingebrachten Biosynthesegene (hinter dem Stoppcodon) abgebrochen werden. Verwendet werden kann hier z.B. der OCS1 Terminator. Wie auch für die Promotoren, so sollten hier für jedes Gen unterschiedliche Terminatorsequenzen verwendet werden.

Das Genkonstrukt kann, wie oben beschrieben, auch weitere Gene umfassen, die in die Organismen eingebracht werden sollen. Es ist möglich und vorteilhaft, in die Wirtspflanzen Regulationsgene, wie Gene für Induktoren, Repressoren oder Enzyme, welche durch ihre Enzymaktivität in die Regulation eines oder mehrerer Gene eines Biosynthesewegs eingreifen, einzubringen und darin zu exprimieren. Diese Gene können heterologen oder homologen Ursprungs sein. Weiterhin können vorteilhaft im Nukleinsäurekonstrukt bzw. Genkonstrukt weitere Biosynthesegene des Fettsäure- oder Lipidstoffwechsels enthalten sein oder aber diese Gene können auf einem weiteren oder mehreren weiteren Nukleinsäurekonstrukten liegen. Vorteilhaft werden als Biosynthesegene des Fettsäure- oder Lipidstoffwechsels ein Gen ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) oder deren Kombinationen verwendet. Besonders vorteilhafte Nukleinsäuresequenzen sind Biosynthesegene des Fettsäure- oder Lipidstoffwechsels ausgewählt aus der Gruppe der Acyl-CoA:Lysophospholipid-Acyltransferase, Δ-4-Desaturase, Δ-8-Desaturase, Δ-5-Elongase und/oder Δ-9-Elongase.

Dabei können die vorgenannten Nukleinsäuren bzw. Gene in Kombination mit anderen Elongasen und Desaturasen in Expressionskassetten, wie den vorgenannten, kloniert werden und zur Transformation von Pflanzen Mithilfe von Agrobakterium eingesetzt werden.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird. Die Expressionskassetten können prinzipiell direkt zum Einbringen in die Pflanze verwendet werden oder aber in einen Vektoren eingebracht werden.

Diese vorteilhaften Vektoren, vorzugsweise Expressionsvektoren, enthalten die im Verfahren verwendeten Nukleinsäuren, die für die Δ-6-Desaturase, Δ-6-Elongase, Δ-5-Desaturase, Δ-5-Elongase, Δ-4-Desaturase, Δ-12-Desaturase und/oder ω-3-Desaturase codieren, oder ein Nukleinsäurekonstrukt, die die verwendeten Nukleinsäure allein oder in Kombination mit weiteren Biosynthesegenen des Fettsäure- oder Lipidstoffwechsels wie den Acyl-CoA:Lysophospholipid-Acyltransferasen, Δ-4-Desaturasen, Δ-8-Desaturasen, Δ-5-Elongasen und/oder Δ-9-Elongasen. Wie hier verwendet, betrifft der Begriff "Vektor" ein Nukleinsäuremolekül, das eine andere Nukleinsäure transportieren kann, an welche es gebunden ist. Ein Vektortyp ist ein "Plasmid", was für eine zirkuläre doppelsträngige DNA-Schleife steht, in die zusätzlichen DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung). Andere Vektoren werden vorteilhaft beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Gewöhnlich haben Expressionsvektoren, die für DNA-Rekombinationstechniken geeignet sind, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch diese anderen Expressionsvektorformen, wie virale Vektoren, die ähnliche Funktionen ausüben, umfassen. Ferner soll der Begriff Vektor auch andere Vektoren, die dem Fachmann bekannt sind, wie Phagen, Viren, wie SV40, CMV, TMV, Transposons, IS-Elemente, Phasmide, Phagemide, Cosmide, lineare oder zirkuläre DNA, umfassen.

Die im Verfahren vorteilhaft verwendeten rekombinanten Expressionsvektoren umfassen die unten beschriebenen Nukleinsäuren oder das oben beschriebene Genkonstrukt in einer Form, die sich zur Expression der verwendeten Nukleinsäuren in einer Wirtszelle eignen, was bedeutet, dass die rekombinanten Expressionsvektoren eine oder mehrere Regulationssequenzen, ausgewählt auf der Basis der zur Expression zu verwendenden Wirtszellen, die mit der zu exprimierenden Nukleinsäuresequenz funktionsfähig verbunden ist, umfasst. In einem rekombinanten Expressionsvektor bedeutet "funktionsfähig verbunden", dass die Nukleotidsequenz von Interesse derart an die Regulationssequenz(en) gebunden ist, dass die Expression der Nukleotidsequenz möglich ist und sie aneinander gebunden sind, so dass beide Sequenzen die vorhergesagte, der Sequenz zugeschriebene Funktion erfüllen (z.B. in einem In-vitro-Transkriptions-/Translationssystem oder in einer Wirtszelle, wenn der Vektor in die Wirtszelle eingebracht wird). Der Begriff "Regulationssequenz" soll Promotoren, Enhancer und andere Expressionskontrollelemente (z.B. Polyadenylierungssignale) umfassen. Diese Regulationssequenzen sind z.B. beschrieben in Goeddel: Gene Expression Technology: Methods in Enzymology 185, Academic Press, San Diego, CA (1990), oder siehe: Gruber und Crosby, in: Methods in Plant Molecular Biology and Biotechnolgy, CRC Press, Boca Raton, Florida, Hrsgb.: Glick und Thompson, Kapitel 7, 89-108, einschließlich der Literaturstellen darin. Regulationssequenzen umfassen solche, welche die konstitutive Expression einer Nukleotidsequenz in vielen Wirtszelltypen steuern, und solche, welche die direkte Expression der Nukleotidsequenz nur in bestimmten Wirtszellen unter bestimmten Bedingungen steuern. Der Fachmann weiß, dass die Gestaltung des Expressionsvektors von Faktoren, wie der Auswahl der zu transformierenden Wirtszelle, dem Ausmaß der Expression des gewünschten Proteins usw., abhängen kann.

Die verwendeten rekombinanten Expressionsvektoren können zur Expression von Δ-6-Desaturasen, Δ-6-Elongasen, Δ-5-Desaturasen, Δ-5-Elongasen, Δ-4-Desaturasen, Δ-12-Desaturasen und/oder ω-3-Desaturasen in prokaryotischen oder eukaryotischen Zellen gestaltet sein. Dies ist vorteilhaft, da häufig Zwischenschritte der Vektorkonstruktion der Einfachheithalber in Mikroorganismen durchgeführt werden. Beispielsweise können die Δ-6-Desaturase-, Δ-6-Elongase-, Δ-5-Desaturase-, Δ-5-Elongase-, Δ-4-Desaturase-, Δ-12-Desaturase- und/oder ω-3-Desaturase-Gene in bakteriellen Zellen, Insektenzellen (unter Verwendung von Baculovirus-Expressionsvektoren), Hefe- und anderen Pilzzellen (siehe Romanos, M.A., et al. (1992) "Foreign gene expression in yeast: a review", Yeast 8:423-488; van den Hondel, C.A.M.J.J., et al. (1991) "Heterologous gene expression in filamentous fungi", in: More Gene Manipulations in Fungi, J.W. Bennet & L.L. Lasure, Hrsgb., S. 396-428: Academic Press: San Diego; und van den Hondel, C.A.M.J.J., & Punt, P.J. (1991) "Gene transfer systems and vector development for filamentous fungi, in: Applied Molecular Genetics of Fungi, Peberdy, J.F., et al., Hrsgb., S. 1-28, Cambridge University Press: Cambridge), Algen (Falciatore et al., 1999, Marine Biotechnology.1, 3:239-251), mit Vektoren nach einem Transformationsverfahren, wie beschrieben in WO 98/01572, sowie bevorzugt in Zellen vielzelliger Pflanzen (siehe Schmidt, R. und Willmitzer, L. (1988) "High efficiency Agrobacterium tumefaciens-mediated transformation of Arabidopsis thaliana leaf and cotyledon explants" Plant Cell Rep.:583-586; Plant Molecular Biology and Biotechnology, C Press, Boca Raton, Florida, Kapitel 6/7, S.71-119 (1993); F.F. White, B. Jenes et al., Techniques for Gene Transfer, in: Transgenic Plants, Bd. 1, Engineering and Utilization, Hrsgb.: Kung und R. Wu, Academic Press (1993), 128-43; Potrykus, Annu. Rev. Plant Physiol. Plant Molec. Biol. 42 (1991), 205-225 (und darin zitierte Literaturstellen)) exprimiert werden. Geeignete Wirtszellen werden ferner erörtert in Goeddel, Gene Expression Technology: Methods in Enzymology 185, Academic Press, San Diego, CA (1990). Der rekombinante Expressionsvektor kann alternativ, zum Beispiel unter Verwendung von T7-Promotor-Regulationssequenzen und T7-Polymerase, in vitro transkribiert und translatiert werden.

Die Expression von Proteinen in Prokaryoten erfolgt meist mit Vektoren, die konstitutive oder induzierbare Promotoren enthalten, welche die Expression von Fusions- oder nicht-Fusionsproteinen steuern. Typische Fusions-Expressionsvektoren sind u.a. pGEX (Pharmacia Biotech Inc; Smith, D.B., und Johnson, K.S. (1988) Gene 67:31-40), pMAL (New England Biolabs, Beverly, MA) und pRIT5 (Pharmacia, Piscataway, NJ), bei denen Glutathion-S-Transferase (GST), Maltose E-bindendes Protein bzw. Protein A an das rekombinante Zielprotein fusioniert wird.

Beispiele für geeignete induzierbare nicht-Fusions-E. coli-Expressionsvektoren sind u.a. pTrc (Amann et al. (1988) Gene 69:301-315) und pET 11 d (Studier et al., Gene Expression Technology: Methods in Enzymology 185, Academic Press, San Diego, Kalifornien (1990) 60-89). Die Zielgenexpression vom pTrc-Vektor beruht auf der Transkription durch Wirts-RNA-Polymerase von einem Hybrid-trp-lac-Fusionspromotor. Die Zielgenexpression aus dem pET 11 d-Vektor beruht auf der Transkription von einem T7-gn10-lac-Fusions-Promotor, die von einer coexprimierten viralen RNA-Polymerase (T7 gn1) vermittelt wird. Diese virale Polymerase wird von den Wirtsstämmen BL21 (DE3) oder HMS174 (DE3) von einem residenten λ-Prophagen bereitgestellt, der ein T7 gn1-Gen unter der Transkriptionskontrolle des lacUV 5-Promotors birgt.

Andere in prokaryotischen Organismen geeignete Vektoren sind dem Fachmann bekannt, diese Vektoren sind beispielsweise in E. coli pLG338, pACYC184, die pBR-Reihe, wie pBR322, die pUC-Reihe, wie pUC18 oder pUC19, die M113mp-Reihe, pKC30, pRep4, pHS1, pHS2, pPLc236, pMBL24, pLG200, pUR290, pIN-III113-B1, λgt11 or pBdCI, in Streptomyces pIJ101, pIJ364, pIJ702 oder pIJ361, in Bacillus pUB110, pC194 oder pBD214, in Corynebacterium pSA77 oder pAJ667.

Bei einer weiteren Ausführungsform ist der Expressionsvektor ein Hefe-Expressionsvektor. Beispiele für Vektoren zur Expression in der Hefe S. cerevisiae umfassen pYeDesaturasec1 (Baldari et al. (1987) Embo J. 6:229-234), pMFa (Kurjan und Herskowitz (1982) Cell 30:933-943), pJRY88 (Schultz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie den filamentösen Pilzen, eignen, umfassen diejenigen, die eingehend beschrieben sind in: van den Hondel, C.A.M.J.J., & Punt, P.J. (1991) "Gene transfer systems and vector development for filamentous fungi, in: Applied Molecular Genetics of fungi, J.F. Peberdy et al., Hrsgb., S. 1-28, Cambridge University Press: Cambridge, oder in: More Gene Manipulations in Fungi [J.W. Bennet & L.L. Lasure, Hrsgb., S. 396-428: Academic Press: San Diego]. Weitere geeignete Hefevektoren sind beispielsweise pAG-1, YEp6, YEp13 oder pEMBLYe23.

Alternativ kann die Δ-6-Desaturase, Δ-6-Elongase, Δ-5-Desaturase, Δ-5-Elongase, Δ-4-Desaturase, Δ-12-Desaturase und/oder ω-3-Desaturase in Insektenzellen unter Verwendung von Baculovirus-Expressionsvektoren exprimiert werden. Baculovirus-Vektoren, die zur Expression von Proteinen in gezüchteten Insektenzellen (z.B. Sf9-Zellen) verfügbar sind, umfassen die pAc-Reihe (Smith et al. (1983) Mol. Cell Biol.. 3:2156-2165) und die pVL-Reihe (Lucklow und Summers (1989) Virology 170:31-39).

Die oben genannten Vektoren bieten nur einen kleinen Überblick über mögliche geeignete Vektoren. Weitere Plasmide sind dem Fachmann bekannt und sind zum Beispiel beschrieben in: Cloning Vectors (Hrsgb. Pouwels, P.H., et al., Elsevier, Amsterdam-New York-Oxford, 1985, ISBN 0 444 904018). Weitere geeignete Expressionssysteme für prokaryotische und eukaryotische Zellen siehe in den Kapiteln 16 und 17 von Sambrook, J., Fritsch, E.F., und Maniatis, T., Molecular Cloning: A Laboratory Manual, 2. Auflage, Cold Spring Harbor Laboratory, Cold Spring Harbor Laboratory Press, Cold Spring Harbor, NY, 1989.

Letztendlich werden die Δ-6-Desaturase-, Δ-6-Elongase-, Δ-5-Desaturase-, Δ-5-Elongase-, Δ-4-Desaturase-, Δ-12-Desaturase- und/oder ω-3-Desaturase-Gene in Pflanzenzellen oder ganzen Pflanzen (z.B. Spermatophyten, wie Feldfrüchten) exprimiert werden. Beispiele für Pflanzen-Expressionsvektoren umfassen solche, die eingehend beschrieben sind in: Becker, D., Kemper, E., Schell, J., und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721; Vectors for Gene Transfer in Higher Plants; in: Transgenic Plants, Bd. 1, Engineering and Utilization, Hrsgb.: Kung und R. Wu, Academic Press, 1993, S. 15-38.

Eine Pflanzen-Expressionskassette enthält vorzugsweise Regulationssequenzen, welche die Genexpression in Pflanzenzellen steuern können und funktionsfähig verbunden sind, so dass jede Sequenz ihre Funktion, wie Termination der Transkription, erfüllen kann, beispielsweise Polyadenylierungssignale. Bevorzugte Polyadenylierungssignale sind diejenigen, die aus Agrobacterium tumefaciens-T-DNA stammen, wie das als Octopinsynthase bekannte Gen 3 des Ti-Plasmids pTiACH5 (Gielen et al., EMBO J. 3 (1984) 835ff.) oder funktionelle Äquivalente davon, aber auch alle anderen in Pflanzen funktionell aktiven Terminatoren sind geeignet.

Da die Pflanzengenexpression sehr oft nicht auf Transkriptionsebenen beschränkt ist, enthält eine Pflanzen-Expressionskassette vorzugsweise andere funktionsfähig verbunden Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al., 1987, Nucl. Acids Research 15:8693-8711).

Die Pflanzengenexpression muss wie oben beschrieben funktionsfähig mit einem geeigneten Promotor verbunden sein, der die Genexpression auf rechtzeitige, zelloder gewebespezifische Weise durchführt. Nutzbare Promotoren sind konstitutive Promotoren (Benfey et al., EMBO J. 8 (1989) 2195-2202), wie diejenigen, die von Pflanzenviren stammen, wie 35S CAMV (Franck et al., Cell 21 (1980) 285-294), 19S CaMV (siehe auch US 5352605 und WO 84/02913) oder Pflanzenpromotoren, wie der in US 4,962,028 beschriebene der kleinen Untereinheit der Rubisco.

Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment notwendig sind (siehe eine Übersicht in Kermode, Crit. Rev. Plant Sci. 15, 4 (1996) 285-423 und darin zitierte Literaturstellen), beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen.

Die Pflanzengenexpression lässt sich auch wie oben beschrieben über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Auch Promotoren, die auf biotische oder abiotische Stressbedingungen reagieren, sind geeignete Promotoren, beispielsweise der pathogeninduzierte PRP1-Gen-Promotor (Ward et al., Plant. Mol. Biol. 22 (1993) 361-366), der hitzeinduzierbare hsp80-Promotor aus Tomate (US 5,187,267), der kälteinduzierbare Alpha-Amylase-Promotor aus Kartoffel (WO 96/12814) oder der durch Wunden induzierbare pinll-Promotor (EP-A-0 375 091).

Es sind insbesondere solche Promotoren bevorzugt, welche die Genexpression in Geweben und Organen herbeiführen, in denen die Fettsäure-, Lipid- und Ölbiosynthese stattfindet, in Samenzellen, wie den Zellen des Endosperms und des sich entwickelnden Embryos. Geeignete Promotoren sind der Napingen-Promotor aus Raps (US 5,608,152), der USP-Promotor aus Vicia faba (Baeumlein et al., Mol Gen Genet, 1991, 225 (3):459-67), der Oleosin-Promotor aus Arabidopsis (WO 98/45461), der Phaseolin-Promotor aus Phaseolus vulgaris (US 5,504,200), der Bce4-Promotor aus Brassica (WO 91/13980) oder der Legumin-B4-Promotor (LeB4; Baeumlein et al., 1992, Plant Journal, 2 (2):233-9) sowie Promotoren, welche die samenspezifische Expression in Monokotyledonen-Pflanzen, wie Mais, Gerste, Weizen, Roggen, Reis usw. herbeiführen. Geeignete beachtenswerte Promotoren sind der Ipt2- oder Ipt1-Gen-Promotor aus Gerste (WO 95/15389 und WO 95/23230) oder die in WO 99/16890 beschriebenen (Promotoren aus dem Gersten-Hordein-Gen, dem Reis-Glutelin-Gen, dem Reis-Oryzin-Gen, dem Reis-Prolamin-Gen, dem Weizen-Gliadin-Gen, Weizen-Glutelin-Gen, dem Mais-Zein-Gen, dem Hafer-Glutelin-Gen, dem Sorghum-Kasirin-Gen, dem Roggen-Secalin-Gen).

Insbesondere kann die multiparallele Expression der im Verfahren verwendeten Δ-6-Desaturase, Δ-6-Elongase, Δ-5-Desaturase, Δ-5-Elongase, Δ-4-Desaturase, Δ-12-Desaturase und/oder ω-3-Desaturase gewünscht sein. Die Einführung solcher Expressionskassetten kann über eine simultane Transformation mehrerer einzelner Expressionskonstrukte erfolgen oder bevorzugt durch Kombination mehrerer Expressionskassetten auf einem Konstrukt. Auch können mehrere Vektoren mit jeweils mehreren Expressionskassetten transformiert und auf die Wirtszelle übertragen werden.

Ebenfalls besonders geeignet sind Promotoren, welche die plastidenspezifische Expression herbeiführen, da Plastiden das Kompartiment sind, in dem die Vorläufer sowie einige Endprodukte der Lipidbiosynthese synthetisiert werden. Geeignete Promotoren, wie der virale RNA-Polymerase-Promotor, sind beschrieben in WO 95/16783 und WO 97/06250, und der clpP-Promotor aus Arabidopsis, beschrieben in WO 99/46394.

Vektor-DNA lässt sich in prokaryotische oder eukaryotische Zellen über herkömmliche Transformations- oder Transfektionstechniken einbringen. Die Begriffe "Transformation" und "Transfektion", Konjugation und Transduktion, wie hier verwendet, sollen eine Vielzahl von im Stand der Technik bekannten Verfahren zum Einbringen fremder Nukleinsäure (z.B. DNA) in eine Wirtszelle, einschließlich Calciumphosphat- oder Calciumchlorid-Copräzipitation, DEAE-Dextran-vermittelte Transfektion, Lipofektion, natürliche Kompetenz, chemisch vermittelter Transfer, Elektroporation oder Teilchenbeschuss, umfassen. Geeignete Verfahren zur Transformation oder Transfektion von Wirtszellen, einschließlich Pflanzenzellen, lassen sich finden in Sambrook et al. (Molecular Cloning: A Laboratory Manual., 2. Aufl., Cold Spring Harbor Laboratory, Cold Spring Harbor Laboratory Press, Cold Spring Harbor, NY, 1989) und anderen Labor-Handbüchern, wie Methods in Molecular Biology, 1995, Bd. 44, Agrobacterium protocols, Hrsgb: Gartland und Davey, Humana Press, Totowa, New Jersey.

Wirtszellen, die im Prinzip zum Aufnehmen der erfindungsgemäßen Nukleinsäure, des erfindungsgemäßen Genproduktes oder des erfindungsgemäßen Vektors geeignet sind, sind alle prokaryotischen oder eukaryotischen Organismen. Die vorteilhafterweise als Zwischenwirte verwendeten Wirtsorganismen sind Mikroorganismen, wie Pilze oder Hefen. Bevorzugt werden im Verfahren Pflanzen, wie Ölfruchtpflanzen, die große Mengen an Lipidverbindungen enthalten, wie Raps, Nachtkerze, Hanf, Diestel, Erdnuss, Canola, Lein, Soja, Saflor, Sonnenblume, Borretsch, oder Pflanzen, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa, Buschpflanzen (Kaffee, Kakao, Tee), Salix-Arten, Bäume (Ölplame, Kokosnuss) und Futterfeldfrüchte verwendet. Besonders bevorzugte erfindungsgemäße Pflanzen sind Ölfruchtpflanzen, wie Soja, Erdnuss, Raps, Canola, Lein, Hanf, Nachtkerze, Sonnenblume, Saflor, Bäume (Ölpalme, Kokosnuss).

Weitere Gegenstände sind die im folgenden aufgezählten Nukleinsäuresequenzen, die für Δ-6-Desaturasen, Δ-6-Elongasen, Δ-5-Desaturasen, ω-3-Desaturasen und/oder Δ-12-Desaturasen codieren.

Isolierte Nukleinsäuresequenzen, die für Polypeptide mit Δ-6-Desaturaseaktivität codieren, ausgewählt aus der Gruppe:
a) einer Nukleinsäuresequenz mit der in SEQ ID NO: 1 dargestellten Sequenz,
b) Nukleinsäuresequenzen, die für ein Protein mit der in SEQ ID NO: 2 dargestellten Sequenz codieren, oder
c) Derivate der in SEQ ID NO: 1 dargestellten Nukleinsäuresequenz, die für Polypeptide mit mindestens 70 % Identität auf Aminosäureebene mit SEQ ID NO: 2 codieren und eine Δ-6-Desaturaseaktivität aufweisen.

Isolierte Nukleinsäuresequenzen, die für Polypeptide mit Δ-6-Elongaseaktivität codiert, ausgewählt aus der Gruppe:
a) einer Nukleinsäuresequenz mit der in SEQ ID NO: 3, SEQ ID NO: 5 oder SEQ ID NO: 7 dargestellten Sequenz,
b) Nukleinsäuresequenzen, die für ein Protein mit der in SEQ ID NO: 4, SEQ ID NO: 6 oder SEQ ID NO: 8 dargestellten Sequenz codieren, oder
c) Derivate der in SEQ ID NO: 3, SEQ ID NO: 5 oder SEQ ID NO: 7 dargestellten Nukleinsäuresequenz, die für Polypeptide mit mindestens 70 % Identität auf Aminosäureebene mit SEQ ID NO: 4, SEQ ID NO: 6 oder SEQ ID NO: 8 codieren und eine Δ-6-Elongaseaktivität aufweisen.

Isolierte Nukleinsäuresequenzen, die für Polypeptide mit Δ-5-Desaturaseaktivität codieren, ausgewählt aus der Gruppe:
a) einer Nukleinsäuresequenz mit der in SEQ ID NO: 9 dargestellten Sequenz,
b) Nukleinsäuresequenzen, die für ein Protein mit der in SEQ ID NO: 10 dargestellten Sequenz codieren, oder
c) Derivate der in SEQ ID NO: 9 dargestellten Nukleinsäuresequenz, die für Polypeptide mit mindestens 40 % Identität auf Aminosäureebene mit SEQ ID NO: 10 codieren und eine Δ-5-Desaturaseaktivität aufweisen.

Isolierte Nukleinsäuresequenzen, die für Polypeptide mit ω-3-Desaturaseaktivität codieren, ausgewählt aus der Gruppe:
a) einer Nukleinsäuresequenz mit der in SEQ ID NO: 23 dargestellten Sequenz,
b) Nukleinsäuresequenzen, die für ein Protein mit der in SEQ ID NO: 24 dargestellten Sequenz codieren, oder
c) Derivate der in SEQ ID NO: 23 dargestellten Nukleinsäuresequenz, die für Polypeptide mit mindestens 40 % Identität auf Aminosäureebene mit SEQ ID NO: 24 codieren und eine ω-3-Desaturaseaktivität aufweisen.

Isolierte Nukleinsäuresequenzen, die für Polypeptide mit Δ-12-Desaturaseaktivität codieren, ausgewählt aus der Gruppe:
a) einer Nukleinsäuresequenz mit der in SEQ ID NO: 25 dargestellten Sequenz, oder
b) Nukleinsäuresequenzen, die für ein Protein mit der in SEQ ID NO: 26 dargestellten Sequenz codieren, oder
c) Derivate der in SEQ ID NO: 25 dargestellten Nukleinsäuresequenz, die für Polypeptide mit mindestens 40 % Identität auf Aminosäureebene mit SEQ ID NO: 26 codieren und eine Δ-12-Desaturasaktivität aufweisen.

Die oben genannten Nukleinsäuren stammen von Organismen, wie Pilzen, Pflanzen wie Algen oder Diatomeen, die PUFAs synthetisieren können.

Vorteilhaft stammen die isolierten oben genannten Nukleinsäuresequenzen aus der Diatomeengattung Thalassiosira oder aus der Familie der Prasinophyceae wie der Gattung Ostreococcus, aus der Klasse der Euglenophyceae wie der Gattung Euglenia oder Pythiaceae wie der Gattung Phytophtora stammt.

Besonders bevorzugte Nukleinsäuresequenzen stammen aus der Gattung und Art Phytophtora sojae. Diese Nukleinsäuresequenzen ermöglichen in einer vorteilhaften Ausführungsform die Synthese von EPA und/oder ARA. Die folgende Tabelle gibt derartige vorteilhafte Nukleinsäuresequenzen wieder.

| Gen | Enzymfunktion | Proteinsequenz | SEQ ID |
|---|---|---|---|
| D6-Des(Ps) | Δ6-Desaturase | 456 As | SEQ ID NO: 1 |
| D6-Elo(Ps) | Δ6-Elongase | 304 As | SEQ ID NO: 3 |
| D6-Elo(Ps)_2 | Δ6-Elongase | 278 As | SEQ ID NO: 5 |
| D6-Elo(Ps)_3 | Δ6-Elongase | 278 As | SEQ ID NO: 7 |
| D5-Des(Ps) | Δ5-Desaturase | 498 As | SEQ ID NO: 9 |
| D12-Des(Ps) | Δ12-Desaturase | 398 As | SEQ ID NO: 25 |
| O3-Des(Ps) | ω3-Desaturase | 363 As | SEQ ID NO: 23 |

Zu den Gegenständen gehören außerdem, wie oben beschrieben, isolierte Nukleinsäuresequenzen, die für Polypeptide mit Δ-6-Desaturase-, Δ-6-Elongase-, Δ-5-Desaturase-, ω-3-Desaturase- und Δ-12-Desaturaseaktivität codieren, wobei die durch diese Nukleinsäuresequenzen codierten Δ-6-Desaturasen, Δ-6-Elongasen, Δ-5-Desaturasen, ω-3-Desaturasen und/oder Δ-12-Desaturasen C₁₈- oder C₂₀-Fettsäuren mit ein, zwei, drei oder vier Doppelbindungen wie C18:1^{Δ9}, C18:2^{Δ9,12} oder C18:3 ^{Δ9,12,15} mehrfach ungesättigte C₂₀-Fettsäuren mit drei oder vier Doppelbindungen wie C20:3^{Δ8,11,14} oder C20:4^{Δ8,11,14,17} umsetzen. Vorteilhaft werden die Fettsäuren in den Phospholipiden oder CoA-Fettsäureestern desaturiert, vorteilhaft in den CoA-Fettsäureester.

Der Begriff "Nukleinsäure(molekül)", wie hier verwendet, umfasst in einer vorteilhaften Ausführungsform zudem die am 3'- und am 5'-Ende des kodierenden Genbereichs gelegene untranslatierte Sequenz: mindestens 500, bevorzugt 200, besonders bevorzugt 100 Nukleotide der Sequenz stromaufwärts des 5'-Endes des kodierenden Bereichs und mindestens 100, bevorzugt 50, besonders bevorzugt 20 Nukleotide der Sequenz stromabwärts des 3'-Endes des kodierenden Genbereichs. Ein "isoliertes" Nukleinsäuremolekül wird von anderen Nukleinsäuremolekülen abgetrennt, die in der natürlichen Quelle der Nukleinsäure vorliegen. Eine "isolierte" Nukleinsäure hat vorzugsweise keine Sequenzen, welche die Nukleinsäure in der genomischen DNA des Organismus, aus dem die Nukleinsäure stammt, natürlicherweise flankieren (z.B. Sequenzen, die sich an den 5'- und 3'-Enden der Nukleinsäure befinden). Bei verschiedenen Ausführungsformen kann das isolierte Δ-6-Desaturase-, Δ-6-Elongase-, Δ-5-Desaturase-, ω-3-Desaturase- und Δ-12-Desaturasemolekül zum Beispiel weniger als etwa 5 kb, 4 kb, 3 kb, 2 kb, 1 kb, 0,5 kb oder 0,1 kb an Nukleotidsequenzen enthalten, die natürlicherweise das Nukleinsäuremolekül in der genomischen DNA der Zelle, aus der die Nukleinsäure stammt flankieren.

Die im Verfahren verwendeten Nukleinsäuremoleküle, z.B. ein Nukleinsäuremolekül mit einer Nukleotidsequenz der SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 oder eines Teils davon, kann unter Verwendung molekularbiologischer Standardtechniken und der hier bereitgestellten Sequenzinformation isoliert werden. Auch kann Mithilfe von Vergleichsalgorithmen beispielsweise eine homologe Sequenz oder homologe, konservierte Sequenzbereiche auf DNA oder Aminosäureebene identifiziert werden. Diese können als Hybridisierungssonde sowie Standard-Hybridisierungstechniken (wie z.B. beschrieben in Sambrook et al., Molecular Cloning: A Laboratory Manual. 2. Aufl., Cold Spring Harbor Laboratory, Cold Spring Harbor Laboratory Press, Cold Spring Harbor, NY, 1989) zur Isolierung weiterer im Verfahren nützlicher Nukleinsäuresequenzen verwendet werden. Überdies lässt sich ein Nukleinsäuremolekül, umfassend eine vollständige Sequenz der SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 oder einen Teil davon, durch Polymerasekettenreaktion isolieren, wobei Oligonukleotidprimer, die auf der Basis dieser Sequenz oder von Teilen davon, verwendet werden (z.B. kann ein Nukleinsäuremolekül, umfassend die vollständigen Sequenz oder einen Teil davon, durch Polymerasekettenreaktion unter Verwendung von Oligonukleotidprimern isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18:5294-5299) und cDNA mittels Reverser Transkriptase (z.B. Moloney-MLV-Reverse-Transkriptase, erhältlich von Gibco/BRL, Bethesda, MD, oder AMV-Reverse-Transkriptase, erhältlich von Seikagaku America, Inc., St.Petersburg, FL) herstellen. Synthetische Oligonukleotidprimer zur Amplifizierung mittels Polymerasekettenreaktion lassen sich auf der Basis einer der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 gezeigten Sequenzen oder Mithilfe der in SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24 oder SEQ ID NO: 26 dargestellten Aminosäuresequenzen erstellen. Eine erfindungsgemäße Nukleinsäure kann unter Verwendung von cDNA oder alternativ von genomischer DNA als Matrize und geeigneten Oligonukleotidprimern gemäß Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer Desaturase-Nukleotidsequenz entsprechen, können durch Standard-Syntheseverfahren, beispielsweise mit einem automatischen DNA-Synthesegerät, hergestellt werden.

Homologe der verwendeten Δ-6-Desaturase-, Δ-6-Elongase-, Δ-5-Desaturase-, Δ-5-Elongase-, Δ-4-Desaturase-, Δ-12-Desaturase- und/oder ω-3-Desaturase-Nukleinsäuresequenzen mit der Sequenz SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 bedeutet beispielsweise allelische Varianten mit mindestens etwa 40 oder 50 %, vorzugsweise mindestens etwa 60 oder 70 %, stärker bevorzugt mindestens etwa 70 oder 80 %, 90 % oder 95 % und noch stärker bevorzugt mindestens etwa 85 %, 86 %, 87 %, 88 %, 89 %, 90 %, 91 %, 92 %, 93 %, 94 %, 95 %, 96 %, 97 %, 98 %, 99 % oder mehr Identität bzw. Homologie zu einer in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 gezeigten Nukleotidsequenzen oder ihren Homologen, Derivaten oder Analoga oder Teilen davon. Weiterhin sind isolierte Nukleinsäuremoleküle einer Nukleotidsequenz, die an eine der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 gezeigten Nukleotidsequenzen oder einen Teil davon hybridisieren, z.B. unter stringenten Bedingungen hybridisiert. Unter einem Teil gemäß der Erfindung ist dabei zu verstehen, dass mindestens 25 Basenpaare (= bp), 50 bp, 75 bp, 100 bp, 125 bp oder 150 bp, bevorzugt mindestens 175 bp, 200 bp, 225 bp, 250 bp, 275 bp oder 300 bp, besonders bevorzugt 350 bp, 400 bp, 450 bp, 500 bp oder mehr Basenpaare für die Hybridisierung verwendet werden. Es kann auch vorteilhaft die Gesamtsequenz verwendet werden. Allelische Varianten umfassen insbesondere funktionelle Varianten, die sich durch Deletion, Insertion oder Substitution von Nukleotiden aus/in der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 dargestellten Sequenz erhalten lassen, wobei aber die Absicht ist, dass die Enzymaktivität der davon herrührenden synthetisierten Proteine für die Insertion eines oder mehrerer Gene vorteilhafterweise beibehalten wird. Proteine, die noch die enzymatische Aktivität der Δ-6-Desaturase, Δ-6-Elongase, Δ-5-Desaturase, Δ-5-Elongase, Δ-4-Desaturase, Δ-12-Desaturase und/oder ω-3-Desaturase besitzen, das heißt deren Aktivität im wesentlichen nicht reduziert ist, bedeutet Proteine mit mindestens 10 %, vorzugsweise 20 %, besonders bevorzugt 30 %, ganz besonders bevorzugt 40 % der ursprünglichen Enzymaktivität, verglichen mit dem durch SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 kodierten Protein. Die Homologie wurde über den gesamten Aminosäure- bzw. Nukleinsäuresequenzbereich berechnet. Für das Vergleichen verschiedener Sequenzen stehen dem Fachmann eine Reihe von Programmen, die auf verschiedenen Algorithmen beruhen zur Verfügung. Dabei liefern die Algorithmen von Needleman und Wunsch oder Smith und Waterman besonders zuverlässige Ergebnisse. Für die Sequenzvergleiche wurde das Programm PileUp verwendet (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm GAP über den gesamten Sequenzbereich mit folgenden Einstellungen ermittelt: Gap Weight: 50, Length Weight: 3, Average Match: 10.000 und Average Mismatch: 0.000. Die falls nicht anders angegeben als Stan-dardeinstellungen immer für Sequenzvergleiche verwendet wurden.

Homologen der SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 bedeuten beispielsweise auch bakterielle, Pilz- und Pflanzenhomologen, verkürzte Sequenzen, einzelsträngige DNA oder RNA der kodierenden und nicht-kodierenden DNA-Sequenz.

Homologen der SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 bedeutet auch Derivate, wie beispielsweise Promotorvarianten. Die Promotoren stromaufwärts der angegebenen Nukleotidsequenzen können durch einen oder mehrere Nukleotidaustausche, durch Insertion(en) und/oder Deletion(en) modifiziert werden, ohne dass jedoch die Funktionalität oder Aktivität der Promotoren gestört wird. Es ist weiterhin möglich, dass die Aktivität der Promotoren durch Modifikation ihrer Sequenz erhöht ist oder dass sie vollständig durch aktivere Promotoren, sogar aus heterologen Organismen, ersetzt werden.

Die Lipidsynthese lässt sich in zwei Abschnitte unterteilen: die Synthese von Fettsäuren und ihre Bindung an sn-Glycerin-3-Phosphat sowie die Addition oder Modifikation einer polaren Kopfgruppe. Übliche Lipide, die in Membranen verwendet werden, umfassen Phospholipide, Glycolipide, Sphingolipide und Phosphoglyceride. Die Fettsäuresynthese beginnt mit der Umwandlung von Acetyl-CoA in Malonyl-CoA durch die Acetyl-CoA-Carboxylase oder in Acetyl-ACP durch die Acetyltransacylase. Nach einer Kondensationsreaktion bilden diese beiden Produktmoleküle zusammen Acetoacetyl-ACP, das über eine Reihe von Kondensations-, Reduktions- und Dehydratisierungsreaktionen umgewandelt wird, so dass ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F.C. Neidhardt et al. (1996) E. coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltenen Literaturstellen). Die so hergestellten an Phospholipide gebundenen Fettsäuren müssen anschließend wieder für die weiteren Elongationen aus den Phospholipiden in den FettsäureCoA-Ester-Pool überführt werden. Dies ermöglichen Acyl-CoA:Lysophospholipid-Acyltransferasen. Weiterhin können diese Enzyme die elongierten Fettsäuren wieder von den CoA-Estern auf die Phospholipide übertragen. Diese Reaktionsabfolge kann gegebenenfalls mehrfach durchlaufen werden.

Vorläufer für die PUFA-Biosynthese sind beispielsweise Ölsäure, Linol- und Linolensäure. Diese C₁₈-Kohlenstoff-Fettsäuren müssen auf C₂₀ und C₂₂ verlängert werden, damit Fettsäuren vom Eicosa- und Docosa-Kettentyp erhalten werden. Mithilfe der im Verfahren verwendeten Desaturasen und/oder Elongasen können Arachidonsäure, Eicosapentaensäure und/oder Docosahexaensäure vorteilhaft Eicosapentaensäure und/oder Docosahexaensäure hergestellt werden und anschließend für verschiedene Zwecke bei Nahrungsmittel-, Futter-, Kosmetik- oder pharmazeutischen Anwendungen verwendet werden. Mit den genannten Enzymen können C₂₀- und/oder C₂₂-Fettsäuren mit mindestens zwei vorteilhaft mindestens vier, fünf oder sechs Doppelbindungen im Fettsäuremolekül, vorzugsweise C₂₀- oder C₂₂-Fettsäuren mit vorteilhaft fünf oder sechs Doppelbindungen im Fettsäuremolekül hergestellt werden. Substrate der verwendeten Desaturasen und Elongasen im Verfahren sind C₁₆-, C₁₈- oder C₂₀-Fettsäuren wie zum Beispiel Linolsäure, γ-Linolensäure, α-Linolensäure, Dihomo-γ-linolensäure, Eicosatetraensäure oder Stearidonsäure. Bevorzugte Substrate sind Linolsäure, γ-Linolensäure und/oder α-Linolensäure, Dihomo-γ-linolensäure bzw. Arachidonsäure, Eicosatetraensäure oder Eicosapentaensäure. Die synthetisierten C₂₀- oder C₂₂-Fettsäuren mit mindestens vier, fünf oder sechs Doppelbindungen in der Fettsäure fallen im Verfahren in Form der freien Fettsäure oder in Form ihrer Ester beispielsweise in Form ihrer Glyceride vorteilhaft ihrer Triglyceride an.

Unter dem Begriff "Glycerid" wird ein mit ein, zwei oder drei Carbonsäureresten verestertes Glycerin verstanden (Mono-, Di- oder Triglycerid). Unter "Glycerid" wird auch ein Gemisch an verschiedenen Glyceriden verstanden. Das Glycerid oder das Glyceridgemisch kann weitere Zusätze, z.B. freie Fettsäuren, Antioxidantien, Proteine, Kohlenhydrate, Vitamine und/oder andere Substanzen enthalten.

Unter einem "Glycerid" im Sinne des Verfahrens werden ferner vom Glycerin abgeleitete Derivate verstanden. Dazu zählen neben den oben beschriebenen Fettsäureglyceriden auch Glycerophospholipide und Glyceroglycolipide. Bevorzugt seien hier die Glycerophospholipide wie Lecithin (Phosphatidylcholin), Cardiolipin, Phosphatidylglycerin, Phosphatidylserin und Alkylacylglycerophospholipide beispielhaft genannt.

Ferner müssen Fettsäuren anschließend an verschiedene Modifikationsorte transportiert und in das Triacylglycerin-Speicherlipid eingebaut werden. Ein weiterer wichtiger Schritt bei der Lipidsynthese ist der Transfer von Fettsäuren auf die polaren Kopfgruppen, beispielsweise durch Glycerin-Fettsäure-Acyltransferase (siehe Frentzen, 1998, Lipid, 100(4-5):161-166).

Veröffentlichungen über die Pflanzen-Fettsäurebiosynthese, Desaturierung, den Lipidstoffwechsel und Membrantransport von fetthaltigen Verbindungen, die Betaoxidation, Fettsäuremodifikation und Cofaktoren, Triacylglycerin-Speicherung und -Assemblierung einschließlich der Literaturstellen darin siehe in den folgenden Artikeln: Kinney, 1997, Genetic Engeneering, Hrsgb.: JK Setlow, 19:149-166; Ohlrogge und Browse, 1995, Plant Cell 7:957-970; Shanklin und Cahoon, 1998, Annu. Rev. Plant Physiol. Plant Mol. Biol. 49:611-641; Voelker, 1996, Genetic Engeneering, Hrsgb.: JK Setlow, 18:111-13; Gerhardt, 1992, Prog. Lipid R. 31:397-417; Gühnemann-Schäfer & Kindl, 1995, Biochim. Biophys Acta 1256:181-186; Kunau et al., 1995, Prog. Lipid Res. 34:267-342; Stymne et al., 1993, in: Biochemistry and Molecular Biology of Membrane and Storage Lipids of Plants, Hrsgb.: Murata und Somerville, Rockville, American Society of Plant Physiologists, 150-158, Murphy & Ross 1998, Plant Journal. 13(1):1-16.

Die im Verfahren hergestellten PUFAs, umfassen eine Gruppe von Molekülen, die höhere Tiere nicht mehr synthetisieren können und somit aufnehmen müssen oder die höhere Tiere nicht mehr ausreichend selbst herstellen können und somit zusätzlich aufnehmen müssen, obwohl sie leicht von anderen Organismen, wie Bakterien, synthetisiert werden, beispielsweise können Katzen Arachidonsäure nicht mehr synthetisieren.

Unter Phospholipiden im Sinne der Erfindung sind zu verstehen Phosphatidylcholin, Phosphatidylethanolamin, Phosphatidylserin, Phosphatidylglycerin und/oder Phosphatidylinositol vorteilhafterweise Phosphatidylcholin. Die Begriffe Produktion oder Produktivität sind im Fachgebiet bekannt und beinhalten die Produktivität innerhalb einer Pflanzenzelle oder einer Pflanze, das heißt den Gehalt an den gewünschten im Verfahren hergestellten Fettsäuren bezogen auf den Gehalt an allen Fettsäuren in dieser Zelle oder Pflanze. Die Begriffe Biosynthese oder Biosyntheseweg sind im Fachgebiet bekannt und umfassen die Synthese einer Verbindung, vorzugsweise einer organischen Verbindung, durch eine Zelle aus Zwischenverbindungen, beispielsweise in einem Mehrschritt- und stark regulierten Prozess. Die Begriffe Abbau oder Abbauweg sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle) beispielsweise in einem Mehrschritt- und stark regulierten Prozess. Der Begriff Stoffwechsel ist im Fachgebiet bekannt und umfasst die Gesamtheit der biochemischen Reaktionen, die in einem Organismus stattfinden. Der Stoffwechsel einer bestimmten Verbindung (z.B. der Stoffwechsel einer Fettsäure) umfasst dann die Gesamtheit der Biosynthese-, Modifikations- und Abbauwege dieser Verbindung in der Zelle, die diese Verbindung betreffen.

Bei einer weiteren Ausführungsform kodieren Derivate des Nukleinsäuremoleküls wiedergegeben in SEQ ID NO: 1, SEQ ID NO: 3, SEQ ID NO: 5, SEQ ID NO: 7, SEQ ID NO: 9, SEQ ID NO: 23 oder SEQ ID NO: 25 Proteine mit mindestens 40 %, vorteilhaft etwa 50 oder 60 %, vorzugsweise mindestens etwa 60 oder 70 % und stärker bevorzugt mindestens etwa 70 oder 80 %, 80 bis 90 %, 90 bis 95 % und am stärksten bevorzugt mindestens etwa 96 %, 97 %, 98 %, 99 % oder mehr Homologie ( Homologie im Sinne der Erfindung soll Identität bedeuten) zu einer vollständigen Aminosäuresequenz der SEQ ID NO: 2, SEQ ID NO: 4, SEQ ID NO: 6, SEQ ID NO: 8, SEQ ID NO: 10, SEQ ID NO: 24 oder SEQ ID NO: 26. Die Homologie wurde über den gesamten Aminosäure- bzw. Nukleinsäuresequenzbereich berechnet. Für die Sequenzvergleiche wurde das Programm PileUp verwendet (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm BestFit über den gesamten Sequenzbereich mit folgenden Einstellungen ermittelt: Gap Weight: 50, Length Weight: 3, Average Match: 10.000 und Average Mismatch: 0.000. Die falls nicht anders angegeben als Standardeinstellungen immer für Sequenzvergleiche verwendet wurden.

Die Erfindung umfasst zudem Nukleinsäuremoleküle, die sich von einer der in SEQ ID NO: 1, SEQ ID NO: 3, SEQ ID NO: 5, SEQ ID NO: 7, SEQ ID NO: 9, SEQ ID NO: 23 oder SEQ ID NO: 25 gezeigten Nukleotidsequenzen (und Teilen davon) aufgrund des degenerierten genetischen Codes unterscheiden und somit die gleiche Δ-6-Desaturase-, Δ-6-Elongase-, Δ-5-Desaturase-, ω-3-Desaturase- oder Δ-12-Desaturaseaktivität codieren wie diejenige, die von den in SEQ ID NO: 1, SEQ ID NO: 3, SEQ ID NO: 5, SEQ ID NO: 7, SEQ ID NO: 9, SEQ ID NO: 23 oder SEQ ID NO: 25 gezeigten Nukleotidsequenzen kodiert wird.

Zusätzlich zu den in SEQ ID NO: 1, SEQ ID NO: 3, SEQ ID NO: 5, SEQ ID NO: 7, SEQ ID NO: 9, SEQ ID NO: 23 oder SEQ ID NO: 25 gezeigten Δ-6-Desaturasen, Δ-6-Elongasen, Δ-5-Desaturasen, ω-3-Desaturasen oder Δ-12-Desaturasen erkennt der Fachmann, dass DNA-Sequenzpolymorphismen, die zu Änderungen in den Aminosäuresequenzen der Δ-6-Desaturasen, Δ-6-Elongasen, Δ-5-Desaturasen, ω-3-Desaturasen und/oder Δ-12-Desaturasen führen, innerhalb einer Population existieren können. Diese genetischen Polymorphismen im Δ-6-Desaturase-, Δ-6-Elongase-, Δ-5-Desaturase-, ω-3-Desaturase- und/oder Δ-12-Desaturase-Gen können zwischen Individuen innerhalb einer Population aufgrund von natürlicher Variation existieren. Diese natürlichen Varianten bewirken üblicherweise eine Varianz von 1 bis 5 % in der Nukleotidsequenz des Δ-6-Desaturase-, Δ-6-Elongase-, Δ-5-Desaturase-, ω-3-Desaturase- und/oder Δ-12-Desaturase-Gens. Sämtliche und alle dieser Nukleotidvariationen und daraus resultierende Aminosäurepolymorphismen in der Δ-6-Desaturase, Δ-6-Elongase, Δ-5-Desaturase, ω-3-Desaturase und/oder Δ-12-Desaturase, die das Ergebnis natürlicher Variation sind und die funktionelle Aktivität von nicht verändern, sollen im Umfang der Erfindung enthalten sein.

Für das Verfahren vorteilhafte Nukleinsäuremoleküle können auf der Grundlage ihrer Homologie zu den hier offenbarten Δ-6-Desaturase-, Δ-6-Elongase-, Δ-5-Desaturase-, Δ-5-Elongase-, Δ-4-Desaturase-, Δ-12-Desaturase- und/oder ω-3-Desaturase-Nukleinsäuren unter Verwendung der Sequenzen oder eines Teils davon als Hybridisierungssonde gemäß Standard-Hybridisierungstechniken unter stringenten Hybridisierungsbedingungen isoliert werden. Dabei können beispielsweise isolierte Nukleinsäuremoleküle verwendet werden, die mindestens 15 Nukleotide lang sind und unter stringenten Bedingungen mit dem Nukleinsäuremolekülen, die eine Nukleotidsequenz der SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 umfassen, hybridisieren. Es können auch Nukleinsäuren mindestens 25, 50, 100, 250 oder mehr Nukleotide verwendet werden. Der Begriff "hybridisiert unter stringenten Bedingungen", wie hier verwendet, soll Hybridisierungs- und Waschbedingungen beschreiben, unter denen Nukleotidsequenzen, die mindestens 60 % homolog zueinander sind, gewöhnlich aneinander hybridisiert bleiben. Die Bedingungen sind vorzugsweise derart, dass Sequenzen, die mindestens etwa 65 %, stärker bevorzugt mindestens etwa 70 % und noch stärker bevorzugt mindestens etwa 75 % oder stärker zueinander homolog sind, gewöhnlich aneinander hybridisiert bleiben. Diese stringenten Bedingungen sind dem Fachmann bekannt und lassen sich in Current Protocols in Molecular Biology, John Wiley & Sons, N. Y. (1989), 6.3.1-6.3.6., finden. Ein bevorzugtes, nicht einschränkendes Beispiel für stringente Hybridisierungsbedingungen sind Hybridisierungen in 6 x Natriumchlorid/Natriumcitrat (sodium chloride/sodiumcitrate = SSC) bei etwa 45°C, gefolgt von einem oder mehreren Waschschritten in 0,2 x SSC, 0,1 % SDS bei 50 bis 65°C. Dem Fachmann ist bekannt, dass diese Hybridisierungsbedingungen sich je nach dem Typ der Nukleinsäure und, wenn beispielsweise organische Lösungsmittel vorliegen, hinsichtlich der Temperatur und der Konzentration des Puffers unterscheiden. Die Temperatur unterscheidet sich beispielsweise unter "Standard-Hybridisierungsbedingungen" je nach dem Typ der Nukleinsäure zwischen 42°C und 58°C in wässrigem Puffer mit einer Konzentration von 0,1 bis 5 x SSC (pH 7,2). Falls organisches Lösungsmittel im obengenannten Puffer vorliegt, zum Beispiel 50 % Formamid, ist die Temperatur unter Standardbedingungen etwa 42°C. Vorzugsweise sind die Hybridisierungsbedingungen für DNA:DNA-Hybride zum Beispiel 0,1 x SSC und 20°C bis 45°C, vorzugsweise zwischen 30°C und 45°C. Vorzugsweise sind die Hybridisierungsbedingungen für DNA:RNA-Hybride zum Beispiel 0,1 x SSC und 30°C bis 55°C, vorzugsweise zwischen 45°C und 55°C. Die vorstehend genannten Hybridisierungstemperaturen sind beispielsweise für eine Nukleinsäure mit etwa 100 bp (= Basenpaare) Länge und einem G + C-Gehalt von 50 % in Abwesenheit von Formamid bestimmt. Der Fachmann weiß, wie die erforderlichen Hybridisierungsbedingungen anhand von Lehrbüchern, wie dem vorstehend erwähnten oder aus den folgenden Lehrbüchern Sambrook et al., "Molecular Cloning", Cold Spring Harbor Laboratory, 1989; Hames und Higgins (Hrsgb.) 1985, "Nucleic Acids Hybridization: A Practical Approach", IRL Press at Oxford University Press, Oxford; Brown (Hrsgb.) 1991, "Essential Molecular Biology: A Practical Approach", IRL Press at Oxford University Press, Oxford, bestimmt werden können.

Zur Bestimmung der prozentualen Homologie (= Identität) von zwei Aminosäuresequenzen (z.B. einer der Sequenzen der SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24 oder SEQ ID NO: 26) oder von zwei Nukleinsäuren (z.B. SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25) werden die Sequenzen zum Zweck des optimalen Vergleichs untereinander geschrieben (z.B. können Lücken in die Sequenz eines Proteins oder einer Nukleinsäure eingefügt werden, um ein optimales Alignment mit dem anderen Protein oder der anderen Nukleinsäure zu erzeugen). Die Aminosäurereste oder Nukleotide an den entsprechenden Aminosäurepositionen oder Nukleotidpositionen werden dann verglichen. Wenn eine Position in einer Sequenz durch den gleichen Aminosäurerest oder das gleiche Nukleotid wie die entsprechende Stelle in der anderen Sequenz belegt wird, dann sind die Moleküle an dieser Position homolog (d.h. Aminosäure- oder Nukleinsäure-"Homologie", wie hier verwendet, entspricht Aminosäure- oder Nukleinsäure-"Identität"). Die prozentuale Homologie zwischen den beiden Sequenzen ist eine Funktion der Anzahl an identischen Positionen, die den Sequenzen gemeinsam sind (d.h. % Homologie = Anzahl der identischen Positionen/Gesamtanzahl der Positionen x 100). Die Begriffe Homologie und Identität sind damit als Synonym anzusehen. Die verwendeten Programme bzw. Algorithmen sind oben beschrieben.

Ein isoliertes Nukleinsäuremolekül, das für eine Δ-6-Desaturasen, Δ-6-Elongase, Δ-5-Desaturase, Δ-5-Elongase, Δ-4-Desaturasen, Δ-12-Desaturase und/oder ω-3-Desaturase kodiert, die zu einer Proteinsequenz der SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24 oder SEQ ID NO: 26 homolog ist, kann durch Einbringen einer oder mehrerer Nukleotidsubstitutionen, -additionen oder-deletionen in eine Nukleotidsequenz der SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 erzeugt werden, so dass eine oder mehrere Aminosäuresubstitutionen, -additionen oder -deletionen in das kodierte Protein eingebracht werden. Mutationen können in eine der Sequenzen der SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 durch Standardtechniken, wie stellenspezifische Mutagenese und PCRvermittelte Mutagenese, eingebracht werden. Vorzugsweise werden konservative Aminosäuresubstitutionen an einer oder mehreren der vorhergesagten nichtessentiellen Aminosäureresten hergestellt. Bei einer "konservativen Aminosäuresubstitution" wird der Aminosäurerest gegen einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure, Glutaminsäure), ungeladenen polaren Seitenketten (z.B. Glycin, Asparagin, Glutamin, Serin, Threonin, Tyrosin, Cystein), unpolaren Seitenketten, (z.B. Alanin, Valin, Leucin, Isoleucin, Prolin, Phenylalanin, Methionin, Tryptophan), betaverzweigten Seitenketten (z.B. Threonin, Valin, Isoleucin) und aromatischen Seitenketten (z.B. Tyrosin, Phenylalanin, Tryptophan, Histidin). Ein vorhergesagter nichtessentieller Aminosäurerest in einer Δ-6-Desaturase, Δ-6-Elongase, Δ-5-Desaturase, Δ-5-Elongase, Δ-4-Desaturasen, Δ-12-Desaturasen oder ω-3-Desaturase wird somit vorzugsweise durch einen anderen Aminosäurerest aus der gleichen Seitenkettenfamilie ausgetauscht. Alternativ können bei einer anderen Ausführungsform die Mutationen zufallsgemäß über die gesamte oder einen Teil der Δ-6-Desaturase, Δ-6-Elongase, Δ-5-Desaturase, Δ-5-Elongase, Δ-4-Desaturase, Δ-12-Desaturase oder ω-3-Desaturase kodierenden Sequenz eingebracht werden, z.B. durch Sättigungsmutagenese, und die resultierenden Mutanten können nach der hier beschriebenen Δ-6-Desaturase-, Δ-6-Elongase-, Δ-5-Desaturase-, Δ-5-Elongase-, Δ-4-Desaturase-, Δ-12-Desaturase -und/oder ω-3-Desaturase-Aktivität durchmustert werden, um Mutanten zu identifizieren, die die Δ-6-Desaturase-, Δ-6-Elongase-, Δ-5-Desaturase-, Δ-5-Elongase-, Δ-4-Desaturase-, Δ-12-Desaturase -und/oder ω-3-Desaturase-Aktivität beibehalten haben. Nach der Mutagenese einer der Sequenzen SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 kann das kodierte Protein rekombinant exprimiert werden, und die Aktivität des Proteins kann z.B. unter Verwendung der hier beschriebenen Tests bestimmt werden.

Weitere Erfindungsgegenstände sind ein transgener nicht-humaner Organismus vorteilhaft eine transgene Pflanze, die die erfindungsgemäße Nukleinsäure SEQ ID NO: 25 enthalten oder ein Genkonstrukt oder einen Vektor, die diese erfindungsgemäßen Nukleinsäuresequenzen enthalten.

Diese Erfindung wird durch die nachstehenden Beispiele weiter veranschaulicht, die nicht als beschränkend aufgefasst werden sollten.

### Beispiele

### Beispiel 1: Allgemeine Klonierungsverfahren:

Die Klonierungsverfahren wie z.B. Restriktionsspaltungen, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von Escherichia coli Zellen, Anzucht von Bakterien und die Sequenzanalyse rekombinanter DNA wurden wie bei Sambrook et al. (1989) (Cold Spring Harbor Laboratory Press: ISBN 0-87969-309-6) beschrieben durchgeführt.

### Beispiel 2: Sequenzanalyse rekombinanter DNA:

Die Sequenzierung rekombinanter DNA-Moleküle erfolgte mit einem Laserfluoreszenz-DNA-Sequenzierer der Firma ABI nach der Methode von Sanger (Sanger et al. (1977) Proc. Natl. Acad. Sci. USA74, 5463-5467). Fragmente resultierend aus einer Polymerase Kettenreaktion wurden zur Vermeidung von Polymerasefehlern in zu exprimierenden Konstrukten sequenziert und überprüft.

### Beispiel 3: Klonierung von PUFA-spezifischen Desaturasen und Elongasen aus Phytophthora sojae

Für die Suche nach neuen Genen mit Δ-6-, Δ-5-, Δ-12-, ω3-Desaturase and Δ-6-Elongase Aktivität wurde eine genomische Datenbank von Phytophtora sojae durchsucht (http:/genome.jgi-psf.org/sojae/sojae1.home.html). Diese Datenbank enthält Rohsequenzen, die etwa 90% der genomischen DNA des Organismus abdecken. Für alle gesuchten Aktivitäten konnten putative Kandidatengene gefunden werden. Durch Herstellung und Charakterisierung von cDNA dieser Kandidatengene konnte deren korrekte kodierende offene Leseraster gefunden und die Aminosäuresequenz abgeleitet werden. Diese ist wie folgt:

| Gen | Enzymfunktion | Proteinsequenz | SEQ ID |
|---|---|---|---|
| D6-Des(Ps) | Δ6-Desaturase | 456 As | SEQ ID NO: 1 |
| D6-Elo(Ps) | Δ6-Elongase | 304 As | SEQ ID NO: 3 |
| D6-Elo(Ps)_2 | Δ6-Elongase | 278 As | SEQ ID NO: 5 |
| D6-Elo(Ps)_3 | Δ6-Elongase | 278 As | SEQ ID NO: 7 |
| D5-Des(Ps) | Δ5-Desaturase | 498 As | SEQ ID NO: 9 |
| D12-Des(Ps) | Δ12-Desaturase | 398 As | SEQ ID NO: 25 |
| O3-Des(Ps) | ω3-Desaturase | 363 As | SEQ ID NO: 23 |

Zur Herstellung von cDNA wurde Gesamt-RNA von Phytophthora sojae mit Hilfe des RNAeasy Kits der Firma Qiagen (Valencia, CA, US) isoliert. Aus der Gesamt-RNA wurde mit Hilfe von oligo-dT-Cellulose poly-A+ RNA (mRNA) isoliert (Sambrook et al., 1989). Die RNA wurde mit dem Reverse Transcription System Kit von Promega revers transcribiert und die synthetisierte cDNA in den lambda ZAP Vektor (lambda ZAP Gold, Stratagene) kloniert. Entsprechend der Herstellerangaben wurde die cDNA zur Plasmid-DNA entpackt. Die Plasmid-Bank wurde dann nach den entsprechenden putativen Kandidatengenen gesichtet (Hybridisierung von Bakterien-Klonen, Sambrook et al. 1989) und positive Klone wurden sequenziert. Die entsprechenden cDNA-Klone wurde dann für die PCR zur Klonierung von Expressionsplasmiden verwendet.

### Beispiel 4: Klonierung von Expressionsplasmiden zur heterologen Expression von Phytophtora sojae Genen in Hefen

Zur heterologen Expression in Hefen werden über PCR mit entsprechenden spezifischen Primern die entsprechenden Sequenzen amplifiziert und in den Hefe-Expressionsvektor pYES2.1-TOPO (Invitrogen) gemäss Herstellerangaben kloniert. Dabei werden ausschließlich die für die PUFA-Proteine kodierende offenen Leserrahmen der Gene amplifiziert. Zusätzlich wird am 5' eine Kozak-Sequenz (Cell 1986, 44:283-292) angehängt:

| Gen | Basenpaare | Primer | SEQ ID |
|---|---|---|---|
| D5-Des(Ps) | 1497 bp | Fwd : gccatggcccccatcgagaccgac | SEQ ID NO: 27 |
| | | Rvs : ttagcccatgtggacggaca | SEQ ID NO: 28 |
| D6-Des(Ps) | 1371 bp | Fwd : accatggtggatggccccaagacca | SEQ ID NO: 29 |
| | | Rvs : ttacatggccgggaactcgagcagg | SEQ ID NO: 30 |
| D12-Des(Ps) | 1197 bp | Fwd : gccatggcgatcctgaacccgg | SEQ ID NO: 31 |
| | | Rvs : tagagcttgttcttgtaga | SEQ ID NO: 32 |
| 03-Des(Ps) | 1092 bp | Fwd: gccatggcgtccaagcaggagca | SEQ ID NO: 33 |
| | | Rvs: tcagttggccttagtcttggtcgcc | SEQ ID NO: 34 |
| D6-Elo(Ps) | 915 bp | Fwd: aagatggagacgaccttcgcgcgc | SEQ ID NO: 35 |
| | | Rvs: ttactgcgtcttcttggcgaccgcagcg | SEQ ID NO: 36 |
| D6-Elo(Ps)_2 | 837 bp | Fwd: gccatggcgtcggagctgctgca | SEQ ID NO: 37 |
| | | Rvs: ttagaggttcttcttggccgg | SEQ ID NO: 38 |
| D6-Elo(Ps)_3 | 837 bp | Fwd: accatgtcggccgacctgctgc | SEQ ID NO: 39 |
| | | Rvs: ttagagcttcttcttggc | SEQ ID NO: 40 |

Zusammensetzung des PCR-Ansatzes (50 µL):
   5,00 µL Template cDNA
   5,00 µL 10x Puffer (Advantage-Polymerase)+ 25mM MgCl₂
   5,00 µL 2mM dNTP
   1,25 µL je Primer (10 pmol/µL des 5'-ATG sowie des 3'-Stopp Primers)
   0,50 µL Advantage-Polymerase
      Die Advantage-Polymerase von Clontech wird eingesetzt.
Reaktionsbedingungen der PCR:
   Anlagerungstemperatur: 1 min 55°C
   Denaturierungstemperatur: 1 min 94°C
   Elongationstemperatur: 2 min 72°C
   Anzahl der Zyklen: 35

Die PCR Produkte werden gemäss Herstellerangaben mit dem Plasmid pYES2.1-TOPO (Invitrogen) inkubiert. Die Inkubationsreaktionen werden dann in E. coli DH5α-Zellen (Invitrogen) nach Herstellerangaben transformiert. Positive Klone werden durch PCR (siehe Reaktion oben) identifiziert und die Plasmid-DNA isoliert (Qiagen Dneasy). Die entstandene Plasmide werden durch Sequenzierung überprüft und in den Saccharomyces Stamm INVSc1 (Invitrogen) durch Elektroporation (1500 V) transformiert. Zur Kontrolle wird pYES2.1 (Leervektor) parallel transformiert. Die Selektion der transformierten Hefen erfolgt auf Komplett-Minimalmedium (CMdum)-Agarplatten mit 2% Glucose, aber ohne Uracil. Nach der Selektion werden je drei Transformanten zur weiteren funktionellen Expression ausgewählt.

Für die Expresssion der Gene aus Phytophtora sojae werden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert. 5 ml CMdum-Flüssigmedium (ohne Uracil) mit 2% Raffinose und 300 µM verschiedener Fettsäuren werden dann mit den Vorkulturen auf eine OD₆₀₀ von 0,05 angeimpft. Die Expression wird durch die Zugabe von 2% (w/v) Galactose induziert. Die Kulturen werden für weitere 96 h bei 20°C inkubiert.

### Beispiel 5: Klonierung von Expressionsplasmiden zur Samen-spezifischen Expression in Pflanzen

Für die Transformation von Pflanzen werden weiterere Transformationsvektoren auf Basis von pSUN-USP erzeugt. Dazu werden mit folgenden Primerpaaren Notl-Schnittstellen am 5' und 3'-Ende der kodierenden Sequenz eingefügt (siehe folgende Tabelle).
Zusammensetzung des PCR-Ansatzes (50 µL):
5,00 µL Template cDNA
5,00 µL 10x Puffer (Advantage-Polymerase)+ 25mM MgCl₂
5,00 µL 2mM dNTP
1,25 µL je Primer (10 pmol/µL)
0,50 µL Advantage-Polymerase

Die Advantage-Polymerase von Clontech wird eingesetzt.
Reaktionsbedingungen der PCR:
Anlagerungstemperatur: 1 min 55°C
Denaturierungstemperatur: 1 min 94°C
Elongationstemperatur: 2 min 72°C
Anzahl der Zyklen: 35

| Gen | Basenpaare | Primer | SEQ ID |
|---|---|---|---|
| D5-Des(Ps) | 1497 bp | Fwd : gcggccgcgccatggcccccatcgagaccgac | SEQ ID NO: 41 |
| | | Rvs : gcggccgcttagcccatgtggacggaca | SEQ ID NO: 42 |
| D6-Des(Ps) | 1371 bp | Fwd : gcggccgcaccatggtggatggccccaagacca | SEQ ID NO: 43 |
| | | Rvs : gcggccgcttacatggccgggaactcgagcagg | SEQ ID NO: 44 |
| D12-Des(Ps) | 1197 bp | Fwd : gcggccgcgccatggcgatcctgaacccgg | SEQ ID NO: 45 |
| | | Rvs : gcggccgctagagcttgttcttgtaga | SEQ ID NO: 46 |
| 03-Des(Ps) | 1092 bp | Fwd: gcggccgcgccatggcgtccaagcaggagca | SEQ ID NO: 47 |
| | | Rvs: gcggccgctcagttggccttagtcttggtcgcc | SEQ ID NO: 48 |
| D6-Elo(Ps) | 915 bp | Fwd: gcggccgcaagatggagacgaccttcgcgcgc | SEQ ID NO: 49 |
| | | Rvs: gcggccgcttactgcgtcttcttggcgaccgcagcg | SEQ ID NO: 50 |
| D6-Elo(Ps)_2 | 837 bp | Fwd: gcggccgcgccatggcgtcggagctgctgca | SEQ ID NO: 51 |
| | | Rvs: gcggccgcttagaggttcttcttggccgg | SEQ ID NO: 52 |
| D6-Elo(Ps)_3 | 837 bp | Fwd: gcggccgcaccatgtcggccgacctgctgc | SEQ ID NO: 53 |
| | | Rvs: gcggccgcttagagcttcttcttggc | SEQ ID NO: 54 |

Die PCR Produkte werden für 4 h bei 37°C mit dem Restriktionsenzym Notl inkubiert. Der Pflanzen-Expressionsvektor pSUN300-USP wird in gleicherweise inkubiert. Anschließend werden die PCR Produkte sowie der 7624 bp große Vektor durch Agarose-Gelelektrophorese aufgetrennt und die entsprechenden DNA-Fragmente ausgeschnitten. Die Aufreinigung der DNA erfolgt mittels Qiagen Gel purification Kit gemäss Herstellerangaben. Anschliessend werden Vektor und PCR-Produkte ligiert.

Dazu wird das Rapid Ligation Kit von Roche verwendet. Die entstandene Plasmide werden durch Sequenzierung verifiziert.

pSUN300 ist ein Derivat des Plasmides pPZP (Hajdukiewicz,P, Svab, Z, Maliga, P., (1994) The small versatile pPZP family of Agrobacterium binary vectors for plant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des OCS-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1 bis 684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert. (Primersequenz: 5'-GTCGACCCGCGGACTAGTGGGCCCTCTAGACCCGGGGGATCC GGATCTGCTGGCTATGAA-3') [SEQ ID NO: 55].

Das PCR-Fragment wurde mit EcoRI/Sall nachgeschnitten und in den Vektor pSUN300 mit OCS Terminator eingesetzt. Es entstand das Plasmid mit der Bezeichnung pSUN-USP, das für die Pflanzen-Transformation mittels *Agrobacterium tumefaciens* eingesetzt werden kann.

### Beispiel 6: Expression von Phytophtora sojae Genen in Hefen

Hefen, die wie unter Beispiel 4 mit dem Plasmid pYES2.1 oder den Plasmiden pYES-d4Des(Ps), pYES-d5Des(Ps), pYES-d6Des(Ps), pYES-d12Des(Ps), pYES-o3Des(Ps), pYES-d6Elo(Ps), pYES-d6Elo-2(Ps) und pYES-d6Elo-3(Ps) transformiert wurden, werden folgendermaßen analysiert:
Die Hefezellen aus den Hauptkulturen werden durch Zentrifugation (100 x g, 5 min, 20°C) geerntet und mit 100 mM NaHCO₃, pH 8,0 gewaschen, um restliches Medium und Fettsäuren zu entfernen. Aus den Hefe-Zellsedimenten werden Fettsäuremethylester (FAMEs) durch saure Methanolyse hergestellt. Hierzu werden die Zellsedimente mit 2 ml 1 N methanolischer Schwefelsäure und 2% (v/v) Dimethoxypropan für 1 h bei 80°C inkubiert. Die Extraktion der FAMEs erfolgt durch zweimalige Extraktion mit Petrolether (PE). Zur Entfernung nicht derivatisierter Fettsäuren werden die organischen Phasen je einmal mit 2 ml 100 mM NaHCO₃, pH 8,0 und 2 ml Aqua dest. gewaschen. Anschließend werden die PE-Phasen mit Na₂SO₄ getrocknet, unter Argon eingedampft und in 100 µl PE aufgenommen. Die Proben werden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse sind wie folgt: Die Ofentemperatur wurde von 50°C bis 250°C mit einer Rate von 5°C/min und schließlich 10 min bei 250°C(halten) programmiert.

Die Identifikation der Signale erfolgt durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

### Beispiel 7: Funktionelle Charakterisierung von Genen aus Phytophtora sojae:

Die Aktivität und Substratspezifität der einzelnen Gene kann nach Expression und Fütterung verschiedener Fettsäuren ermittelt werden. Die gefütterten Substrate liegen in großen Mengen in allen transgenen Hefen vor, wodurch die Aufnahme dieser Fettsäuren in die Hefen bewiesen ist. Die transgenen Hefen zeigen die Synthese neuer Fettsäuren, den Produkten der Gene. Dies bedeutet, dass die Gene aus Phytophtora sojae funktional exprimiert werden können.

Die Substratspezifität von Desaturasen und Elongasen kann nach Expression in Hefe durch die Fütterung mittels verschiedener Hefen ermittelt werden. Beschreibungen für die Bestimmung der einzelnen Aktivitäten finden sich in WO 93/11245 für o3-Desaturasen, WO 94/11516 für Δ12-Desaturasen, WO 93/06712, US 5,614,393, US5614393, WO 96/21022, WO0021557 und WO 99/27111 für Δ6-Desaturasen, Qiu et al. 2001, J. Biol. Chem. 276, 31561-31566 für Δ4-Desaturasen, Hong et al. 2002, Lipids 37,863-868 für Δ5-Desaturasen, für Elongasen in Zank, T.K. et al. Plant Journal 31:255-268, 2002.

Die Aktivität der einzelnen Desaturasen und Elongasen wird aus der Konversionsrate errechnet nach der Formel [Substrat/(Substrat+Produkt)*100].

### Beispiel 8: Erzeugung von transgenen Pflanzen

### a) Erzeugung transgener Rapspflanzen (verändert nach Moloney et al., 1992, Plant Cell Reports, 8:238-242)

Zur Erzeugung transgener Rapspflanzen werden binäre Vektoren wie die unter Beispiel 5 erzeugten pSUN Plasmide mit Genen aus Phytophtora sojae in Agrobacterium tumefaciens C58C1:pGV2260 transformiert (Deblaere et al, 1984, Nucl. Acids. Res. 13, 4777-4788). Zur Transformation von Rapspflanzen (Var. Drakkar, NPZ Nordeutsche Pflanzenzucht, Hohenlieth, Deutschland), wird eine 1:50 Verdünnung einer Übernachtkultur einer positiv transformierten Agrobakterienkolonie in Murashige-Skoog Medium (Murashige und Skoog 1962 Physiol. Plant. 15, 473) mit 3 % Saccharose (3MS-Medium) benutzt. Petiolen oder Hypokotyledonen frisch gekeimter steriler Rapspflanzen (zu je ca. 1 cm²) wurden in einer Petrischale mit einer 1:50 Agrobakterienverdünnung für 5-10 Minuten inkubiert. Es folgt eine 3-tägige Co-Inkubation in Dunkelheit bei 25°C auf 3MS-Medium mit 0,8 % Bacto-Agar. Die Kultivierung wurde nach 3 Tagen mit 16 Stunden Licht / 8 Stunden Dunkelheit weitergeführt und in wöchentlichem Rhythmus auf MS-Medium mit 500 mg/l Claforan (Cefotaxime-Natrium), 50 mg/l Kanamycin, 20 mikroM Benzylaminopurin (BAP) und 1,6 g/l Glukose weitergeführt. Wachsende Sprosse wurden auf MS-Medium mit 2 % Saccharose, 250 mg/l Claforan und 0,8 % Bacto-Agar überführt. Bildeten sich nach drei Wochen keine Wurzeln, so wurde als Wachstumshormon 2-Indolbuttersäure zum Bewurzeln zum Medium gegeben.

Regenerierte Sprosse wurden auf 2MS-Medium mit Kanamycin und Claforan erhalten, nach Bewurzelung in Erde überführt und nach Kultivierung für zwei Wochen in einer Klimakammer oder im Gewächshaus angezogen, zur Blüte gebracht, reife Samen geerntet und auf Expression der Desaturase- bzw. Elongase-Gene mittels Lipidanalysen untersucht wie beispielhaft in Qiu et al. 2001, J. Biol. Chem. 276, 31561-31566 beschrieben.

### b) Herstellung von transgenen Leinpflanzen

Die Herstellung von transgenen Leinpflanzen können zum Beispiel nach der Methode von Bell et al., 1999, In Vitro Cell. Dev. Biol.-Plant. 35(6):456-465 mittels particle bombartment erzeugt werden. Agrobakterien-vermittelte Transformationen können zum Beispiel nach Mlynarova et al. (1994), Plant Cell Report 13: 282-285 hergestellt werden

### Beispiel 9: Lipidextraktion aus Samen:

Die Auswirkung der genetischen Modifikation in Pflanzen, Pilzen, Algen, Ciliaten oder auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder einer Fettsäure) untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen.

Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, S. 103-129; 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (d.h. Sigma), definiert werden.

Pflanzenmaterial wird zunächst mechanisch durch Mörsern homogenisiert, um es einer Extraktion zugänglicher zu machen.

Dann wird 10 min auf 100°C erhitzt und nach dem Abkühlen auf Eis erneut sedimentiert. Das Zellsediment wird mit 1 M methanolischer Schwefelsäure und 2 % Dimethoxypropan 1h bei 90°C hydrolysiert und die Lipide transmethyliert. Die resultierenden Fettsäuremethylester (FAME) werden in Petrolether extrahiert. Die extrahierten FAME werden durch Gasflüssigkeitschromatographie mit einer Kapillarsäule (Chrompack, WCOT Fused Silica, CP-Wax-52 CB, 25 m, 0,32 mm) und einem Temperaturgradienten von 170°C auf 240°C in 20 min und 5 min bei 240°C analysiert. Die Identität der Fettsäuremethylester wird durch Vergleich mit entsprechenden FAME-Standards (Sigma) bestätigt. Die Identität und die Position der Doppelbindung kann durch geeignete chemische Derivatisierung der FAME-Gemische z.B. zu 4,4-Dimethoxyoxazolin-Derivaten (Christie, 1998) mittels GC-MS weiter analysiert werden.

### Äquivalente:

Der Fachmann erkennt oder kann viele Äquivalente der hier beschriebenen erfindungsgemäßen spezifischen Ausführungsformen feststellen, indem er lediglich Routineexperimente verwendet. Diese Äquivalente sollen von den Patentansprüchen umfasst sein.

## Patentansprüche

1. Nukleinsäure codierend für ein Polypeptid mit Δ-12-Desaturaseaktivität, wobei die Nukleinsäure ausgewählt ist aus der Gruppe bestehend aus:
a) einer Nukleinsäure mit der in SEQ ID NO: 25 dargestellten Sequenz, oder
b) Nukleinsäuren, die für ein Polypeptid mit der in SEQ ID NO: 26 dargestellten Sequenz codieren, oder
c) Nukleinsäuren, die für ein Polypeptid mit mindestens 70 % Identität auf Aminosäureebene mit SEQ ID NO: 26 codieren, oder
d) einer Nukleinsäure erhältlich mit Amplifikation aus Phytophthora sojae mit den Primerpaar (i) SEQ ID NO. 45 und SEQ ID NO. 46 oder (ii) SEQ ID NO. 31 und SEQ ID NO. 32.

2. Protein codiert von einer Nukleinsäure gemäß Anspruch 1.

3. Genkonstrukt, enthaltend eine Nukleinsäure gemäß Anspruch 1, wobei die Nukleinsäure funktionsfähig mit einem oder mehreren Regulationssignalen verbunden ist.

4. Genkonstrukt gemäß Anspruch 3, **dadurch gekennzeichnet, dass** das Nukleinsäurekonstrukt zusätzliche Biosynthesegene des Fettsäure- oder Lipidstoffwechsels enthält ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n).

5. Genkonstrukt gemäß Anspruch 3 oder 4, **dadurch gekennzeichnet, dass** das Nukleinsäurekonstrukt zusätzliche Biosynthesegene des Fettsäure- oder Lipidstoffwechsels enthält ausgewählt aus der Gruppe der Δ-4-Desaturasen, Δ-5-Desaturasen, Δ-6-Desaturasen, Δ-8-Desaturasen, Δ-12-Desaturasen oder Δ-9-Elongasen.

6. Vektor, enthaltend eine Nukleinsäure gemäß Anspruch 1 oder
ein Genkonstrukt gemäß einem der Ansprüche 3 bis 5.

7. Nicht-humaner Organismus, transgen für mindestens ein Genkonstrukt gemäß einem der Ansprüche 3 bis 5 oder einen Vektor gemäß Anspruch 6 oder die Nukleinsäure gemäß Anspruch 1.

8. Transgener nicht-humaner Organismus, enthaltend mindestens eine Nukleinsäure gemäß Anspruch 1, ein Genkonstrukt gemäß einem der Ansprüche 3 bis 5, einen Vektor gemäß Anspruch 6, oder einen Organismus nach Anspruch 7, wobei der Organismus eine Pflanze ist.

9. Transgene Pflanze gemäß Anspruch 8, **dadurch gekennzeichnet, dass** die Pflanze eine Ölfruchtpflanze oder Nutzpflanze ist und vorzugsweise ausgewählt ist aus der Gruppe Erdnuss, Raps, Canola, Sonnenblume, Safflor (Carthamus tinctoria), Mohn, Senf, Hanf, Rizinus, Olive, Sesam, Calendula, Punica, Nachtkerze, Königskerze, Distel, Haselnuss, Mandel, Macadamia, Avocado, Lorbeer, Kürbis, Lein, Soja, Pistazien, Borretsch, Ölpalme, Kokosnuss, Walnuss, Mais, Weizen, Roggen, Hafer, Triticale, Reis, Gerste, Baumwolle, Maniok oder Pfeffer.

10. Transgener Teil einer transgenen Pflanze gemäß Anspruch 8 oder 9, vorzugsweise transgener Same einer transgenen Pflanze gemäß Anspruch 8 oder 9.

11. Verfahren zur Isolierung und Herstellung von Öl, Fett, Lipiden und/oder freien Fettsäuren, wobei das Verfahren das Pressen oder die Extraktion der Pflanzenteile oder Pflanzensamen nach Anspruch 10 umfasst.

12. Verfahren zur Herstellung von Futtermitteln, Nahrungsmitteln, Kosmetika oder Pharmazeutika, enthaltend die Schritte des Verfahrens nach Anspruch 11 und Mischen dieser Öle, Lipide und/oder freien Fettsäuren mit tierischen, mikrobiellen oder pflanzlichen Ölen, Lipiden oder Fettsäuren.

13. Verwendung der Nukleinsäure gemäß Anspruch 1, der transgenen Pflanze gemäß Anspruch 9 oder des transgenen Teils einer transgenen Pflanze gemäß Anspruch 10 für die Herstellung von Öl, Fett, Lipiden oder freien Fettsäuren.

14. Verwendung der Nukleinsäure gemäß Anspruch 1, der transgenen Pflanze gemäß Anspruch 9 oder des transgenen Teils einer transgenen Pflanze gemäß Anspruch 10 zur Herstellung chemisch reiner mehrfach ungesättigter Fettsäure oder Fettsäurezusammenstellung für Anwendungen im Bereich der Lebensmittelindustrie, Kosmetikindustrie oder Pharmaindustrie.

## Claims

1. A nucleic acid coding for a polypeptide with Δ12-desaturase activity, where the nucleic acid is selected from the group consisting of:
a) a nucleic acid with the sequence shown in SEQ ID NO: 25, or
b) nucleic acids which code for a polypeptide with the sequence shown in SEQ ID NO: 26, or
c) nucleic acids which code for a polypeptide with at least 70% identity at the amino acid level with SEQ ID NO: 26, or
d) a nucleic acid obtainable with amplification from Phytophthora sojae with the primer pair (i) SEQ ID NO: 45 and SEQ ID NO: 46 or (ii) SEQ ID NO: 31 and SEQ ID NO: 32.

2. A protein encoded by a nucleic acid according to claim 1.

3. A gene construct comprising a nucleic acid according to claim 1, where the nucleic acid is linked operably with one or more regulatory signals.

4. The gene construct according to claim 3, **characterized in that** the nucleic acid construct comprises additional biosynthesis genes of the fatty acid or lipid metabolism selected from the group acyl-CoA dehydrogenase(s), acyl-ACP [= acyl carrier protein] desaturase(s), acyl-ACP thioesterase(s),fatty acid acyltransferase(s), acyl-CoA:lysophospholipid acyltransferase(s), fatty acid synthase(s), fatty acid hydroxylase(s), acetyl-coenzyme A carboxylase(s), acyl-coenzyme A oxidase(s), fatty acid desaturase(s), fatty acid acetylenases, lipoxygenases, triacylglycerol lipases, allenoxide synthases, hydroperoxide lyases or fatty acid elongase(s).

5. The gene construct as claimed in claim 3 or 4, **characterized in that** the nucleic acid construct comprises additional biosynthesis genes of the fatty acid or lipid metabolism selected from the group of the Δ4-desaturases, Δ5-desaturases, Δ6-desaturases, Δ8-desaturases, Δ12-desaturases or Δ9-elongases.

6. A vector comprising a nucleic acid according to claim 1 or a gene construct according to any of claims 3 to 5.

7. A nonhuman organism, transgenic for at least one gene construct according to any of claims 3 to 5 or a vector according to claim 6 or the nucleic acid according to claim 1.

8. A transgenic nonhuman organism, comprising at least one nucleic acid according to claim 1, a gene construct according to any of claims 3 to 5, a vector according to claim 6, or an organism according to claim 7, where the organism is a plant.

9. A transgenic plant according to claim 8, **characterized in that** the plant is an oil crop plant or useful plant and preferably is selected from the group consisting of peanut, oilseed rape, canola, sunflower, safflower (Carthamus tinctoria), poppy, mustard, hemp, castor-oil plant, olive, sesame, Calendula, Punica, evening primrose, verbascum, thistle, hazelnut, almond, macadamia, avocado, bay, pumpkin, linseed, soya, pistachios, borage, oil palm, coconut, walnut, maize, wheat, rye, oats, triticale, rice, barley, cotton, cassava or pepper.

10. A transgenic part of a transgenic plant according to claim 8 or 9, preferably transgenic seed of a transgenic plant according to claim 8 or 9.

11. A process for the isolation and production of oil, fat, lipids and/or free fatty acids, the process comprising the pressing or extraction of the plant parts or plant seeds according to claim 10.

12. A process for the production of feedstuffs, foodstuffs, cosmetics or pharmaceuticals, comprising the steps of the process according to claim 11 and mixing of these oils, lipids and/or free fatty acids with animal, microbial or vegetable oils, lipids or fatty acids.

13. The use of the nucleic acid according to claim 1, the transgenic plant according to claim 9 or the transgenic part of a transgenic plant according to claim 10 for the production of oil, fat, lipids or free fatty acids.

14. The use of the nucleic acid according to claim 1, the transgenic plant according to claim 9 or the transgenic part of a transgenic plant according to claim 10 for the production of chemically pure polyunsaturated fatty acid or fatty acid composition for applications in the sector of the food industry, cosmetic industry or pharmaceutical industry.

## Revendications

1. Acide nucléique codant pour un polypeptide à activité Δ-12-désaturase, l'acide nucléique étant choisi dans le groupe constitué par :
a) un acide nucléique ayant la séquence représentée dans SEQ ID N° : 25 ou
b) des acides nucléiques qui codent pour un polypeptide ayant la séquence représentée dans SEQ ID N° : 26, ou
c) des acides nucléiques qui codent pour un polypeptide ayant un degré d'identité d'au moins 70 % sur le plan des acides aminés avec SEQ ID N° : 26, ou
d) un acide nucléique pouvant être obtenu par amplification à partir de *Phytophthora sojae* avec la paire d'amorces (i) SEQ ID N° : 45 et SEQ ID N° : 46 ou (ii) SEQ ID N° : 31 et SEQ ID N° : 32.

2. Protéine codée par un acide nucléique selon la revendication 1.

3. Produit de construction génique, contenant un acide nucléique selon la revendication 1, l'acide nucléique étant fonctionnellement lié à un ou plusieurs signaux de régulation.

4. Produit de construction génique selon la revendication 3, **caractérisé en ce que** le produit de construction d'acide nucléique contient des gènes de biosynthèse supplémentaires du métabolisme des acides gras ou des lipides, choisis dans le groupe des acyl-CoA-déshydrogénase(s), acyl-ACP[= acyl carrier protein]-désaturase(s), Acyl-ACP-thioestérase(s), acide gras acyltransférase(s), acyl-CoA:lysophospholipideacyltransférase(s), acide gras-synthase(s), acide grashydroxylase(s), acétyl-coenzyme A-carboxylase(s), acylcoenzyme A-oxydase(s), acide gras-désaturase(s), acide gras-acétylénases, lipoxygénases, triacylglycérollipases, oxyde d'allène-synthases, hydroperoxyde-lyases ou acide gras-élongase(s).

5. Produit de construction génique selon la revendication 3 ou 4, **caractérisé en ce que** le produit de construction d'acide nucléique contient des gènes de biosynthèse supplémentaires du métabolisme des acides gras ou des lipides, choisis dans le groupe des Δ-4-désaturases, Δ-5-désaturases, Δ-6-désaturases, Δ-8-désaturases, Δ-12-désaturases ou Δ-9-élongases.

6. Vecteur, contenant un acide nucléique selon la revendication 1 ou un produit de construction génique selon l'une quelconque des revendications 3 à 5.

7. Organisme non humain, transgénique pour au moins un produit de construction génique selon l'une quelconque des revendications 3 à 5 ou un vecteur selon la revendication 6 ou l'acide nucléique selon la revendication 1.

8. Organisme non humain transgénique, contenant au moins un acide nucléique selon la revendication 1, un produit de construction génique selon l'une quelconque des revendications 3 à 5, un vecteur selon la revendication 6, ou un organisme selon la revendication 7, l'organisme étant une plante.

9. Plante transgénique selon la revendication 8, **caractérisée en ce que** la plante est une plante oléagineuse ou une plante utile et est de préférence choisie dans le groupe constitué par l'arachide, le colza, le colza canola, le tournesol, le carthame (*Carthamus tinctoria*), le pavot, la moutarde, le chanvre, le ricin, l'olivier, le sésame, le souci, le grenadier, l'onagre, la molène à fleurs denses, le chardon, le noisetier, l'amandier, le macadamier, l'avocat, le laurier, la courge, le lin, le soja, le pistachier, la bourrache, le palmier à huile, le cocotier, le noyer, le maïs, le blé, le seigle, l'avoine, le triticale, le riz, l'orge, le cotonnier, le manioc ou le poivrier.

10. Élément transgénique d'une plante transgénique selon la revendication 8 ou 9, de préférence graines transgéniques d'une plante transgénique selon la revendication 8 ou 9.

11. Procédé pour l'isolement et la production d'huile, de graisse, de lipides et/ou d'acides gras libres, le procédé comprenant le pressage ou l'extraction des parties de plantes ou graines de plantes selon la revendication 10.

12. Procédé pour la fabrication d'aliments pour animaux, de produits alimentaires, de cosmétiques ou de produits pharmaceutiques, comportant les étapes du procédé selon la revendication 11 et le mélange de ces huiles, lipides et/ou acides gras libres avec des huiles animales, microbiennes ou végétales ou des lipides ou acides gras animaux, microbiens ou végétaux.

13. Utilisation de l'acide nucléique selon la revendication 1, des plantes transgéniques selon la revendication 9 ou de la partie transgénique d'une plante transgénique selon la revendication 10, pour la fabrication d'huile, de graisse, de lipides ou d'acides gras libres.

14. Utilisation de l'acide nucléique selon la revendication 1, de la plante transgénique selon la revendication 9 ou de la partie transgénique d'une plante transgénique selon la revendication 10, pour la fabrication d'acide gras, ou de composition d'acide gras, polyinsaturé, chimiquement pur, pour des applications dans le secteur de l'industrie alimentaire, de l'industrie des cosmétiques ou de l'industrie pharmaceutique.


### SEQUENCE LISTING

<110> BASF Plant Science GmbH
<120> Verfahren zur Herstellung von mehrfach ungesättigten C20- und C22- Fettsäuren mit mindestens vier Doppelbindungen in transgenen Pflanzen
<130> 2005/0115
<140> PF 56465
   <141> 2005-03-22
<160> 55
<170> PatentIn version 3.1
<210> 1
   <211> 1371
   <212> DNA
   <213> Phytophthora sojae
<220>
   <221> CDS
   <222> (1)..(1371)
   <223> Delta-6-Desaturase
<400> 1
   atg gtg gat ggc ccc aag acc aag cgc ctc atc tcg tgg cag gag atc       48
   Met Val Asp Gly Pro Lys Thr Lys Arg Leu Ile Ser Trp Gln Glu Ile
   1               5                   10                  15
   cag cag cac tcg acg tac gcc aac gcg tgg atc gtc atc cac cac aag       96
   Gln Gln His Ser Thr Tyr Ala Asn Ala Trp Ile Val Ile His His Lys
               20                  25                  30
   gtc tac gac atc tcc aag tgg gac tcg cac ccg ggc ggc atg gtc atg      144
   Val Tyr Asp Ile Ser Lys Trp Asp Ser His Pro Gly Gly Met Val Met
           35                  40                  45
   ctc tcg cag gcc ggc gag gac gcc acc gac atc ttc acc gtg tgc cac      192
   Leu Ser Gln Ala Gly Glu Asp Ala Thr Asp Ile Phe Thr Val Cys His
       50                  55                  60
   ccg acg agc tcc tgg aag ctg ctg gag cag ttc tac atc ggc gac gtg       240
   Pro Thr Ser Ser Trp Lys Leu Leu Glu Gln Phe Tyr Ile Gly Asp Val
   65                  70                  75                  80
   gac gag agc acg gcc ccg ggc acg acc ggc ctc tcg gag gag cag aag       288
   Asp Glu Ser Thr Ala Pro Gly Thr Thr Gly Leu Ser Glu Glu Gln Lys
                   85                  90                  95
   gcc aag aag gcc aag acg aac gag ttc atc agc gcc tac cgc cgc ctg       336
   Ala Lys Lys Ala Lys Thr Asn Glu Phe Ile Ser Ala Tyr Arg Arg Leu
               100                 105                 110
   cgc atc aag atc aag ggc atg ggc ctc tac gac gcg tcc atg gtc tac       384
   Arg Ile Lys Ile Lys Gly Met Gly Leu Tyr Asp Ala Ser Met Val Tyr
           115                 120                 125
   tac gcg tgg aag atc ctc agc acc ttc ggc att tgg atg gcc tcc gtg       432
   Tyr Ala Trp Lys Ile Leu Ser Thr Phe Gly Ile Trp Met Ala Ser Val
       130                 135                 140
   gcg atc tgc tac cac ttc gac agc tgg ccc atg tac atg ctc gcg gcc       480
   Ala Ile Cys Tyr His Phe Asp Ser Trp Pro Met Tyr Met Leu Ala Ala
   145                 150                 155                 160
   tgc gtc atg ggg ctc ttc tgg cag cag tcg ggc tgg ctc gcg cac gac       528
   Cys Val Met Gly Leu Phe Trp Gln Gln Ser Gly Trp Leu Ala His Asp
                   165                 170                 175
   gtg ctg cac cac caa gtg tgg gac aac cac atg atc ggc aac gtc atg       576
   Val Leu His His Gln Val Trp Asp Asn His Met Ile Gly Asn Val Met
               180                 185                 190
   ggc gtc atc atc ggc gac gtc tgg atg ggc ttc agc gtg cag tgg tgg       624
   Gly Val Ile Ile Gly Asp Val Trp Met Gly Phe Ser Val Gln Trp Trp
           195                 200                 205
   aag aac aag cac aac ttc cac cac gcc gtg ccc aac ctc atc ggc gac       672
   Lys Asn Lys His Asn Phe His His Ala Val Pro Asn Leu Ile Gly Asp
       210                 215                 220
   gaa aag acc aag tac ctc ggc gac ccg gac atc gac acc atg ccc ctg       720
   Glu Lys Thr Lys Tyr Leu Gly Asp Pro Asp Ile Asp Thr Met Pro Leu
   225                 230                 235                 240
   ctg gcc tgg agc aag cac atg gcc tcg cgc gcg tac gag tcg tcg tgg       768
   Leu Ala Trp Ser Lys His Met Ala Ser Arg Ala Tyr Glu Ser Ser Trp
                   245                 250                 255
   ggc ccc ttc ttc gtg agc cac cag gcc gtc atc tac ttc ccg ctg ctg       816
   Gly Pro Phe Phe Val Ser His Gln Ala Val Ile Tyr Phe Pro Leu Leu
               260                 265                 270
   ctc ttc gcg cgc ttc agc tgg ctg ctg cag agc tac tac tac gtc ttc       864
   Leu Phe Ala Arg Phe Ser Trp Leu Leu Gln Ser Tyr Tyr Tyr Val Phe
           275                 280                 285
   aag ggc ttc gcc ttc ggc aag tac gac ccc gtg gac ctc ccg aac ggc       912
   Lys Gly Phe Ala Phe Gly Lys Tyr Asp Pro Val Asp Leu Pro Asn Gly
       290                 295                 300
   gag aag ctc ggc ctc tcg ctg cac tac ctc tgg aac gtg ctg ctg ccg       960
   Glu Lys Leu Gly Leu Ser Leu His Tyr Leu Trp Asn Val Leu Leu Pro
   305                 310                 315                 320
   gtg ctc acc ggc atg tcg gtg gcc cag ggc ctg gcc ttc ttc atg ctc      1008
   Val Leu Thr Gly Met Ser Val Ala Gln Gly Leu Ala Phe Phe Met Leu
                   325                 330                 335
   tcg cag atg tcg tgc ggc gcc ttc ctg gcc gcc gtc ttc agc gtc ggc      1056
   Ser Gln Met Ser Cys Gly Ala Phe Leu Ala Ala Val Phe Ser Val Gly
               340                 345                 350
   cac aac ggc atg tcg gtg tac gag cgc gac cag aag ccc gac ttc tgg      1104
   His Asn Gly Met Ser Val Tyr Glu Arg Asp Gln Lys Pro Asp Phe Trp
           355                 360                 365
   cag ctg cag gtc acc acc acg cgc aac atc acg ccg ggc ttc ttc atg      1152
   Gln Leu Gln Val Thr Thr Thr Arg Asn Ile Thr Pro Gly Phe Phe Met
       370                 375                 380
   gac tgg ttc tgc ggc ggc ctc aac tac cag atc gag cac cac ttg ttc      1200
   Asp Trp Phe Cys Gly Gly Leu Asn Tyr Gln Ile Glu His His Leu Phe
   385                 390                 395                 400
   ccc atg atg ccg cgc cac aac ctg cag aag gtc aac ccg ctc gtc aag      1248
   Pro Met Met Pro Arg His Asn Leu Gln Lys Val Asn Pro Leu Val Lys
                   405                 410                 415
   tcg ctc tgc aag cag tac gac gtc aag ttc cac gag acg ggc ttc tac      1296
   Ser Leu Cys Lys Gln Tyr Asp Val Lys Phe His Glu Thr Gly Phe Tyr
               420                 425                 430
   cgc gga ctc gtc gag gtc gtg gac gag ctg gcc gac atc agc aag gag      1344
   Arg Gly Leu Val Glu Val Val Asp Glu Leu Ala Asp Ile Ser Lys Glu
           435                 440                 445
   ttc ctg ctc gag ttc ccg gcc atg taa                                  1371
   Phe Leu Leu Glu Phe Pro Ala Met
       450                 455
<210> 2
   <211> 456
   <212> PRT
   <213> Phytophthora sojae
<400> 2
   Met Val Asp Gly Pro Lys Thr Lys Arg Leu Ile Ser Trp Gln Glu Ile
   1               5                   10                  15
   Gln Gln His Ser Thr Tyr Ala Asn Ala Trp Ile Val Ile His His Lys
               20                  25                  30
   Val Tyr Asp Ile Ser Lys Trp Asp Ser His Pro Gly Gly Met Val Met
           35                  40                  45
   Leu Ser Gln Ala Gly Glu Asp Ala Thr Asp Ile Phe Thr Val Cys His
       50                  55                  60
   Pro Thr Ser Ser Trp Lys Leu Leu Glu Gln Phe Tyr Ile Gly Asp Val
   65                  70                  75                  80
   Asp Glu Ser Thr Ala Pro Gly Thr Thr Gly Leu Ser Glu Glu Gln Lys
                   85                  90                  95
   Ala Lys Lys Ala Lys Thr Asn Glu Phe Ile Ser Ala Tyr Arg Arg Leu
               100                 105                 110
   Arg Ile Lys Ile Lys Gly Met Gly Leu Tyr Asp Ala Ser Met Val Tyr
           115                 120                 125
   Tyr Ala Trp Lys Ile Leu Ser Thr Phe Gly Ile Trp Met Ala Ser Val
       130                 135                 140
   Ala Ile Cys Tyr His Phe Asp Ser Trp Pro Met Tyr Met Leu Ala Ala
   145                 150                 155                 160
   Cys Val Met Gly Leu Phe Trp Gln Gln Ser Gly Trp Leu Ala His Asp
                   165                 170                 175
   Val Leu His His Gln Val Trp Asp Asn His Met Ile Gly Asn Val Met
               180                 185                 190
   Gly Val Ile Ile Gly Asp Val Trp Met Gly Phe Ser Val Gln Trp Trp
           195                 200                 205
   Lys Asn Lys His Asn Phe His His Ala Val Pro Asn Leu Ile Gly Asp
       210                 215                 220
   Glu Lys Thr Lys Tyr Leu Gly Asp Pro Asp Ile Asp Thr Met Pro Leu
   225                 230                 235                 240
   Leu Ala Trp Ser Lys His Met Ala Ser Arg Ala Tyr Glu Ser Ser Trp
                   245                 250                 255
   Gly Pro Phe Phe Val Ser His Gln Ala Val Ile Tyr Phe Pro Leu Leu
               260                 265                 270
   Leu Phe Ala Arg Phe Ser Trp Leu Leu Gln Ser Tyr Tyr Tyr Val Phe
           275                 280                 285
   Lys Gly Phe Ala Phe Gly Lys Tyr Asp Pro Val Asp Leu Pro Asn Gly
       290                 295                 300
   Glu Lys Leu Gly Leu Ser Leu His Tyr Leu Trp Asn Val Leu Leu Pro
   305                 310                 315                 320
   Val Leu Thr Gly Met Ser Val Ala Gln Gly Leu Ala Phe Phe Met Leu
                   325                 330                 335
   Ser Gln Met Ser Cys Gly Ala Phe Leu Ala Ala Val Phe Ser Val Gly
               340                 345                 350
   His Asn Gly Met Ser Val Tyr Glu Arg Asp Gln Lys Pro Asp Phe Trp
           355                 360                 365
   Gln Leu Gln Val Thr Thr Thr Arg Asn Ile Thr Pro Gly Phe Phe Met
       370                 375                 380
   Asp Trp Phe Cys Gly Gly Leu Asn Tyr Gln Ile Glu His His Leu Phe
   385                 390                 395                 400
   Pro Met Met Pro Arg His Asn Leu Gln Lys Val Asn Pro Leu Val Lys
                   405                 410                 415
   Ser Leu Cys Lys Gln Tyr Asp Val Lys Phe His Glu Thr Gly Phe Tyr
               420                 425                 430
   Arg Gly Leu Val Glu Val Val Asp Glu Leu Ala Asp Ile Ser Lys Glu
           435                 440                 445
   Phe Leu Leu Glu Phe Pro Ala Met
       450                 455
<210> 3
   <211> 915
   <212> DNA
   <213> Phytophthora sojae
<220>
   <221> CDS
   <222> (1)..(915)
   <223> Delta-6-Elongase
<400> 3
   atg gag acg acc ttc gcg cgc acg ccc aag ctg cac gac gag gtg ccg        48
   Met Glu Thr Thr Phe Ala Arg Thr Pro Lys Leu His Asp Glu Val Pro
   1               5                   10                  15
   gcg ctg cgc tgg atg tac ccg tcc cag tac gag cgc gac tgg cta cac        96
   Ala Leu Arg Trp Met Tyr Pro Ser Gln Tyr Glu Arg Asp Trp Leu His
               20                  25                  30
   tat gcc tgg aac cga cgg ccc act aac ttc tct cgt atc ttc cgc cag       144
   Tyr Ala Trp Asn Arg Arg Pro Thr Asn Phe Ser Arg Ile Phe Arg Gln
           35                  40                  45
   gcc atc tcg tgg gag ccg ggc ttc tgc atg gag tcg ctg ccc atg gcc       192
   Ala Ile Ser Trp Glu Pro Gly Phe Cys Met Glu Ser Leu Pro Met Ala
       50                  55                  60
   gtg gcg ctg tgc gcc gcc tac tgc gtg ctg tgc ttc gcc ggc cgc cgc       240
   Val Ala Leu Cys Ala Ala Tyr Cys Val Leu Cys Phe Ala Gly Arg Arg
   65                  70                  75                  80
   gtc atg cgc gac ctc aag ccc ttc aac ctc aaa gtc ccg ctc gcg ctc       288
   Val Met Arg Asp Leu Lys Pro Phe Asn Leu Lys Val Pro Leu Ala Leu
                   85                  90                  95
   tgg aac ctg gcg ctg gcc acg ttc agc gcc atc ggc gcc tcc agg acg       336
   Trp Asn Leu Ala Leu Ala Thr Phe Ser Ala Ile Gly Ala Ser Arg Thr
               100                 105                 110
   gtg ccc ttc ctc atc aac acc gtc tac cgc cgc ggc gtg tac cac tcg       384
   Val Pro Phe Leu Ile Asn Thr Val Tyr Arg Arg Gly Val Tyr His Ser
           115                 120                 125
   gtg tgc gcg ccg ccc acg ccg cac tac ggc cac ggc ccc gtg gcg ctc       432
   Val Cys Ala Pro Pro Thr Pro His Tyr Gly His Gly Pro Val Ala Leu
       130                 135                 140
   tgg gtc atg ctc ttc atc ttc tcc aag gtg ccg gag ctc gtg gac acg       480
   Trp Val Met Leu Phe Ile Phe Ser Lys Val Pro Glu Leu Val Asp Thr
   145                 150                 155                 160
   gcc ttc atc gtg ctg cgc aag aag ccg ctc atc ttc ctg cac tgg tac       528
   Ala Phe Ile Val Leu Arg Lys Lys Pro Leu Ile Phe Leu His Trp Tyr
                   165                 170                 175
   cac cac atc acc gtg ctg ctc ttc tgc tgg cac gcg ttc gcc acg ctc       576
   His His Ile Thr Val Leu Leu Phe Cys Trp His Ala Phe Ala Thr Leu
               180                 185                 190
   tcg gct agc ggc ctg tac ttc gtg gcc atg aac tac tcg gtg cac gcc       624
   Ser Ala Ser Gly Leu Tyr Phe Val Ala Met Asn Tyr Ser Val His Ala
           195                 200                 205
   atc atg tac ttc tac tac ttc ctg acg gcg tgc ggc tac cga ccg cgc       672
   Ile Met Tyr Phe Tyr Tyr Phe Leu Thr Ala Cys Gly Tyr Arg Pro Arg
       210                 215                 220
   tgg gct cgc ctc gtg acg atc ttc cag ctg agc cag atg ggc gtg ggc       720
   Trp Ala Arg Leu Val Thr Ile Phe Gln Leu Ser Gln Met Gly Val Gly
   225                 230                 235                 240
   gtc gcc gtg tgc ggc ctc aac gtg tac tac atg aag cag ggc gcc acg       768
   Val Ala Val Cys Gly Leu Asn Val Tyr Tyr Met Lys Gln Gly Ala Thr
                   245                 250                 255
   tgc agc gtg gac ccg gac aac ctc aag tgg ggc atc atc atg tac tcg       816
   Cys Ser Val Asp Pro Asp Asn Leu Lys Trp Gly Ile Ile Met Tyr Ser
               260                 265                 270
   agc tac ttt gcg ctc ttc ctc aag ttc ttc atc gag cgc tac ctg ctg      864
   Ser Tyr Phe Ala Leu Phe Leu Lys Phe Phe Ile Glu Arg Tyr Leu Leu
           275                 280                 285
   cgc agc gcc aag aag ccc gcc gcc gct gcg gtc gcc aag aag acg cag      912
   Arg Ser Ala Lys Lys Pro Ala Ala Ala Ala Val Ala Lys Lys Thr Gln
       290                 295                 300
   taa                                                                  915
<210> 4
   <211> 304
   <212> PRT
   <213> Phytophthora sojae
<400> 4
   Met Glu Thr Thr Phe Ala Arg Thr Pro Lys Leu His Asp Glu Val Pro
   1               5                   10                  15
   Ala Leu Arg Trp Met Tyr Pro Ser Gln Tyr Glu Arg Asp Trp Leu His
               20                  25                  30
   Tyr Ala Trp Asn Arg Arg Pro Thr Asn Phe Ser Arg Ile Phe Arg Gln
           35                  40                  45
   Ala Ile Ser Trp Glu Pro Gly Phe Cys Met Glu Ser Leu Pro Met Ala
       50                  55                  60
   Val Ala Leu Cys Ala Ala Tyr Cys Val Leu Cys Phe Ala Gly Arg Arg
   65                  70                  75                  80
   Val Met Arg Asp Leu Lys Pro Phe Asn Leu Lys Val Pro Leu Ala Leu
                   85                  90                  95
   Trp Asn Leu Ala Leu Ala Thr Phe Ser Ala Ile Gly Ala Ser Arg Thr
               100                 105                 110
   Val Pro Phe Leu Ile Asn Thr Val Tyr Arg Arg Gly Val Tyr His Ser
           115                 120                 125
   Val Cys Ala Pro Pro Thr Pro His Tyr Gly His Gly Pro Val Ala Leu
       130                 135                 140
   Trp Val Met Leu Phe Ile Phe Ser Lys Val Pro Glu Leu Val Asp Thr
   145                 150                 155                 160
   Ala Phe Ile Val Leu Arg Lys Lys Pro Leu Ile Phe Leu His Trp Tyr
                   165                 170                 175
   His His Ile Thr Val Leu Leu Phe Cys Trp His Ala Phe Ala Thr Leu
               180                 185                 190
   Ser Ala Ser Gly Leu Tyr Phe Val Ala Met Asn Tyr Ser Val His Ala
           195                 200                 205
   Ile Met Tyr Phe Tyr Tyr Phe Leu Thr Ala Cys Gly Tyr Arg Pro Arg
       210                 215                 220
   Trp Ala Arg Leu Val Thr Ile Phe Gln Leu Ser Gln Met Gly Val Gly
   225                 230                 235                 240
   Val Ala Val Cys Gly Leu Asn Val Tyr Tyr Met Lys Gln Gly Ala Thr
                   245                 250                 255
   Cys Ser Val Asp Pro Asp Asn Leu Lys Trp Gly Ile Ile Met Tyr Ser
               260                 265                 270
   Ser Tyr Phe Ala Leu Phe Leu Lys Phe Phe Ile Glu Arg Tyr Leu Leu
           275                 280                 285
   Arg Ser Ala Lys Lys Pro Ala Ala Ala Ala Val Ala Lys Lys Thr Gln
       290                 295                 300
<210> 5
   <211> 837
   <212> DNA
   <213> Phytophthora sojae
<220>
   <221> CDS
   <222> (1)..(837)
   <223> Delta-6-Elongase
<400> 5
   atg gcg tcg gag ctg ctg cag agt tac tac gag tgg gcg aat gcc acc      48
   Met Ala Ser Glu Leu Leu Gln Ser Tyr Tyr Glu Trp Ala Asn Ala Thr
   1               5                   10                  15
   gag atc aag gtg ctc gac tgg gtg gac ccc gaa ggc ggc tgg aag gtc      96
   Glu Ile Lys Val Leu Asp Trp Val Asp Pro Glu Gly Gly Trp Lys Val
                   20                  25                  30
   cac ccc atg gcg gac tac ccg ctc gcc aac ttc gcc agc gtg ttc gcc      144
   His Pro Met Ala Asp Tyr Pro Leu Ala Asn Phe Ala Ser Val Phe Ala
           35                  40                  45
   atc tgc gtc ggc tac ctg ctc ttc gtc atc ttc ggc acg gcc ctg atg      192
   Ile Cys Val Gly Tyr Leu Leu Phe Val Ile Phe Gly Thr Ala Leu Met
       50                  55                  60
   aag atg ggc atc ccc gcc atc aag acg agc ccc atc cag ttc atc tac      240
   Lys Met Gly Ile Pro Ala Ile Lys Thr Ser Pro Ile Gln Phe Ile Tyr
   65                  70                  75                  80
   aac ccc atc cag gtc atc gcc tgc tcc tac atg ttc gtg gag acc gcc      288
   Asn Pro Ile Gln Val Ile Ala Cys Ser Tyr Met Phe Val Glu Thr Ala
                   85                  90                  95
   atc cag gcc tac cgc aat ggg tac tcg cca gct ccg tgc aac gcc ttc      336
   Ile Gln Ala Tyr Arg Asn Gly Tyr Ser Pro Ala Pro Cys Asn Ala Phe
               100                 105                 110
   aag acg gac gcg ccc gtc atg ggc aac gtg ctc tac ctg ttc tac ctg      384
   Lys Thr Asp Ala Pro Val Met Gly Asn Val Leu Tyr Leu Phe Tyr Leu
           115                 120                 125
   tcc aag atg ctg gac ctg tgc gac acc ttc ttc atc gtc gtg ggc aag      432
   Ser Lys Met Leu Asp Leu Cys Asp Thr Phe Phe Ile Val Val Gly Lys
       130                 135                 140
   aaa tgg cgc cag ctc tcg ttc ctg cac gtg tac cac cac ctc tcg gtg      480
   Lys Trp Arg Gln Leu Ser Phe Leu His Val Tyr His His Leu Ser Val
   145                 150                 155                 160
   ctg ctc atg tac tac atc gtc ttc cgc gtg gcg cag gac ggc gac tcg      528
   Leu Leu Met Tyr Tyr Ile Val Phe Arg Val Ala Gln Asp Gly Asp Ser
                   165                 170                 175
   tac gcg tcc gtc gtg ctc aac ggc ttc gtg cac acc atc atg tac acg      576
   Tyr Ala Ser Val Val Leu Asn Gly Phe Val His Thr Ile Met Tyr Thr
               180                 185                 190
   tac tac ttc gtg agc gcg cac acg cgg gac att tgg tgg aag cgc tac      624
   Tyr Tyr Phe Val Ser Ala His Thr Arg Asp Ile Trp Trp Lys Arg Tyr
           195                 200                 205
   ctg acg ctc att cag ttg gtg cag ttc gtg acc atg aac gtg cag ggc      672
   Leu Thr Leu Ile Gln Leu Val Gln Phe Val Thr Met Asn Val Gln Gly
       210                 215                 220
   tac ctc atg tac tcg cgc cag tgc cca ggc atg ccg ccc aag atc ccg      720
   Tyr Leu Met Tyr Ser Arg Gln Cys Pro Gly Met Pro Pro Lys Ile Pro
   225                 230                 235                 240
   ctc atc tat ctg gcc tac gtg cag tcg ctc ttc tgg ctg ttc gtc aac      768
   Leu Ile Tyr Leu Ala Tyr Val Gln Ser Leu Phe Trp Leu Phe Val Asn
                   245                 250                 255
   ttc tac gtg cgc tcg tac gtg ctc gcc ccc aag aag acc aag gcg tcc      816
   Phe Tyr Val Arg Ser Tyr Val Leu Ala Pro Lys Lys Thr Lys Ala Ser
               260                 265                 270
   ccg gcc aag aag aac ctc taa                                          837
   Pro Ala Lys Lys Asn Leu
   275
<210> 6
   <211> 278
   <212> PRT
   <213> Phytophthora sojae
<400> 6
   Met Ala Ser Glu Leu Leu Gln Ser Tyr Tyr Glu Trp Ala Asn Ala Thr
   1               5                   10                  15
   Glu Ile Lys Val Leu Asp Trp Val Asp Pro Glu Gly Gly Trp Lys Val
               20                  25                  30
   His Pro Met Ala Asp Tyr Pro Leu Ala Asn Phe Ala Ser Val Phe Ala
           35                  40                  45
   Ile Cys Val Gly Tyr Leu Leu Phe Val Ile Phe Gly Thr Ala Leu Met
       50                  55                  60
   Lys Met Gly Ile Pro Ala Ile Lys Thr Ser Pro Ile Gln Phe Ile Tyr
   65                  70                  75                  80
   Asn Pro Ile Gln Val Ile Ala Cys Ser Tyr Met Phe Val Glu Thr Ala
                   85                  90                  95
   Ile Gln Ala Tyr Arg Asn Gly Tyr Ser Pro Ala Pro Cys Asn Ala Phe
               100                 105                 110
   Lys Thr Asp Ala Pro Val Met Gly Asn Val Leu Tyr Leu Phe Tyr Leu
           115                 120                 125
   Ser Lys Met Leu Asp Leu Cys Asp Thr Phe Phe Ile Val Val Gly Lys
       130                 135                 140
   Lys Trp Arg Gln Leu Ser Phe Leu His Val Tyr His His Leu Ser Val
   145                 150                 155                 160
   Leu Leu Met Tyr Tyr Ile Val Phe Arg Val Ala Gln Asp Gly Asp Ser
                   165                 170                 175
   Tyr Ala Ser Val Val Leu Asn Gly Phe Val His Thr Ile Met Tyr Thr
               180                 185                 190
   Tyr Tyr Phe Val Ser Ala His Thr Arg Asp Ile Trp Trp Lys Arg Tyr
                   195                 200                 205
   Leu Thr Leu Ile Gln Leu Val Gln Phe Val Thr Met Asn Val Gln Gly
       210                 215                 220
   Tyr Leu Met Tyr Ser Arg Gln Cys Pro Gly Met Pro Pro Lys Ile Pro
   225                 230                 235                 240
   Leu Ile Tyr Leu Ala Tyr Val Gln Ser Leu Phe Trp Leu Phe Val Asn
                   245                 250                 255
   Phe Tyr Val Arg Ser Tyr Val Leu Ala Pro Lys Lys Thr Lys Ala Ser
               260                 265                 270
   Pro Ala Lys Lys Asn Leu
           275
<210> 7
   <211> 837
   <212> DNA
   <213> Phytophthora sojae
<220>
   <221> CDS
   <222> (1)..(837)
   <223> Delta-6-Elongase
<400> 7
   atg tcg gcc gac ctg ctg cag agc tac tac gac tgg acc aac gcc acc        48
   Met Ser Ala Asp Leu Leu Gln Ser Tyr Tyr Asp Trp Thr Asn Ala Thr
   1               5                   10                  15
   gag gcc aag ctg ctc gac tgg gtg gac ccc gaa ggc ggc tgg aag gtc        96
   Glu Ala Lys Leu Leu Asp Trp Val Asp Pro Glu Gly Gly Trp Lys Val
               20                  25                  30
   cac ccc atg gcc gac tac ccg ctc gcc aac ttc gcc agc gtc tac gcc       144
   His Pro Met Ala Asp Tyr Pro Leu Ala Asn Phe Ala Ser Val Tyr Ala
           35                  40                  45
   atc tgc gtc ggc tac ctg ctc ttc gtc atc ttc ggc acg gcc ctg atg       192
   Ile Cys Val Gly Tyr Leu Leu Phe Val Ile Phe Gly Thr Ala Leu Met
       50                  55                  60
   aag atg ggc atc ccc gcc atc aag acg agc ccc atc cag ttc atc tac       240
   Lys Met Gly Ile Pro Ala Ile Lys Thr Ser Pro Ile Gln Phe Ile Tyr
   65                  70                  75                  80
   aac ccc atc cag gtc atc gcc tgc tcc tac atg tgc gtg gag gcc gcc      288
   Asn Pro Ile Gln Val Ile Ala Cys Ser Tyr Met Cys Val Glu Ala Ala
                   85                  90                  95
   atc cag gcc tac cgc aac ggc tac agc gcg gcg ccc tgc aac gcc ttc      336
   Ile Gln Ala Tyr Arg Asn Gly Tyr Ser Ala Ala Pro Cys Asn Ala Phe
               100                 105                 110
   aag gcg gac gcg ccc gtc atg ggc aac gtg ctc tac ctg ttc tac ctg      384
   Lys Ala Asp Ala Pro Val Met Gly Asn Val Leu Tyr Leu Phe Tyr Leu
           115                 120                 125
   tcc aag atg ctg gac ctg tgc gac acg gtc ttc atc atc ctc ggc aag      432
   Ser Lys Met Leu Asp Leu Cys Asp Thr Val Phe Ile Ile Leu Gly Lys
       130                 135                 140
   aag tgg aag cag ctc tcc atc ctg cac gtg tac cac cac ctg acc gtg      480
   Lys Trp Lys Gln Leu Ser Ile Leu His Val Tyr His His Leu Thr Val
   145                 150                 155                 160
   ctc ttc gtc tac tac gtg acg ttc cgc gcc gcc cag gac ggc gac tcg      528
   Leu Phe Val Tyr Tyr Val Thr Phe Arg Ala Ala Gln Asp Gly Asp Ser
                   165                 170                 175
   tac gcc acc atc gtg ctc aac ggc ttc gtg cac acc atc atg tac acg      576
   Tyr Ala Thr Ile Val Leu Asn Gly Phe Val His Thr Ile Met Tyr Thr
               180                 185                 190
   tac tac ttc gtg agc gcg cac acg cgc aac att tgg tgg aag aag tac      624
   Tyr Tyr Phe Val Ser Ala His Thr Arg Asn Ile Trp Trp Lys Lys Tyr
           195                 200                 205
   ctc acg cgc atc cag ctc atc cag ttc gtg acc atg aac gtg cag ggc      672
   Leu Thr Arg Ile Gln Leu Ile Gln Phe Val Thr Met Asn Val Gln Gly
       210                 215                 220
   tac ctg acg tac tcg cgc cag tgc ccg ggc atg ccc ccc aag gtg ccg      720
   Tyr Leu Thr Tyr Ser Arg Gln Cys Pro Gly Met Pro Pro Lys Val Pro
   225                 230                 235                 240
   ctc atg tac ctc gtg tac gtg cag tcg ctc ttc tgg ctc ttc atg aac      768
   Leu Met Tyr Leu Val Tyr Val Gln Ser Leu Phe Trp Leu Phe Met Asn
                   245                 250                 255
   ttc tac atc cgc gcg tac gtc ttc ggc ccc aag aag ccc gcc gtc gag      816
   Phe Tyr Ile Arg Ala Tyr Val Phe Gly Pro Lys Lys Pro Ala Val Glu
               260                 265                 270
   gac gcc aag aag aag ctc taa                                          837
   Asp Ala Lys Lys Lys Leu
           275
<210> 8
   <211> 278
   <212> PRT
   <213> Phytophthora sojae
<400> 8
   Met Ser Ala Asp Leu Leu Gln Ser Tyr Tyr Asp Trp Thr Asn Ala Thr
   1 5 10 15
   Glu Ala Lys Leu Leu Asp Trp Val Asp Pro Glu Gly Gly Trp Lys Val
   20 25 30
   His Pro Met Ala Asp Tyr Pro Leu Ala Asn Phe Ala Ser Val Tyr Ala
   35 40 45
   Ile Cys Val Gly Tyr Leu Leu Phe Val Ile Phe Gly Thr Ala Leu Met
   50 55 60
   Lys Met Gly Ile Pro Ala Ile Lys Thr Ser Pro Ile Gln Phe Ile Tyr
   65 70 75 80
   Asn Pro Ile Gln Val Ile Ala Cys Ser Tyr Met Cys Val Glu Ala Ala
   85 90 95
   Ile Gln Ala Tyr Arg Asn Gly Tyr Ser Ala Ala Pro Cys Asn Ala Phe
   100 105 110
   Lys Ala Asp Ala Pro Val Met Gly Asn Val Leu Tyr Leu Phe Tyr Leu
   115 120 125
   Ser Lys Met Leu Asp Leu Cys Asp Thr Val Phe Ile Ile Leu Gly Lys
   130 135 140
   Lys Trp Lys Gln Leu Ser Ile Leu His Val Tyr His His Leu Thr Val
   145 150 155 160
   Leu Phe Val Tyr Tyr Val Thr Phe Arg Ala Ala Gln Asp Gly Asp Ser
   165 170 175
   Tyr Ala Thr Ile Val Leu Asn Gly Phe Val His Thr Ile Met Tyr Thr
   180 185 190
   Tyr Tyr Phe Val Ser Ala His Thr Arg Asn Ile Trp Trp Lys Lys Tyr
   195 200 205
   Leu Thr Arg Ile Gln Leu Ile Gln Phe Val Thr Met Asn Val Gln Gly
   210 215 220
   Tyr Leu Thr Tyr Ser Arg Gln Cys Pro Gly Met Pro Pro Lys Val Pro
   225 230 235 240
   Leu Met Tyr Leu Val Tyr Val Gln Ser Leu Phe Trp Leu Phe Met Asn
   245 250 255
   Phe Tyr Ile Arg Ala Tyr Val Phe Gly Pro Lys Lys Pro Ala Val Glu
               260                 265                 270
   Asp Ala Lys Lys Lys Leu
           275
<210> 9
   <211> 1497
   <212> DNA
   <213> Phytophthora sojae
<220>
   <221> CDS
   <222> (1)..(1497)
   <223> Delta-5-Desaturase
<400> 9
   atg gcc ccc atc gag acc gac aag gcc gtg agt gcc aac gag ggc ctg      48
   Met Ala Pro Ile Glu Thr Asp Lys Ala Val Ser Ala Asn Glu Gly Leu
   1               5                   10                  15
   cac cag cgc aag ggc gcc gcc tcg gcc gac aag gac gcc acc tac acg      96
   His Gln Arg Lys Gly Ala Ala Ser Ala Asp Lys Asp Ala Thr Tyr Thr
               20                  25                  30
   tgg cag gac gtg gcc aag cac aac acg gcc aag agc gcc tgg gtc atc     144
   Trp Gln Asp Val Ala Lys His Asn Thr Ala Lys Ser Ala Trp Val Ile
           35                  40                  45
   atc cgc ggc gtc gtc tac gac gtc act gat act ctg aaa aca ccc caa     192
   Ile Arg Gly Val Val Tyr Asp Val Thr Asp Thr Leu Lys Thr Pro Gln
       50                  55                  60
   agt aaa atc cta acc tgt tcc tgc gtg atg gca cca cca gaa tgg gcg     240
   Ser Lys Ile Leu Thr Cys Ser Cys Val Met Ala Pro Pro Glu Trp Ala
   65                  70                  75                  80
   gac cgt cac cct ggc ggc cgc gag ctc gtg ctg ctg cac gcc gga cgc     288
   Asp Arg His Pro Gly Gly Arg Glu Leu Val Leu Leu His Ala Gly Arg
                   85                  90                  95
   gag tgc acg gac acg ttc gac tcg tac cac ccc ttc tcc aac cgc gcc     336
   Glu Cys Thr Asp Thr Phe Asp Ser Tyr His Pro Phe Ser Asn Arg Ala
               100                 105                 110
   gac aag atc ctg gcc aag tac gcc gtt ggt aag ctc gtg ggc ggc tcc     384
   Asp Lys Ile Leu Ala Lys Tyr Ala Val Gly Lys Leu Val Gly Gly Ser
           115                 120                 125
   gag ttc ccc cag tac aag ccc gac acg ggc ttc tac aag gag tgc tgc     432
   Glu Phe Pro Gln Tyr Lys Pro Asp Thr Gly Phe Tyr Lys Glu Cys Cys
       130                 135                 140
   gag cgc gtg cac cag tac ttt aag gac aac aac ctg gac ccg cgc agc      480
   Glu Arg Val His Gln Tyr Phe Lys Asp Asn Asn Leu Asp Pro Arg Ser
   145                 150                 155                 160
   ccg tac tcg ggc atg tgg cgc atg atg atc gtc gct gcg ctc ggc gcc      528
   Pro Tyr Ser Gly Met Trp Arg Met Met Ile Val Ala Ala Leu Gly Ala
                   165                 170                 175
   atc tcg tac ctg ggc atg aac cag ctg ctc tcg gac aac atc tac gcg      576
   Ile Ser Tyr Leu Gly Met Asn Gln Leu Leu Ser Asp Asn Ile Tyr Ala
               180                 185                 190
   cac tac gcg tgg ggc gcc ctc ttc ggc gtg tgc cag gcg ctg ccg ctg      624
   His Tyr Ala Trp Gly Ala Leu Phe Gly Val Cys Gln Ala Leu Pro Leu
           195                 200                 205
   ctc cac gtg atg cac gac gcg tcg cac gcc gcc atc acc agc agc ccc      672
   Leu His Val Met His Asp Ala Ser His Ala Ala Ile Thr Ser Ser Pro
       210                 215                 220
   acg ggc tgg agg ctc att ggc cgc ttc gcg atg gac tgg gtg gcc ggc      720
   Thr Gly Trp Arg Leu Ile Gly Arg Phe Ala Met Asp Trp Val Ala Gly
   225                 230                 235                 240
   gcc aac atg gta tcg tgg ctc aac cag cac gtt gtg ggc cac cac atc      768
   Ala Asn Met Val Ser Trp Leu Asn Gln His Val Val Gly His His Ile
                   245                 250                 255
   tac aca aac gtg gcc ggc gct gac ccc gac ctt ccc gtg gac ttc aag      816
   Tyr Thr Asn Val Ala Gly Ala Asp Pro Asp Leu Pro Val Asp Phe Lys
               260                 265                 270
   agc gac gtg cgc cgc att gtg tac cgt cag gtg ctg ctg ccc atc tac      864
   Ser Asp Val Arg Arg Ile Val Tyr Arg Gln Val Leu Leu Pro Ile Tyr
           275                 280                 285
   aag ttc cag cac ttg tac ctg ccg ccg ctg tac ggc gtg ctc ggc ctc      912
   Lys Phe Gln His Leu Tyr Leu Pro Pro Leu Tyr Gly Val Leu Gly Leu
       290                 295                 300
   aag ttc cgc gtg cag gac att ttc gag acg ttc atc tcg ctc acg aac      960
   Lys Phe Arg Val Gln Asp Ile Phe Glu Thr Phe Ile Ser Leu Thr Asn
   305                 310                 315                 320
   ggt ccg ctg cgc gtg aac ccg cac tca gtc ggc gac tgg gtc gag atg     1008
   Gly Pro Leu Arg Val Asn Pro His Ser Val Gly Asp Trp Val Glu Met
                   325                 330                 335
   atc ctg tcc aag gcc ttc tgg gcg ttc tac cgc atc tac atc ccg ctg     1056
   Ile Leu Ser Lys Ala Phe Trp Ala Phe Tyr Arg Ile Tyr Ile Pro Leu
               340                 345                 350
   gtc gtg ctg cag gtg gac tcg tcc cgc ttc tgg ggc gtc ttc ttc ctg     1104
   Val Val Leu Gln Val Asp Ser Ser Arg Phe Trp Gly Val Phe Phe Leu
           355                 360                 365
   gcc gag ttc atg acg ggc tgg tac ctg gcc ttc aac ttc cag gtg agc     1152
   Ala Glu Phe Met Thr Gly Trp Tyr Leu Ala Phe Asn Phe Gln Val Ser
       370                 375                 380
   cac gtc tcc acg gcc tgc gaa tac ccg ggc ggt gac gag gag gtg acg      1200
   His Val Ser Thr Ala Cys Glu Tyr Pro Gly Gly Asp Glu Glu Val Thr
   385                 390                 395                 400
   gcc atc gag gac gag tgg gct gtc tcg cag atc aag tcg tcg gtg gac      1248
   Ala Ile Glu Asp Glu Trp Ala Val Ser Gln Ile Lys Ser Ser Val Asp
                   405                 410                 415
   tac ggc cac ggc tcg ttc ctc acg gcg ttc ctc acg ggc gcg ctg aac      1296
   Tyr Gly His Gly Ser Phe Leu Thr Ala Phe Leu Thr Gly Ala Leu Asn
               420                 425                 430
   tac cag gtg acc cac cac ctc ttc ccg ggc gtc tcg cag tac cac tac      1344
   Tyr Gln Val Thr His His Leu Phe Pro Gly Val Ser Gln Tyr His Tyr
           435                 440                 445
   ccg gcc atc gcg ccg atc atc atc gac gtg tgc aac aaa tac aag atc      1392
   Pro Ala Ile Ala Pro Ile Ile Ile Asp Val Cys Asn Lys Tyr Lys Ile
       450                 455                 460
   aag tac acg gtg ctc ccc acg ttc acg gag gcg ctg gcc ggc cac ttc      1440
   Lys Tyr Thr Val Leu Pro Thr Phe Thr Glu Ala Leu Ala Gly His Phe
   465                 470                 475                 480
   gac cac ctc gtc gtc atg ggc aag atg ggc aag ccc gtg tcc gtc cac      1488
   Asp His Leu Val Val Met Gly Lys Met Gly Lys Pro Val Ser Val His
                   485                 490                 495
   atg ggc taa                                                          1497
   Met Gly
<210> 10
   <211> 498
   <212> PRT
   <213> Phytophthora sojae
<400> 10
   Met Ala Pro Ile Glu Thr Asp Lys Ala Val Ser Ala Asn Glu Gly Leu
   1               5                   10                  15
   His Gln Arg Lys Gly Ala Ala Ser Ala Asp Lys Asp Ala Thr Tyr Thr
               20                  25                  30
   Trp Gln Asp Val Ala Lys His Asn Thr Ala Lys Ser Ala Trp Val Ile
           35                  40                  45
   Ile Arg Gly Val Val Tyr Asp Val Thr Asp Thr Leu Lys Thr Pro Gln
       50                  55                  60
   Ser Lys Ile Leu Thr Cys Ser Cys Val Met Ala Pro Pro Glu Trp Ala
   65 70 75 80
   Asp Arg His Pro Gly Gly Arg Glu Leu Val Leu Leu His Ala Gly Arg
   85 90 95
   Glu Cys Thr Asp Thr Phe Asp Ser Tyr His Pro Phe Ser Asn Arg Ala
   100 105 110
   Asp Lys Ile Leu Ala Lys Tyr Ala Val Gly Lys Leu Val Gly Gly Ser
   115 120 125
   Glu Phe Pro Gln Tyr Lys Pro Asp Thr Gly Phe Tyr Lys Glu Cys Cys
   130 135 140
   Glu Arg Val His Gln Tyr Phe Lys Asp Asn Asn Leu Asp Pro Arg Ser
   145 150 155 160
   Pro Tyr Ser Gly Met Trp Arg Met Met Ile Val Ala Ala Leu Gly Ala
   165 170 175
   Ile Ser Tyr Leu Gly Met Asn Gln Leu Leu Ser Asp Asn Ile Tyr Ala
   180 185 190
   His Tyr Ala Trp Gly Ala Leu Phe Gly Val Cys Gln Ala Leu Pro Leu
   195 200 205
   Leu His Val Met His Asp Ala Ser His Ala Ala Ile Thr Ser Ser Pro
   210 215 220
   Thr Gly Trp Arg Leu Ile Gly Arg Phe Ala Met Asp Trp Val Ala Gly
   225 230 235 240
   Ala Asn Met Val Ser Trp Leu Asn Gln His Val Val Gly His His Ile
   245 250 255
   Tyr Thr Asn Val Ala Gly Ala Asp Pro Asp Leu Pro Val Asp Phe Lys
   260 265 270
   Ser Asp Val Arg Arg Ile Val Tyr Arg Gln Val Leu Leu Pro Ile Tyr
   275 280 285
   Lys Phe Gln His Leu Tyr Leu Pro Pro Leu Tyr Gly Val Leu Gly Leu
   290 295 300
   Lys Phe Arg Val Gln Asp Ile Phe Glu Thr Phe Ile Ser Leu Thr Asn
   305 310 315 320
   Gly Pro Leu Arg Val Asn Pro His Ser Val Gly Asp Trp Val Glu Met
                   325                 330                 335
   Ile Leu Ser Lys Ala Phe Trp Ala Phe Tyr Arg Ile Tyr Ile Pro Leu
               340                 345                 350
   Val Val Leu Gln Val Asp Ser Ser Arg Phe Trp Gly Val Phe Phe Leu
           355                 360                 365
   Ala Glu Phe Met Thr Gly Trp Tyr Leu Ala Phe Asn Phe Gln Val Ser
       370                 375                 380
   His Val Ser Thr Ala Cys Glu Tyr Pro Gly Gly Asp Glu Glu Val Thr
   385                 390                 395                 400
   Ala Ile Glu Asp Glu Trp Ala Val Ser Gln Ile Lys Ser Ser Val Asp
                   405                 410                 415
   Tyr Gly His Gly Ser Phe Leu Thr Ala Phe Leu Thr Gly Ala Leu Asn
               420                 425                 430
   Tyr Gln Val Thr His His Leu Phe Pro Gly Val Ser Gln Tyr His Tyr
           435                 440                 445
   Pro Ala Ile Ala Pro Ile Ile Ile Asp Val Cys Asn Lys Tyr Lys Ile
       450                 455                 460
   Lys Tyr Thr Val Leu Pro Thr Phe Thr Glu Ala Leu Ala Gly His Phe
   465                 470                 475                 480
   Asp His Leu Val Val Met Gly Lys Met Gly Lys Pro Val Ser Val His
                   485                 490                 495
   Met Gly
<210> 11
   <211> 903
   <212> DNA
   <213> Ostreococcus tauri
<220>
   <221> CDS
   <222> (1)..(903)
   <223> Delta-5-Elongase
<400> 11
   atg tct gct tct gga gct ttg ttg cct gct att gct ttc gct gct tac      48
   Met Ser Ala Ser Gly Ala Leu Leu Pro Ala Ile Ala Phe Ala Ala Tyr
   1               5                   10                  15
   gct tac gct acc tac gct tat gct ttc gag tgg tct cat gct aac gga      96
   Ala Tyr Ala Thr Tyr Ala Tyr Ala Phe Glu Trp Ser His Ala Asn Gly
               20                  25                  30
   atc gat aac gtg gat gct aga gag tgg att gga gct ttg tct ttg aga     144
   Ile Asp Asn Val Asp Ala Arg Glu Trp Ile Gly Ala Leu Ser Leu Arg
           35                  40                  45
   ctc cct gca att gct acc acc atg tac ctc ttg ttc tgc ctt gtg gga     192
   Leu Pro Ala Ile Ala Thr Thr Met Tyr Leu Leu Phe Cys Leu Val Gly
       50                  55                  60
   cct aga ttg atg gct aag agg gag gct ttt gat cct aag gga ttc atg     240
   Pro Arg Leu Met Ala Lys Arg Glu Ala Phe Asp Pro Lys Gly Phe Met
   65                  70                  75                  80
   ctc gct tac aac gct tac caa acc gct ttc aac gtt gtg gtg ctc gga     288
   Leu Ala Tyr Asn Ala Tyr Gln Thr Ala Phe Asn Val Val Val Leu Gly
                   85                  90                  95
   atg ttc gct aga gag atc tct gga ttg gga caa cct gtt tgg gga tct     336
   Met Phe Ala Arg Glu Ile Ser Gly Leu Gly Gln Pro Val Trp Gly Ser
               100                 105                 110
   act atg cct tgg agc gat agg aag tcc ttc aag att ttg ttg gga gtg     384
   Thr Met Pro Trp Ser Asp Arg Lys Ser Phe Lys Ile Leu Leu Gly Val
           115                 120                 125
   tgg ctc cat tac aac aat aag tac ctc gag ttg ttg gat act gtg ttc     432
   Trp Leu His Tyr Asn Asn Lys Tyr Leu Glu Leu Leu Asp Thr Val Phe
       130                 135                 140
   atg gtg gct agg aaa aag acc aag cag ctc tct ttc ttg cat gtg tac     480
   Met Val Ala Arg Lys Lys Thr Lys Gln Leu Ser Phe Leu His Val Tyr
   145                 150                 155                 160
   cat cat gct ttg ttg att tgg gct tgg tgg ctt gtt tgt cat ctc atg     528
   His His Ala Leu Leu Ile Trp Ala Trp Trp Leu Val Cys His Leu Met
                   165                 170                 175
   gct acc aac gat tgc atc gat gct tat ttc gga gct gct tgc aac tct     576
   Ala Thr Asn Asp Cys Ile Asp Ala Tyr Phe Gly Ala Ala Cys Asn Ser
               180                 185                 190
   ttc atc cac atc gtg atg tac tcc tac tac ctc atg tct gct ttg gga     624
   Phe Ile His Ile Val Met Tyr Ser Tyr Tyr Leu Met Ser Ala Leu Gly
           195                 200                 205
   att aga tgc cct tgg aag aga tat atc acc cag gct cag atg ttg caa     672
   Ile Arg Cys Pro Trp Lys Arg Tyr Ile Thr Gln Ala Gln Met Leu Gln
       210                 215                 220
   ttc gtg atc gtg ttc gct cat gct gtt ttc gtg ctc aga caa aag cac     720
   Phe Val Ile Val Phe Ala His Ala Val Phe Val Leu Arg Gln Lys His
   225                 230                 235                 240
   tgc cct gtt act ttg cct tgg gca caa atg ttc gtg atg aca aat atg      768
   Cys Pro Val Thr Leu Pro Trp Ala Gln Met Phe Val Met Thr Asn Met
                   245                 250                 255
   ttg gtg ctc ttc gga aac ttc tac ctc aag gct tac tct aac aag tct      816
   Leu Val Leu Phe Gly Asn Phe Tyr Leu Lys Ala Tyr Ser Asn Lys Ser
               260                 265                 270
   agg gga gat gga gct tct tct gtt aag cct gct gag act act aga gca      864
   Arg Gly Asp Gly Ala Ser Ser Val Lys Pro Ala Glu Thr Thr Arg Ala
           275                 280                 285
   cct tct gtg aga aga acc agg tcc agg aag atc gat tga                  903
   Pro Ser Val Arg Arg Thr Arg Ser Arg Lys Ile Asp
       290                 295                 300
<210> 12
   <211> 300
   <212> PRT
   <213> Ostreococcus tauri
<400> 12
   Met Ser Ala Ser Gly Ala Leu Leu Pro Ala Ile Ala Phe Ala Ala Tyr
   1               5                   10                  15
   Ala Tyr Ala Thr Tyr Ala Tyr Ala Phe Glu Trp Ser His Ala Asn Gly
               20                  25                  30
   Ile Asp Asn Val Asp Ala Arg Glu Trp Ile Gly Ala Leu Ser Leu Arg
           35                  40                  45
   Leu Pro Ala Ile Ala Thr Thr Met Tyr Leu Leu Phe Cys Leu Val Gly
       50                  55                  60
   Pro Arg Leu Met Ala Lys Arg Glu Ala Phe Asp Pro Lys Gly Phe Met
   65                  70                  75                  80
   Leu Ala Tyr Asn Ala Tyr Gln Thr Ala Phe Asn Val Val Val Leu Gly
                   85                  90                  95
   Met Phe Ala Arg Glu Ile Ser Gly Leu Gly Gln Pro Val Trp Gly Ser
               100                 105                 110
   Thr Met Pro Trp Ser Asp Arg Lys Ser Phe Lys Ile Leu Leu Gly Val
           115                 120                 125
   Trp Leu His Tyr Asn Asn Lys Tyr Leu Glu Leu Leu Asp Thr Val Phe
       130                 135                 140
   Met Val Ala Arg Lys Lys Thr Lys Gln Leu Ser Phe Leu His Val Tyr
   145                 150                 155                 160
   His His Ala Leu Leu Ile Trp Ala Trp Trp Leu Val Cys His Leu Met
                   165                 170                 175
   Ala Thr Asn Asp Cys Ile Asp Ala Tyr Phe Gly Ala Ala Cys Asn Ser
               180                 185                 190
   Phe Ile His Ile Val Met Tyr Ser Tyr Tyr Leu Met Ser Ala Leu Gly
           195                 200                 205
   Ile Arg Cys Pro Trp Lys Arg Tyr Ile Thr Gln Ala Gln Met Leu Gln
       210                 215                 220
   Phe Val Ile Val Phe Ala His Ala Val Phe Val Leu Arg Gln Lys His
   225                 230                 235                 240
   Cys Pro Val Thr Leu Pro Trp Ala Gln Met Phe Val Met Thr Asn Met
                   245                 250                 255
   Leu Val Leu Phe Gly Asn Phe Tyr Leu Lys Ala Tyr Ser Asn Lys Ser
               260                 265                 270
   Arg Gly Asp Gly Ala Ser Ser Val Lys Pro Ala Glu Thr Thr Arg Ala
           275                 280                 285
   Pro Ser Val Arg Arg Thr Arg Ser Arg Lys Ile Asp
       290                 295                 300
<210> 13
   <211> 903
   <212> DNA
   <213> Ostreococcus tauri
<220>
   <221> CDS
   <222> (1)..(903)
   <223> Delta-5-Elongase
<400> 13
   atg agc gcc tcc ggt gcg ctg ctg ccc gcg atc gcg ttc gcc gcg tac       48
   Met Ser Ala Ser Gly Ala Leu Leu Pro Ala Ile Ala Phe Ala Ala Tyr
   1               5                   10                  15
   gcg tac gcg acg tac gcc tac gcc ttt gag tgg tcg cac gcg aat ggc       96
   Ala Tyr Ala Thr Tyr Ala Tyr Ala Phe Glu Trp Ser His Ala Asn Gly
               20                  25                  30
   atc gac aac gtc gac gcg cgc gag tgg atc ggt gcg ctg tcg ttg agg      144
   Ile Asp Asn Val Asp Ala Arg Glu Trp Ile Gly Ala Leu Ser Leu Arg
           35                  40                  45
   ctc ccg gcg atc gcg acg acg atg tac ctg ttg ttc tgc ctg gtc gga      192
   Leu Pro Ala Ile Ala Thr Thr Met Tyr Leu Leu Phe Cys Leu Val Gly
       50                  55                  60
   ccg agg ttg atg gcg aag cgc gag gcg ttc gac ccg aag ggg ttc atg      240
   Pro Arg Leu Met Ala Lys Arg Glu Ala Phe Asp Pro Lys Gly Phe Met
   65                  70                  75                  80
   ctg gcg tac aat gcg tat cag acg gcg ttc aac gtc gtc gtg ctc ggg      288
   Leu Ala Tyr Asn Ala Tyr Gln Thr Ala Phe Asn Val Val Val Leu Gly
                   85                  90                  95
   atg ttc gcg cga gag atc tcg ggg ctg ggg cag ccc gtg tgg ggg tca      336
   Met Phe Ala Arg Glu Ile Ser Gly Leu Gly Gln Pro Val Trp Gly Ser
               100                 105                 110
   acc atg ccg tgg agc gat aga aaa tcg ttt aag atc ctc ctc ggg gtg      384
   Thr Met Pro Trp Ser Asp Arg Lys Ser Phe Lys Ile Leu Leu Gly Val
           115                 120                 125
   tgg ttg cac tac aac aac aaa tat ttg gag cta ttg gac act gtg ttc      432
   Trp Leu His Tyr Asn Asn Lys Tyr Leu Glu Leu Leu Asp Thr Val Phe
       130                 135                 140
   atg gtt gcg cgc aag aag acg aag cag ttg agc ttc ttg cac gtt tat      480
   Met Val Ala Arg Lys Lys Thr Lys Gln Leu Ser Phe Leu His Val Tyr
   145                 150                 155                 160
   cat cac gcc ctg ttg atc tgg gcg tgg tgg ttg gtg tgt cac ttg atg      528
   His His Ala Leu Leu Ile Trp Ala Trp Trp Leu Val Cys His Leu Met
                   165                 170                 175
   gcc acg aac gat tgt atc gat gcc tac ttc ggc gcg gcg tgc aac tcg      576
   Ala Thr Asn Asp Cys Ile Asp Ala Tyr Phe Gly Ala Ala Cys Asn Ser
               180                 185                 190
   ttc att cac atc gtg atg tac tcg tat tat ctc atg tcg gcg ctc ggc      624
   Phe Ile His Ile Val Met Tyr Ser Tyr Tyr Leu Met Ser Ala Leu Gly
           195                 200                 205
   att cga tgc ccg tgg aag cga tac atc acc cag gct caa atg ctc caa      672
   Ile Arg Cys Pro Trp Lys Arg Tyr Ile Thr Gln Ala Gln Met Leu Gln
       210                 215                 220
   ttc gtc att gtc ttc gcg cac gcc gtg ttc gtg ctg cgt cag aag cac      720
   Phe Val Ile Val Phe Ala His Ala Val Phe Val Leu Arg Gln Lys His
   225                 230                 235                 240
   tgc ccg gtc acc ctt cct tgg gcg caa atg ttc gtc atg acg aac atg      768
   Cys Pro Val Thr Leu Pro Trp Ala Gln Met Phe Val Met Thr Asn Met
                   245                 250                 255
   ctc gtg ctc ttc ggg aac ttc tac ctc aag gcg tac tcg aac aag tcg        816
   Leu Val Leu Phe Gly Asn Phe Tyr Leu Lys Ala Tyr Ser Asn Lys Ser
               260                 265                 270
   cgc ggc gac ggc gcg agt tcc gtg aaa cca gcc gag acc acg cgc gcg        864
   Arg Gly Asp Gly Ala Ser Ser Val Lys Pro Ala Glu Thr Thr Arg Ala
           275                 280                 285
   ccc agc gtg cga cgc acg cga tct cga aaa att gac taa                    903
   Pro Ser Val Arg Arg Thr Arg Ser Arg Lys Ile Asp
       290                 295                 300
<210> 14
   <211> 300
   <212> PRT
   <213> Ostreococcus tauri
<400> 14
   Met Ser Ala Ser Gly Ala Leu Leu Pro Ala Ile Ala Phe Ala Ala Tyr
   1               5                   10                  15
   Ala Tyr Ala Thr Tyr Ala Tyr Ala Phe Glu Trp Ser His Ala Asn Gly
               20                  25                  30
   Ile Asp Asn Val Asp Ala Arg Glu Trp Ile Gly Ala Leu Ser Leu Arg
           35                  40                  45
   Leu Pro Ala Ile Ala Thr Thr Met Tyr Leu Leu Phe Cys Leu Val Gly
       50                  55                  60
   Pro Arg Leu Met Ala Lys Arg Glu Ala Phe Asp Pro Lys Gly Phe Met
   65                  70                  75                  80
   Leu Ala Tyr Asn Ala Tyr Gln Thr Ala Phe Asn Val Val Val Leu Gly
                   85                  90                  95
   Met Phe Ala Arg Glu Ile Ser Gly Leu Gly Gln Pro Val Trp Gly Ser
               100                 105                 110
   Thr Met Pro Trp Ser Asp Arg Lys Ser Phe Lys Ile Leu Leu Gly Val
           115                 120                 125
   Trp Leu His Tyr Asn Asn Lys Tyr Leu Glu Leu Leu Asp Thr Val Phe
       130                 135                 140
   Met Val Ala Arg Lys Lys Thr Lys Gln Leu Ser Phe Leu His Val Tyr
   145                 150                 155                 160
   His His Ala Leu Leu Ile Trp Ala Trp Trp Leu Val Cys His Leu Met
                   165                 170                 175
   Ala Thr Asn Asp Cys Ile Asp Ala Tyr Phe Gly Ala Ala Cys Asn Ser
               180                 185                 190
   Phe Ile His Ile Val Met Tyr Ser Tyr Tyr Leu Met Ser Ala Leu Gly
           195                 200                 205
   Ile Arg Cys Pro Trp Lys Arg Tyr Ile Thr Gln Ala Gln Met Leu Gln
       210                 215                 220
   Phe Val Ile Val Phe Ala His Ala Val Phe Val Leu Arg Gln Lys His
   225                 230                 235                 240
   Cys Pro Val Thr Leu Pro Trp Ala Gln Met Phe Val Met Thr Asn Met
                   245                 250                 255
   Leu Val Leu Phe Gly Asn Phe Tyr Leu Lys Ala Tyr Ser Asn Lys Ser
               260                 265                 270
   Arg Gly Asp Gly Ala Ser Ser Val Lys Pro Ala Glu Thr Thr Arg Ala
           275                 280                 285
   Pro Ser Val Arg Arg Thr Arg Ser Arg Lys Ile Asp
       290                 295                 300
<210> 15
   <211> 1512
   <212> DNA
   <213> Thalassiosira pseudonana
<220>
   <221> CDS
   <222> (1)..(1512)
   <223> Delta-4-Desaturase
<400> 15
   atg tgc aac ggc aac ctc cca gca tcc acc gca cag ctc aag tcc acc       48
   Met Cys Asn Gly Asn Leu Pro Ala Ser Thr Ala Gln Leu Lys Ser Thr
   1               5                   10                  15
   tcg aag ccc cag cag caa cat gag cat cgc acc atc tcc aag tcc gag       96
   Ser Lys Pro Gln Gln Gln His Glu His Arg Thr Ile Ser Lys Ser Glu
               20                  25                  30
   ctc gcc caa cac aac acg ccc aaa tca gca tgg tgt gcc gtc cac tcc      144
   Leu Ala Gln His Asn Thr Pro Lys Ser Ala Trp Cys Ala Val His Ser
           35                  40                  45
   act ccc gcc acc gac cca tcc cac tcc aac aac aaa caa cac gca cac      192
   Thr Pro Ala Thr Asp Pro Ser His Ser Asn Asn Lys Gln His Ala His
       50                  55                  60
   cta gtc ctc gac att acc gac ttt gcg tcc cgc cat cca ggg gga gac      240
   Leu Val Leu Asp Ile Thr Asp Phe Ala Ser Arg His Pro Gly Gly Asp
   65                  70                  75                  80
   ctc atc ctc ctc gct tcc ggc aaa gac gcc tcg gtg ctg ttt gaa aca      288
   Leu Ile Leu Leu Ala Ser Gly Lys Asp Ala Ser Val Leu Phe Glu Thr
                   85                  90                  95
   tac cat cca cgt gga gtt ccg acg tct ctc att caa aag ctg cag att      336
   Tyr His Pro Arg Gly Val Pro Thr Ser Leu Ile Gln Lys Leu Gln Ile
               100                 105                 110
   gga gtg atg gag gag gag gcg ttt cgg gat tcg ttt tac agt tgg act      384
   Gly Val Met Glu Glu Glu Ala Phe Arg Asp Ser Phe Tyr Ser Trp Thr
           115                 120                 125
   gat tct gac ttt tat act gtg ttg aag agg agg gtt gtg gag cgg ttg      432
   Asp Ser Asp Phe Tyr Thr Val Leu Lys Arg Arg Val Val Glu Arg Leu
       130                 135                 140
   gag gag agg ggg ttg gac agg agg gga tcg aaa gag att tgg atc aag      480
   Glu Glu Arg Gly Leu Asp Arg Arg Gly Ser Lys Glu Ile Trp Ile Lys
   145                 150                 155                 160
   gct ttg ttc ttg ttg gtt gga ttt tgg tac tgt ttg tac aag atg tat      528
   Ala Leu Phe Leu Leu Val Gly Phe Trp Tyr Cys Leu Tyr Lys Met Tyr
                   165                 170                 175
   act acg tcg gat atc gat cag tac ggt att gcc att gcc tat tct att      576
   Thr Thr Ser Asp Ile Asp Gln Tyr Gly Ile Ala Ile Ala Tyr Ser Ile
               180                 185                 190
   gga atg gga acc ttt gcg gca ttc atc ggc acg tgt att caa cac gat      624
   Gly Met Gly Thr Phe Ala Ala Phe Ile Gly Thr Cys Ile Gln His Asp
           195                 200                 205
   gga aat cac ggt gca ttc gct cag aac aag tta ctc aac aag ttg gct      672
   Gly Asn His Gly Ala Phe Ala Gln Asn Lys Leu Leu Asn Lys Leu Ala
       210                 215                 220
   ggg tgg acg ttg gat atg att ggt gcg agt gcg ttt acg tgg gag ctt      720
   Gly Trp Thr Leu Asp Met Ile Gly Ala Ser Ala Phe Thr Trp Glu Leu
   225                 230                 235                 240
   cag cac atg ctg ggg cat cat cca tat acg aat gtg ttg gat ggg gtg      768
   Gln His Met Leu Gly His His Pro Tyr Thr Asn Val Leu Asp Gly Val
                   245                 250                 255
   gag gag gag agg aag gag agg ggg gag gat gtt gct ttg gaa gaa aag      816
   Glu Glu Glu Arg Lys Glu Arg Gly Glu Asp Val Ala Leu Glu Glu Lys
               260                 265                 270
   gat cag gat ttt gaa gtt gcc aca tcc gga cga tta tat cat att gat      864
   Asp Gln Asp Phe Glu Val Ala Thr Ser Gly Arg Leu Tyr His Ile Asp
           275                 280                 285
   gcc aat gta cgt tat ggt tcg gta tgg aat gtc atg agg ttt tgg gct      912
   Ala Asn Val Arg Tyr Gly Ser Val Trp Asn Val Met Arg Phe Trp Ala
       290                 295                 300
   atg aag gtc att acg atg gga tat atg atg gga tta cca atc tac ttt      960
   Met Lys Val Ile Thr Met Gly Tyr Met Met Gly Leu Pro Ile Tyr Phe
   305                 310                 315                 320
   cat gga gta ctg agg gga gtt gga ttg ttt gtt att ggg cat ttg gcg     1008
   His Gly Val Leu Arg Gly Val Gly Leu Phe Val Ile Gly His Leu Ala
                   325                 330                 335
   tgt gga gag ttg ttg gcg acg atg ttt att gtg aat cac gtc att gag     1056
   Cys Gly Glu Leu Leu Ala Thr Met Phe Ile Val Asn His Val Ile Glu
               340                 345                 350
   ggt gtg agt tat gga acg aag gat ttg gtt ggt ggt gcg agt cat gta     1104
   Gly Val Ser Tyr Gly Thr Lys Asp Leu Val Gly Gly Ala Ser His Val
           355                 360                 365
   gat gag aag aag att gtc aag cca acg act gta ttg gga gat aca cca     1152
   Asp Glu Lys Lys Ile Val Lys Pro Thr Thr Val Leu Gly Asp Thr Pro
       370                 375                 380
   atg gta aag act cgc gag gag gca ttg aaa agc aac agc aat aac aac     1200
   Met Val Lys Thr Arg Glu Glu Ala Leu Lys Ser Asn Ser Asn Asn Asn
   385                 390                 395                 400
   aag aag aag gga gag aag aac tcg gta cca tcc gtt cca ttc aac gac     1248
   Lys Lys Lys Gly Glu Lys Asn Ser Val Pro Ser Val Pro Phe Asn Asp
                   405                 410                 415
   tgg gca gca gtc caa tgc cag acc tcc gtg aat tgg tct cca ggc tca     1296
   Trp Ala Ala Val Gln Cys Gln Thr Ser Val Asn Trp Ser Pro Gly Ser
               420                 425                 430
   tgg ttc tgg aat cac ttt tct ggg gga ctc tct cat cag att gag cat     1344
   Trp Phe Trp Asn His Phe Ser Gly Gly Leu Ser His Gln Ile Glu His
           435                 440                 445
   cac ttg ttc ccc agc att tgt cat aca aac tac tgt cat atc cag gat     1392
   His Leu Phe Pro Ser Ile Cys His Thr Asn Tyr Cys His Ile Gln Asp
       450                 455                 460
   gtt gtg gag agt acg tgt gct gag tac gga gtt ccg tat cag agt gag     1440
   Val Val Glu Ser Thr Cys Ala Glu Tyr Gly Val Pro Tyr Gln Ser Glu
   465                 470                 475                 480
   agt aat ttg ttt gtt gct tat gga aag atg att agt cat ttg aag ttt     1488
   Ser Asn Leu Phe Val Ala Tyr Gly Lys Met Ile Ser His Leu Lys Phe
                   485                 490                 495
   ttg ggt aaa gcc aag tgt gag tag                                     1512
   Leu Gly Lys Ala Lys Cys Glu
               500
<210> 16
   <211> 503
   <212> PRT
   <213> Thalassiosira pseudonana
<400> 16
   Met Cys Asn Gly Asn Leu Pro Ala Ser Thr Ala Gln Leu Lys Ser Thr
   1               5                   10                  15
   Ser Lys Pro Gln Gln Gln His Glu His Arg Thr Ile Ser Lys Ser Glu
               20                  25                  30
   Leu Ala Gln His Asn Thr Pro Lys Ser Ala Trp Cys Ala Val His Ser
           35                  40                  45
   Thr Pro Ala Thr Asp Pro Ser His Ser Asn Asn Lys Gln His Ala His
       50                  55                  60
   Leu Val Leu Asp Ile Thr Asp Phe Ala Ser Arg His Pro Gly Gly Asp
   65                  70                  75                  80
   Leu Ile Leu Leu Ala Ser Gly Lys Asp Ala Ser Val Leu Phe Glu Thr
                   85                  90                  95
   Tyr His Pro Arg Gly Val Pro Thr Ser Leu Ile Gln Lys Leu Gln Ile
               100                 105                 110
   Gly Val Met Glu Glu Glu Ala Phe Arg Asp Ser Phe Tyr Ser Trp Thr
           115                 120                 125
   Asp Ser Asp Phe Tyr Thr Val Leu Lys Arg Arg Val Val Glu Arg Leu
       130                 135                 140
   Glu Glu Arg Gly Leu Asp Arg Arg Gly Ser Lys Glu Ile Trp Ile Lys
   145                 150                 155                 160
   Ala Leu Phe Leu Leu Val Gly Phe Trp Tyr Cys Leu Tyr Lys Met Tyr
                   165                 170                 175
   Thr Thr Ser Asp Ile Asp Gln Tyr Gly Ile Ala Ile Ala Tyr Ser Ile
               180                 185                 190
   Gly Met Gly Thr Phe Ala Ala Phe Ile Gly Thr Cys Ile Gln His Asp
           195                 200                 205
   Gly Asn His Gly Ala Phe Ala Gln Asn Lys Leu Leu Asn Lys Leu Ala
       210                 215                 220
   Gly Trp Thr Leu Asp Met Ile Gly Ala Ser Ala Phe Thr Trp Glu Leu
   225                 230                 235                 240
   Gln His Met Leu Gly His His Pro Tyr Thr Asn Val Leu Asp Gly Val
                   245                 250                 255
   Glu Glu Glu Arg Lys Glu Arg Gly Glu Asp Val Ala Leu Glu Glu Lys
               260                 265                 270
   Asp Gln Asp Phe Glu Val Ala Thr Ser Gly Arg Leu Tyr His Ile Asp
           275                 280                 285
   Ala Asn Val Arg Tyr Gly Ser Val Trp Asn Val Met Arg Phe Trp Ala
       290                 295                 300
   Met Lys Val Ile Thr Met Gly Tyr Met Met Gly Leu Pro Ile Tyr Phe
   305                 310                 315                 320
   His Gly Val Leu Arg Gly Val Gly Leu Phe Val Ile Gly His Leu Ala
                   325                 330                 335
   Cys Gly Glu Leu Leu Ala Thr Met Phe Ile Val Asn His Val Ile Glu
               340                 345                 350
   Gly Val Ser Tyr Gly Thr Lys Asp Leu Val Gly Gly Ala Ser His Val
           355                 360                 365
   Asp Glu Lys Lys Ile Val Lys Pro Thr Thr Val Leu Gly Asp Thr Pro
       370                 375                 380
   Met Val Lys Thr Arg Glu Glu Ala Leu Lys Ser Asn Ser Asn Asn Asn
   385                 390                 395                 400
   Lys Lys Lys Gly Glu Lys Asn Ser Val Pro Ser Val Pro Phe Asn Asp
                   405                 410                 415
   Trp Ala Ala Val Gln Cys Gln Thr Ser Val Asn Trp Ser Pro Gly Ser
               420                 425                 430
   Trp Phe Trp Asn His Phe Ser Gly Gly Leu Ser His Gln Ile Glu His
           435                 440                 445
   His Leu Phe Pro Ser Ile Cys His Thr Asn Tyr Cys His Ile Gln Asp
       450                 455                 460
   Val Val Glu Ser Thr Cys Ala Glu Tyr Gly Val Pro Tyr Gln Ser Glu
   465                 470                 475                 480
   Ser Asn Leu Phe Val Ala Tyr Gly Lys Met Ile Ser His Leu Lys Phe
                   485                 490                 495
   Leu Gly Lys Ala Lys Cys Glu
               500
<210> 17
   <211> 1611
   <212> DNA
   <213> Ostreococcus tauri
<220>
   <221> CDS
   <222> (1)..(1611)
   <223> Delta-4-Desaturase
<400> 17
   atg tac ctc gga cgc ggc cgt ctc gag agc ggg acg acg cga ggg atg       48
   Met Tyr Leu Gly Arg Gly Arg Leu Glu Ser Gly Thr Thr Arg Gly Met
   1               5                   10                  15
   atg cgg acg cac gcg cgg cga ccg tcg acg acg tcg aat ccg tgc gcg       96
   Met Arg Thr His Ala Arg Arg Pro Ser Thr Thr Ser Asn Pro Cys Ala
               20                  25                  30
   cgg tca cgc gtg cgt aag acg acg gag cga tcg ctc gcg cga gtg cga      144
   Arg Ser Arg Val Arg Lys Thr Thr Glu Arg Ser Leu Ala Arg Val Arg
           35                  40                  45
   cga tcg acg agt gag aag gga agc gcg ctc gtg ctc gag cga gag agc      192
   Arg Ser Thr Ser Glu Lys Gly Ser Ala Leu Val Leu Glu Arg Glu Ser
       50                  55                  60
   gaa cgg gag aag gag gag gga ggg aaa gcg cga gcg gag gga ttg cga      240
   Glu Arg Glu Lys Glu Glu Gly Gly Lys Ala Arg Ala Glu Gly Leu Arg
   65                  70                  75                  80
   ttc caa cgc ccg gac gtc gcc gcg ccg ggg gga gcg gat cct tgg aac      288
   Phe Gln Arg Pro Asp Val Ala Ala Pro Gly Gly Ala Asp Pro Trp Asn
                   85                  90                  95
   gac gag aag tgg aca aag acc aag tgg acg gta ttc aga gac gtc gcg      336
   Asp Glu Lys Trp Thr Lys Thr Lys Trp Thr Val Phe Arg Asp Val Ala
               100                 105                 110
   tac gat ctc gat cct ttc ttc gct cga cac ccc gga gga gac tgg ctc      384
   Tyr Asp Leu Asp Pro Phe Phe Ala Arg His Pro Gly Gly Asp Trp Leu
           115                 120                 125
   ctg aac ttg gcc gtg gga cga gac tgc acc gcg ctc atc gaa tcc tat      432
   Leu Asn Leu Ala Val Gly Arg Asp Cys Thr Ala Leu Ile Glu Ser Tyr
       130                 135                 140
   cac ttg cga cca gag gtg gcg acg gct cgt ttc aga atg ctg ccc aaa      480
   His Leu Arg Pro Glu Val Ala Thr Ala Arg Phe Arg Met Leu Pro Lys
   145                 150                 155                 160
   ctc gag gat ttt ccc gtc gag gcc gtg ccc aag tcc ccg aga ccg aac      528
   Leu Glu Asp Phe Pro Val Glu Ala Val Pro Lys Ser Pro Arg Pro Asn
                   165                 170                 175
   gat tcg ccg tta tac aac aac att cgc aac cga gtc cgc gaa gag ctc      576
   Asp Ser Pro Leu Tyr Asn Asn Ile Arg Asn Arg Val Arg Glu Glu Leu
               180                 185                 190
   ttc cca gag gag gga aag aat atg cac aga cag ggc ggc gac cac ggc      624
   Phe Pro Glu Glu Gly Lys Asn Met His Arg Gln Gly Gly Asp His Gly
           195                 200                 205
   gac ggt gac gat tct ggg ttt cgc cgc ctt ttg ctt atg ccg tgt acc      672
   Asp Gly Asp Asp Ser Gly Phe Arg Arg Leu Leu Leu Met Pro Cys Thr
       210                 215                 220
   tat tcc ctt ccg ggg gtt cct ttc cgg ctg cct cct cgg gtc tcg cgg      720
   Tyr Ser Leu Pro Gly Val Pro Phe Arg Leu Pro Pro Arg Val Ser Arg
   225                 230                 235                 240
   ggg cgt gga ttg gtc tca cga ttc agg cac tgc gcc aac cac ggc gcg      768
   Gly Arg Gly Leu Val Ser Arg Phe Arg His Cys Ala Asn His Gly Ala
                   245                 250                 255
   atg tct cct tcg ccg gcc gtt aac ggc gtc ctc ggt ttg acg aac gat      816
   Met Ser Pro Ser Pro Ala Val Asn Gly Val Leu Gly Leu Thr Asn Asp
               260                 265                 270
   ctc atc ggc ggc tcg tcc ttg atg tgg aga tat cac cac caa gtc agc      864
   Leu Ile Gly Gly Ser Ser Leu Met Trp Arg Tyr His His Gln Val Ser
           275                 280                 285
   cac cac att cat tgc aac gac aac gcc atg gat caa gac gtg tac acg      912
   His His Ile His Cys Asn Asp Asn Ala Met Asp Gln Asp Val Tyr Thr
       290                 295                 300
   gcg atg cca tta ttg cgt ttc gac gct cgc cgg ccc aag tcc tgg tac      960
   Ala Met Pro Leu Leu Arg Phe Asp Ala Arg Arg Pro Lys Ser Trp Tyr
   305                 310                 315                 320
   cat cgc ttc cag cag tgg tac atg ttt tta gcg ttc ccg ttg ttg cag     1008
   His Arg Phe Gln Gln Trp Tyr Met Phe Leu Ala Phe Pro Leu Leu Gln
                   325                 330                 335
   gtt gcc ttc caa gtc gga gac att gcc gca ctg ttc acg cgt gat acc     1056
   Val Ala Phe Gln Val Gly Asp Ile Ala Ala Leu Phe Thr Arg Asp Thr
               340                 345                 350
   gaa ggc gct aag ctt cac ggg gcg acg acg tgg gag ctt acc acg gtt     1104
   Glu Gly Ala Lys Leu His Gly Ala Thr Thr Trp Glu Leu Thr Thr Val
           355                 360                 365
   gtc ctc ggt aag att gtg cac ttc ggt ctt ttg ttg ggg ccg ttg atg     1152
   Val Leu Gly Lys Ile Val His Phe Gly Leu Leu Leu Gly Pro Leu Met
       370                 375                 380
   aac cac gcg gtg agt tct gtt ttg ctg ggg atc gtc ggt ttc atg gcg     1200
   Asn His Ala Val Ser Ser Val Leu Leu Gly Ile Val Gly Phe Met Ala
   385                 390                 395                 400
   tgc caa ggt ata gtt ctg gcg tgc acg ttt gct gtg agt cac aat gtc      1248
   Cys Gln Gly Ile Val Leu Ala Cys Thr Phe Ala Val Ser His Asn Val
                   405                 410                 415
   gcg gag gcg aag ata cct gag gac acc gga gga gaa gcc tgg gag aga      1296
   Ala Glu Ala Lys Ile Pro Glu Asp Thr Gly Gly Glu Ala Trp Glu Arg
               420                 425                 430
   gat tgg ggt gtc cag cag ttg gtg act agc gcc gac tgg ggt gga aag      1344
   Asp Trp Gly Val Gln Gln Leu Val Thr Ser Ala Asp Trp Gly Gly Lys
           435                 440                 445
   ata ggt aac ttc ttc acg ggt ggc ctc aac ttg caa gtt gag cac cac      1392
   Ile Gly Asn Phe Phe Thr Gly Gly Leu Asn Leu Gln Val Glu His His
       450                 455                 460
   ttg ttt ccg gcg att tgc ttc gtc cac tac ccg gac atc gcg aag atc      1440
   Leu Phe Pro Ala Ile Cys Phe Val His Tyr Pro Asp Ile Ala Lys Ile
   465                 470                 475                 480
   gtg aag gaa gaa gcg gcc aag ctc aac atc cct tac gcg tct tac agg      1488
   Val Lys Glu Glu Ala Ala Lys Leu Asn Ile Pro Tyr Ala Ser Tyr Arg
                   485                 490                 495
   act ctt cct ggt att ttc gtc caa ttc tgg aga ttt atg aag gac atg      1536
   Thr Leu Pro Gly Ile Phe Val Gln Phe Trp Arg Phe Met Lys Asp Met
               500                 505                 510
   ggc acg gct gag caa att ggt gaa gtt cca ttg ccg aag att ccc aac      1584
   Gly Thr Ala Glu Gln Ile Gly Glu Val Pro Leu Pro Lys Ile Pro Asn
           515                 520                 525
   ccg cag ctc gcg ccg aag ctc gct tag                                  1611
   Pro Gln Leu Ala Pro Lys Leu Ala
       530                 535
<210> 18
   <211> 536
   <212> PRT
   <213> Ostreococcus tauri
<400> 18
   Met Tyr Leu Gly Arg Gly Arg Leu Glu Ser Gly Thr Thr Arg Gly Met
   1               5                   10                  15
   Met Arg Thr His Ala Arg Arg Pro Ser Thr Thr Ser Asn Pro Cys Ala
               20                  25                  30
   Arg Ser Arg Val Arg Lys Thr Thr Glu Arg Ser Leu Ala Arg Val Arg
           35                  40                  45
   Arg Ser Thr Ser Glu Lys Gly Ser Ala Leu Val Leu Glu Arg Glu Ser
       50                  55                  60
   Glu Arg Glu Lys Glu Glu Gly Gly Lys Ala Arg Ala Glu Gly Leu Arg
   65                  70                  75                  80
   Phe Gln Arg Pro Asp Val Ala Ala Pro Gly Gly Ala Asp Pro Trp Asn
                   85                  90                  95
   Asp Glu Lys Trp Thr Lys Thr Lys Trp Thr Val Phe Arg Asp Val Ala
               100                 105                 110
   Tyr Asp Leu Asp Pro Phe Phe Ala Arg His Pro Gly Gly Asp Trp Leu
           115                 120                 125
   Leu Asn Leu Ala Val Gly Arg Asp Cys Thr Ala Leu Ile Glu Ser Tyr
       130                 135                 140
   His Leu Arg Pro Glu Val Ala Thr Ala Arg Phe Arg Met Leu Pro Lys
   145                 150                 155                 160
   Leu Glu Asp Phe Pro Val Glu Ala Val Pro Lys Ser Pro Arg Pro Asn
                   165                 170                 175
   Asp Ser Pro Leu Tyr Asn Asn Ile Arg Asn Arg Val Arg Glu Glu Leu
               180                 185                 190
   Phe Pro Glu Glu Gly Lys Asn Met His Arg Gln Gly Gly Asp His Gly
           195                 200                 205
   Asp Gly Asp Asp Ser Gly Phe Arg Arg Leu Leu Leu Met Pro Cys Thr
       210                 215                 220
   Tyr Ser Leu Pro Gly Val Pro Phe Arg Leu Pro Pro Arg Val Ser Arg
   225                 230                 235                 240
   Gly Arg Gly Leu Val Ser Arg Phe Arg His Cys Ala Asn His Gly Ala
                   245                 250                 255
   Met Ser Pro Ser Pro Ala Val Asn Gly Val Leu Gly Leu Thr Asn Asp
               260                 265                 270
   Leu Ile Gly Gly Ser Ser Leu Met Trp Arg Tyr His His Gln Val Ser
           275                 280                 285
   His His Ile His Cys Asn Asp Asn Ala Met Asp Gln Asp Val Tyr Thr
       290                 295                 300
   Ala Met Pro Leu Leu Arg Phe Asp Ala Arg Arg Pro Lys Ser Trp Tyr
   305                 310                 315                 320
   His Arg Phe Gln Gln Trp Tyr Met Phe Leu Ala Phe Pro Leu Leu Gln
                   325                 330                 335
   Val Ala Phe Gln Val Gly Asp Ile Ala Ala Leu Phe Thr Arg Asp Thr
               340                 345                 350
   Glu Gly Ala Lys Leu His Gly Ala Thr Thr Trp Glu Leu Thr Thr Val
           355                 360                 365
   Val Leu Gly Lys Ile Val His Phe Gly Leu Leu Leu Gly Pro Leu Met
       370                 375                 380
   Asn His Ala Val Ser Ser Val Leu Leu Gly Ile Val Gly Phe Met Ala
   385                 390                 395                 400
   Cys Gln Gly Ile Val Leu Ala Cys Thr Phe Ala Val Ser His Asn Val
                   405                 410                 415
   Ala Glu Ala Lys Ile Pro Glu Asp Thr Gly Gly Glu Ala Trp Glu Arg
               420                 425                 430
   Asp Trp Gly Val Gln Gln Leu Val Thr Ser Ala Asp Trp Gly Gly Lys
           435                 440                 445
   Ile Gly Asn Phe Phe Thr Gly Gly Leu Asn Leu Gln Val Glu His His
       450                 455                 460
   Leu Phe Pro Ala Ile Cys Phe Val His Tyr Pro Asp Ile Ala Lys Ile
   465                 470                 475                 480
   Val Lys Glu Glu Ala Ala Lys Leu Asn Ile Pro Tyr Ala Ser Tyr Arg
                   485                 490                 495
   Thr Leu Pro Gly Ile Phe Val Gln Phe Trp Arg Phe Met Lys Asp Met
               500                 505                 510
   Gly Thr Ala Glu Gln Ile Gly Glu Val Pro Leu Pro Lys Ile Pro Asn
           515                 520                 525
   Pro Gln Leu Ala Pro Lys Leu Ala
       530                 535
<210> 19
   <211> 1548
   <212> DNA
   <213> Thraustochytrium
<220>
   <221> CDS
   <222> (1)..(1548)
   <223> Delta-4-Desaturase
<400> 19
   atg acg gtc ggg ttt gac gaa acg gtg act atg gac acg gtc cgc aac      48
   Met Thr Val Gly Phe Asp Glu Thr Val Thr Met Asp Thr Val Arg Asn
   1               5                   10                  15
   cac aac atg ccg gac gac gcc tgg tgc gcg atc cac ggc acc gtg tac      96
   His Asn Met Pro Asp Asp Ala Trp Cys Ala Ile His Gly Thr Val Tyr
               20                  25                  30
   gac atc acc aag ttc agc aag gtg cac ccc ggc ggg gac atc atc atg     144
   Asp Ile Thr Lys Phe Ser Lys Val His Pro Gly Gly Asp Ile Ile Met
           35                  40                  45
   ctg gcc gct ggc aag gag gcc acc atc ctg ttc gag acc tac cac atc     192
   Leu Ala Ala Gly Lys Glu Ala Thr Ile Leu Phe Glu Thr Tyr His Ile
       50                  55                  60
   aag ggc gtc ccg gac gcg gtg ctg cgc aag tac aag gtc ggc aag ctc     240
   Lys Gly Val Pro Asp Ala Val Leu Arg Lys Tyr Lys Val Gly Lys Leu
   65                  70                  75                  80
   ccc cag ggc aag aag ggc gaa acg agc cac atg ccc acc ggg ctc gac     288
   Pro Gln Gly Lys Lys Gly Glu Thr Ser His Met Pro Thr Gly Leu Asp
                   85                  90                  95
   tcg gcc tcc tac tac tcg tgg gac agc gag ttt tac agg gtg ctc cgc     336
   Ser Ala Ser Tyr Tyr Ser Trp Asp Ser Glu Phe Tyr Arg Val Leu Arg
               100                 105                 110
   gag cgc gtc gcc aag aag ctg gcc gag ccc ggc ctc atg cag cgc gcg     384
   Glu Arg Val Ala Lys Lys Leu Ala Glu Pro Gly Leu Met Gln Arg Ala
           115                 120                 125
   cgc atg gag ctc tgg gcc aag gcg atc ttc ctc ctg gca ggt ttc tgg     432
   Arg Met Glu Leu Trp Ala Lys Ala Ile Phe Leu Leu Ala Gly Phe Trp
       130                 135                 140
   ggc tcc ctt tac gcc atg tgc gtg cta gac ccg cac ggc ggt gcc atg     480
   Gly Ser Leu Tyr Ala Met Cys Val Leu Asp Pro His Gly Gly Ala Met
   145                 150                 155                 160
   gta gcc gcc gtt acg ctc ggc gtg ttc gct gcc ttt gtc gga act tgc     528
   Val Ala Ala Val Thr Leu Gly Val Phe Ala Ala Phe Val Gly Thr Cys
                   165                 170                 175
   atc cag cac gac ggc agc cac ggc gcc ttc tcc aag tcg cga ttc atg     576
   Ile Gln His Asp Gly Ser His Gly Ala Phe Ser Lys Ser Arg Phe Met
               180                 185                 190
   aac aag gcg gcg ggc tgg acc ctc gac atg atc ggc gcg agt gcg atg      624
   Asn Lys Ala Ala Gly Trp Thr Leu Asp Met Ile Gly Ala Ser Ala Met
           195                 200                 205
   acc tgg gag atg cag cac gtt ctt ggc cac cac ccg tac acc aac ctc      672
   Thr Trp Glu Met Gln His Val Leu Gly His His Pro Tyr Thr Asn Leu
       210                 215                 220
   atc gag atg gag aac ggt ttg gcc aag gtc aag ggc gcc gac gtc gac      720
   Ile Glu Met Glu Asn Gly Leu Ala Lys Val Lys Gly Ala Asp Val Asp
   225                 230                 235                 240
   ccg aag aag gtc gac cag gag agc gac ccg gac gtc ttc agt acg tac      768
   Pro Lys Lys Val Asp Gln Glu Ser Asp Pro Asp Val Phe Ser Thr Tyr
                   245                 250                 255
   ccg atg ctt cgc ctg cac ccg tgg cac cgc cag cgg ttt tac cac aag      816
   Pro Met Leu Arg Leu His Pro Trp His Arg Gln Arg Phe Tyr His Lys
               260                 265                 270
   ttc cag cac ctg tac gcc ccg ttt atc ttt ggg tct atg acg att aac      864
   Phe Gln His Leu Tyr Ala Pro Phe Ile Phe Gly Ser Met Thr Ile Asn
           275                 280                 285
   aag gtg att tcc cag gat gtc ggg gtt gtg ctg cgc aag cgc ctg ttc      912
   Lys Val Ile Ser Gln Asp Val Gly Val Val Leu Arg Lys Arg Leu Phe
       290                 295                 300
   cag atc gac gcc aac tgc cgg tat ggc agc ccc tgg tac gtg gcc cgc      960
   Gln Ile Asp Ala Asn Cys Arg Tyr Gly Ser Pro Trp Tyr Val Ala Arg
   305                 310                 315                 320
   ttc tgg atc atg aag ctc ctc acc acg ctc tac atg gtg gcg ctt ccc     1008
   Phe Trp Ile Met Lys Leu Leu Thr Thr Leu Tyr Met Val Ala Leu Pro
                   325                 330                 335
   atg tac atg cag ggg cct gct cag ggc ttg aag ctt ttc ttc atg gcc     1056
   Met Tyr Met Gln Gly Pro Ala Gln Gly Leu Lys Leu Phe Phe Met Ala
               340                 345                 350
   cac ttc acc tgc gga gag gtc ctc gcc acc atg ttt att gtc aac cac     1104
   His Phe Thr Cys Gly Glu Val Leu Ala Thr Met Phe Ile Val Asn His
           355                 360                 365
   atc atc gag ggc gtc agc tac gct tcc aag gac gcg gtc aag ggc gtc     1152
   Ile Ile Glu Gly Val Ser Tyr Ala Ser Lys Asp Ala Val Lys Gly Val
       370                 375                 380
   atg gct ccg ccg cgc act gtg cac ggt gtc acc ccg atg cag gtg acg     1200
   Met Ala Pro Pro Arg Thr Val His Gly Val Thr Pro Met Gln Val Thr
   385                 390                 395                 400
   caa aag gcg ctc agt gcg gcc gag tcg gcc aag tcg gac gcc gac aag     1248
   Gln Lys Ala Leu Ser Ala Ala Glu Ser Ala Lys Ser Asp Ala Asp Lys
                   405                 410                 415
   acg acc atg atc ccc ctc aac gac tgg gcc gct gtg cag tgc cag acc     1296
   Thr Thr Met Ile Pro Leu Asn Asp Trp Ala Ala Val Gln Cys Gln Thr
               420                 425                 430
   tct gtg aac tgg gct gtc ggg tcg tgg ttt tgg aac cac ttt tcg ggc     1344
   Ser Val Asn Trp Ala Val Gly Ser Trp Phe Trp Asn His Phe Ser Gly
           435                 440                 445
   ggc ctc aac cac cag att gag cac cac tgc ttc ccc caa aac ccc cac     1392
   Gly Leu Asn His Gln Ile Glu His His Cys Phe Pro Gln Asn Pro His
       450                 455                 460
   acg gtc aac gtc tac atc tcg ggc atc gtc aag gag acc tgc gaa gaa     1440
   Thr Val Asn Val Tyr Ile Ser Gly Ile Val Lys Glu Thr Cys Glu Glu
   465                 470                 475                 480
   tac ggc gtg ccg tac cag gct gag atc agc ctc ttc tct gcc tat ttc     1488
   Tyr Gly Val Pro Tyr Gln Ala Glu Ile Ser Leu Phe Ser Ala Tyr Phe
                   485                 490                 495
   aag atg ctg tcg cac ctc cgc acg ctc ggc aac gag gac ctc acg gcc     1536
   Lys Met Leu Ser His Leu Arg Thr Leu Gly Asn Glu Asp Leu Thr Ala
               500                 505                 510
   tgg tcc acg tga                                                     1548
   Trp Ser Thr
           515
<210> 20
   <211> 515
   <212> PRT
   <213> Thraustochytrium
<400> 20
   Met Thr Val Gly Phe Asp Glu Thr Val Thr Met Asp Thr Val Arg Asn
   1               5                   10                  15
   His Asn Met Pro Asp Asp Ala Trp Cys Ala Ile His Gly Thr Val Tyr
               20                  25                  30
   Asp Ile Thr Lys Phe Ser Lys Val His Pro Gly Gly Asp Ile Ile Met
           35                  40                  45
   Leu Ala Ala Gly Lys Glu Ala Thr Ile Leu Phe Glu Thr Tyr His Ile
       50                  55                  60
   Lys Gly Val Pro Asp Ala Val Leu Arg Lys Tyr Lys Val Gly Lys Leu
   65                  70                  75                  80
   Pro Gln Gly Lys Lys Gly Glu Thr Ser His Met Pro Thr Gly Leu Asp
                   85                  90                  95
   Ser Ala Ser Tyr Tyr Ser Trp Asp Ser Glu Phe Tyr Arg Val Leu Arg
               100                 105                 110
   Glu Arg Val Ala Lys Lys Leu Ala Glu Pro Gly Leu Met Gln Arg Ala
           115                 120                 125
   Arg Met Glu Leu Trp Ala Lys Ala Ile Phe Leu Leu Ala Gly Phe Trp
   130 135 140
   Gly Ser Leu Tyr Ala Met Cys Val Leu Asp Pro His Gly Gly Ala Met
   145 150 155 160
   Val Ala Ala Val Thr Leu Gly Val Phe Ala Ala Phe Val Gly Thr Cys
   165 170 175
   Ile Gln His Asp Gly Ser His Gly Ala Phe Ser Lys Ser Arg Phe Met
   180 185 190
   Asn Lys Ala Ala Gly Trp Thr Leu Asp Met Ile Gly Ala Ser Ala Met
   195 200 205
   Thr Trp Glu Met Gln His Val Leu Gly His His Pro Tyr Thr Asn Leu
   210 215 220
   Ile Glu Met Glu Asn Gly Leu Ala Lys Val Lys Gly Ala Asp Val Asp
   225 230 235 240
   Pro Lys Lys Val Asp Gln Glu Ser Asp Pro Asp Val Phe Ser Thr Tyr
   245 250 255
   Pro Met Leu Arg Leu His Pro Trp His Arg Gln Arg Phe Tyr His Lys
   260 265 270
   Phe Gln His Leu Tyr Ala Pro Phe Ile Phe Gly Ser Met Thr Ile Asn
   275 280 285
   Lys Val Ile Ser Gln Asp Val Gly Val Val Leu Arg Lys Arg Leu Phe
   290 295 300
   Gln Ile Asp Ala Asn Cys Arg Tyr Gly Ser Pro Trp Tyr Val Ala Arg
   305 310 315 320
   Phe Trp Ile Met Lys Leu Leu Thr Thr Leu Tyr Met Val Ala Leu Pro
   325 330 335
   Met Tyr Met Gln Gly Pro Ala Gln Gly Leu Lys Leu Phe Phe Met Ala
   340 345 350
   His Phe Thr Cys Gly Glu Val Leu Ala Thr Met Phe Ile Val Asn His
   355 360 365
   Ile Ile Glu Gly Val Ser Tyr Ala Ser Lys Asp Ala Val Lys Gly Val
   370 375 380
   Met Ala Pro Pro Arg Thr Val His Gly Val Thr Pro Met Gln Val Thr
   385                 390                 395                 400
   Gln Lys Ala Leu Ser Ala Ala Glu Ser Ala Lys Ser Asp Ala Asp Lys
                   405                 410                 415
   Thr Thr Met Ile Pro Leu Asn Asp Trp Ala Ala Val Gln Cys Gln Thr
               420                 425                 430
   Ser Val Asn Trp Ala Val Gly Ser Trp Phe Trp Asn His Phe Ser Gly
           435                 440                 445
   Gly Leu Asn His Gln Ile Glu His His Cys Phe Pro Gln Asn Pro His
       450                 455                 460
   Thr Val Asn Val Tyr Ile Ser Gly Ile Val Lys Glu Thr Cys Glu Glu
   465                 470                 475                 480
   Tyr Gly Val Pro Tyr Gln Ala Glu Ile Ser Leu Phe Ser Ala Tyr Phe
                   485                 490                 495
   Lys Met Leu Ser His Leu Arg Thr Leu Gly Asn Glu Asp Leu Thr Ala
               500                 505                 510
   Trp Ser Thr
           515
<210> 21
   <211> 1626
   <212> DNA
   <213> Euglena gracilis
<220>
   <221> CDS
   <222> (1)..(1626)
   <223> Delta-4-Desaturase
<400> 21
   atg ttg gtg ctg ttt ggc aat ttc tat gtc aag caa tac tcc caa aag      48
   Met Leu Val Leu Phe Gly Asn Phe Tyr Val Lys Gln Tyr Ser Gln Lys
   1               5                   10                  15
   aac ggc aag ccg gag aac gga gcc acc cct gag aac gga gcg aag ccg      96
   Asn Gly Lys Pro Glu Asn Gly Ala Thr Pro Glu Asn Gly Ala Lys Pro
               20                  25                  30
   caa cct tgc gag aac ggc acg gtg gaa aag cga gag aat gac acc gcc      144
   Gln Pro Cys Glu Asn Gly Thr Val Glu Lys Arg Glu Asn Asp Thr Ala
           35                  40                  45
   aac gtt cgg ccc acc cgt cca gct gga ccc ccg ccg gcc acg tac tac      192
   Asn Val Arg Pro Thr Arg Pro Ala Gly Pro Pro Pro Ala Thr Tyr Tyr
       50                  55                  60
   gac tcc ctg gca gtg tcg ggg cag ggc aag gag cgg ctg ttc acc acc      240
   Asp Ser Leu Ala Val Ser Gly Gln Gly Lys Glu Arg Leu Phe Thr Thr
   65                  70                  75                  80
   gat gag gtg agg cgg cac atc ctc ccc acc gat ggc tgg ctg acg tgc      288
   Asp Glu Val Arg Arg His Ile Leu Pro Thr Asp Gly Trp Leu Thr Cys
                   85                  90                  95
   cac gaa gga gtc tac gat gtc act gat ttc ctt gcc aag cac cct ggt      336
   His Glu Gly Val Tyr Asp Val Thr Asp Phe Leu Ala Lys His Pro Gly
               100                 105                 110
   ggc ggt gtc atc acg ctg ggc ctt gga agg gac tgc aca atc ctc atc      384
   Gly Gly Val Ile Thr Leu Gly Leu Gly Arg Asp Cys Thr Ile Leu Ile
           115                 120                 125
   gag tca tac cac cct gct ggg cgc ccg gac aag gtg atg gag aag tac      432
   Glu Ser Tyr His Pro Ala Gly Arg Pro Asp Lys Val Met Glu Lys Tyr
       130                 135                 140
   cgc att ggt acg ctg cag gac ccc aag acg ttc tat gct tgg gga gag      480
   Arg Ile Gly Thr Leu Gln Asp Pro Lys Thr Phe Tyr Ala Trp Gly Glu
   145                 150                 155                 160
   tcc gat ttc tac cct gag ttg aag cgc cgg gcc ctt gca agg ctg aag      528
   Ser Asp Phe Tyr Pro Glu Leu Lys Arg Arg Ala Leu Ala Arg Leu Lys
                   165                 170                 175
   gag gct ggt cag gcg cgg cgc ggc ggc ctt ggg gtg aag gcc ctc ctg      576
   Glu Ala Gly Gln Ala Arg Arg Gly Gly Leu Gly Val Lys Ala Leu Leu
               180                 185                 190
   gtg ctc acc ctc ttc ttc gtg tcg tgg tac atg tgg gtg gcc cac aag      624
   Val Leu Thr Leu Phe Phe Val Ser Trp Tyr Met Trp Val Ala His Lys
           195                 200                 205
   tcc ttc ctc tgg gcc gcc gtc tgg ggc ttc gcc ggc tcc cac gtc ggg      672
   Ser Phe Leu Trp Ala Ala Val Trp Gly Phe Ala Gly Ser His Val Gly
       210                 215                 220
   ctg agc atc cag cac gat ggc aac cac ggc gcg ttc agc cgc aac aca      720
   Leu Ser Ile Gln His Asp Gly Asn His Gly Ala Phe Ser Arg Asn Thr
   225                 230                 235                 240
   ctg gtg aac cgc ctg gcg ggg tgg ggc atg gac ttg atc ggc gcg tcg      768
   Leu Val Asn Arg Leu Ala Gly Trp Gly Met Asp Leu Ile Gly Ala Ser
                   245                 250                 255
   tcc acg gtg tgg gag tac cag cac gtc atc ggc cac cac cag tac acc      816
   Ser Thr Val Trp Glu Tyr Gln His Val Ile Gly His His Gln Tyr Thr
               260                 265                 270
   aac ctc gtg tcg gac acg cta ttc agt ctg cct gag aac gat ccg gac      864
   Asn Leu Val Ser Asp Thr Leu Phe Ser Leu Pro Glu Asn Asp Pro Asp
           275                 280                 285
   gtc ttc tcc agc tac ccg ctg atg cgc atg cac ccg gat acg gcg tgg      912
   Val Phe Ser Ser Tyr Pro Leu Met Arg Met His Pro Asp Thr Ala Trp
       290                 295                 300
   cag ccg cac cac cgc ttc cag cac ctg ttc gcg ttc cca ctg ttc gcc      960
   Gln Pro His His Arg Phe Gln His Leu Phe Ala Phe Pro Leu Phe Ala
   305                 310                 315                 320
   ctg atg aca atc agc aag gtg ctg acc agc gat ttc gct gtc tgc ctc     1008
   Leu Met Thr Ile Ser Lys Val Leu Thr Ser Asp Phe Ala Val Cys Leu
                   325                 330                 335
   agc atg aag aag ggg tcc atc gac tgc tcc tcc agg ctc gtc cca ctg     1056
   Ser Met Lys Lys Gly Ser Ile Asp Cys Ser Ser Arg Leu Val Pro Leu
               340                 345                 350
   gag ggg cag ctg ctg ttc tgg ggg gcc aag ctg gcg aac ttc ctg ttg     1104
   Glu Gly Gln Leu Leu Phe Trp Gly Ala Lys Leu Ala Asn Phe Leu Leu
           355                 360                 365
   cag att gtg ttg cca tgc tac ctc cac ggg aca gct atg ggc ctg gcc     1152
   Gln Ile Val Leu Pro Cys Tyr Leu His Gly Thr Ala Met Gly Leu Ala
       370                 375                 380
   ctc ttc tct gtt gct cac ctt gtg tcg ggg gag tac ctc gcg atc tgc     1200
   Leu Phe Ser Val Ala His Leu Val Ser Gly Glu Tyr Leu Ala Ile Cys
   385                 390                 395                 400
   ttc atc atc aac cac atc agc gag tct tgt gag ttt atg aat aca agc     1248
   Phe Ile Ile Asn His Ile Ser Glu Ser Cys Glu Phe Met Asn Thr Ser
                   405                 410                 415
   ttt caa acc gcc gcc cgg agg aca gag atg ctt cag gca gca cat cag     1296
   Phe Gln Thr Ala Ala Arg Arg Thr Glu Met Leu Gln Ala Ala His Gln
               420                 425                 430
   gca gcg gag gcc aag aag gtg aag ccc acc cct cca ccg aac gat tgg     1344
   Ala Ala Glu Ala Lys Lys Val Lys Pro Thr Pro Pro Pro Asn Asp Trp
           435                 440                 445
   gct gtg aca cag gtc caa tgc tgc gtg aat tgg aga tca ggt ggc gtg     1392
   Ala Val Thr Gln Val Gln Cys Cys Val Asn Trp Arg Ser Gly Gly Val
       450                 455                 460
   ttg gcc aat cac ctc tct gga ggc ttg aac cac cag atc gag cat cat     1440
   Leu Ala Asn His Leu Ser Gly Gly Leu Asn His Gln Ile Glu His His
   465                 470                 475                 480
   ctg ttc ccc agc atc tcg cat gcc aac tac ccc acc atc gcc cct gtt     1488
   Leu Phe Pro Ser Ile Ser His Ala Asn Tyr Pro Thr Ile Ala Pro Val
                   485                 490                 495
   gtg aag gag gtg tgc gag gag tac ggg ttg ccg tac aag aat tac gtc     1536
   Val Lys Glu Val Cys Glu Glu Tyr Gly Leu Pro Tyr Lys Asn Tyr Val
               500                 505                 510
   acg ttc tgg gat gca gtc tgt ggc atg gtt cag cac ctc cgg ttg atg     1584
   Thr Phe Trp Asp Ala Val Cys Gly Met Val Gln His Leu Arg Leu Met
       515                 520                 525
   ggt gct cca ccg gtg cca acg aac ggg gac aaa aag tca taa          1626
   Gly Ala Pro Pro Val Pro Thr Asn Gly Asp Lys Lys Ser
       530                 535                 540
<210> 22
   <211> 541
   <212> PRT
   <213> Euglena gracilis
<400> 22
   Met Leu Val Leu Phe Gly Asn Phe Tyr Val Lys Gln Tyr Ser Gln Lys
   1               5                   10                  15
   Asn Gly Lys Pro Glu Asn Gly Ala Thr Pro Glu Asn Gly Ala Lys Pro
               20                  25                  30
   Gln Pro Cys Glu Asn Gly Thr Val Glu Lys Arg Glu Asn Asp Thr Ala
           35                  40                  45
   Asn Val Arg Pro Thr Arg Pro Ala Gly Pro Pro Pro Ala Thr Tyr Tyr
       50                  55                  60
   Asp Ser Leu Ala Val Ser Gly Gln Gly Lys Glu Arg Leu Phe Thr Thr
   65                  70                  75                  80
   Asp Glu Val Arg Arg His Ile Leu Pro Thr Asp Gly Trp Leu Thr Cys
                   85                  90                  95
   His Glu Gly Val Tyr Asp Val Thr Asp Phe Leu Ala Lys His Pro Gly
               100                 105                 110
   Gly Gly Val Ile Thr Leu Gly Leu Gly Arg Asp Cys Thr Ile Leu Ile
           115                 120                 125
   Glu Ser Tyr His Pro Ala Gly Arg Pro Asp Lys Val Met Glu Lys Tyr
       130                 135                 140
   Arg Ile Gly Thr Leu Gln Asp Pro Lys Thr Phe Tyr Ala Trp Gly Glu
   145                 150                 155                 160
   Ser Asp Phe Tyr Pro Glu Leu Lys Arg Arg Ala Leu Ala Arg Leu Lys
                   165                 170                 175
   Glu Ala Gly Gln Ala Arg Arg Gly Gly Leu Gly Val Lys Ala Leu Leu
   180 185 190
   Val Leu Thr Leu Phe Phe Val Ser Trp Tyr Met Trp Val Ala His Lys
   195 200 205
   Ser Phe Leu Trp Ala Ala Val Trp Gly Phe Ala Gly Ser His Val Gly
   210 215 220
   Leu Ser Ile Gln His Asp Gly Asn His Gly Ala Phe Ser Arg Asn Thr
   225 230 235 240
   Leu Val Asn Arg Leu Ala Gly Trp Gly Met Asp Leu Ile Gly Ala Ser
   245 250 255
   Ser Thr Val Trp Glu Tyr Gln His Val Ile Gly His His Gln Tyr Thr
   260 265 270
   Asn Leu Val Ser Asp Thr Leu Phe Ser Leu Pro Glu Asn Asp Pro Asp
   275 280 285
   Val Phe Ser Ser Tyr Pro Leu Met Arg Met His Pro Asp Thr Ala Trp
   290 295 300
   Gln Pro His His Arg Phe Gln His Leu Phe Ala Phe Pro Leu Phe Ala
   305 310 315 320
   Leu Met Thr Ile Ser Lys Val Leu Thr Ser Asp Phe Ala Val Cys Leu
   325 330 335
   Ser Met Lys Lys Gly Ser Ile Asp Cys Ser Ser Arg Leu Val Pro Leu
   340 345 350
   Glu Gly Gln Leu Leu Phe Trp Gly Ala Lys Leu Ala Asn Phe Leu Leu
   355 360 365
   Gln Ile Val Leu Pro Cys Tyr Leu His Gly Thr Ala Met Gly Leu Ala
   370 375 380
   Leu Phe Ser Val Ala His Leu Val Ser Gly Glu Tyr Leu Ala Ile Cys
   385 390 395 400
   Phe Ile Ile Asn His Ile Ser Glu Ser Cys Glu Phe Met Asn Thr Ser
   405 410 415
   Phe Gln Thr Ala Ala Arg Arg Thr Glu Met Leu Gln Ala Ala His Gln
   420 425 430
   Ala Ala Glu Ala Lys Lys Val Lys Pro Thr Pro Pro Pro Asn Asp Trp
           435                 440                 445
   Ala Val Thr Gln Val Gln Cys Cys Val Asn Trp Arg Ser Gly Gly Val
       450                 455                 460
   Leu Ala Asn His Leu Ser Gly Gly Leu Asn His Gln Ile Glu His His
   465                 470                 475                 480
   Leu Phe Pro Ser Ile Ser His Ala Asn Tyr Pro Thr Ile Ala Pro Val
                   485                 490                 495
   Val Lys Glu Val Cys Glu Glu Tyr Gly Leu Pro Tyr Lys Asn Tyr Val
               500                 505                 510
   Thr Phe Trp Asp Ala Val Cys Gly Met Val Gln His Leu Arg Leu Met
           515                 520                 525
   Gly Ala Pro Pro Val Pro Thr Asn Gly Asp Lys Lys Ser
       530                 535                 540
<210> 23
   <211> 1092
   <212> DNA
   <213> Phytophthora sojae
<220>
   <221> CDS
   <222> (1)..(1092)
   <223> Omega-3-Desaturase
<400> 23
   atg gcg tcc aag cag gag cag ccg tac cag ttc ccg acg ctg acg gag      48
   Met Ala Ser Lys Gln Glu Gln Pro Tyr Gln Phe Pro Thr Leu Thr Glu
   1               5                   10                  15
   atc aag cgc tcg ctg ccc agc gag tgt ttc gag gcg tcc gtg ccg ctc      96
   Ile Lys Arg Ser Leu Pro Ser Glu Cys Phe Glu Ala Ser Val Pro Leu
               20                  25                  30
   tcg ctc tac tac acg gtg cgc tgc ctg gtg atc gcc gtg tcg ctg gcc     144
   Ser Leu Tyr Tyr Thr Val Arg Cys Leu Val Ile Ala Val Ser Leu Ala
           35                  40                  45
   ttc ggg ctc cac cac gcg cgc tcg ctg ccc gtg gtc gag ggc ctc tgg     192
   Phe Gly Leu His His Ala Arg Ser Leu Pro Val Val Glu Gly Leu Trp
       50                  55                  60
   gcg ctg gac gcc gcg ctc tgc acg ggc tac gtg ctg ctg cag ggc atc      240
   Ala Leu Asp Ala Ala Leu Cys Thr Gly Tyr Val Leu Leu Gln Gly Ile
   65                  70                  75                  80
   gtg ttc tgg ggc ttc ttc acc gtg ggc cat gac gcc ggc cac ggc gcc      288
   Val Phe Trp Gly Phe Phe Thr Val Gly His Asp Ala Gly His Gly Ala
                   85                  90                  95
   ttc tcg cgc tac cac ctg ctc aac ttc gtg atc ggc acc ttc atc cac      336
   Phe Ser Arg Tyr His Leu Leu Asn Phe Val Ile Gly Thr Phe Ile His
               100                 105                 110
   tcg ctc atc ctg acg ccc ttc gag tcg tgg aag ctc acg cac cgc cac      384
   Ser Leu Ile Leu Thr Pro Phe Glu Ser Trp Lys Leu Thr His Arg His
           115                 120                 125
   cac cac aag aac acg ggc aac atc gac cgc gac gag atc ttc tac ccg      432
   His His Lys Asn Thr Gly Asn Ile Asp Arg Asp Glu Ile Phe Tyr Pro
       130                 135                 140
   cag cgc aag gcc gac gac cac ccg ctc tcg cgt aac ctc atc ctg gcg      480
   Gln Arg Lys Ala Asp Asp His Pro Leu Ser Arg Asn Leu Ile Leu Ala
   145                 150                 155                 160
   ctg ggc gcc gcg tgg ttc gcc tac ctg gtc gag ggc ttc ccg ccg cgc      528
   Leu Gly Ala Ala Trp Phe Ala Tyr Leu Val Glu Gly Phe Pro Pro Arg
                   165                 170                 175
   aag gtc aac cac ttc aac ccg ttc gag ccg ctg ttc gtc cgc cag gtg      576
   Lys Val Asn His Phe Asn Pro Phe Glu Pro Leu Phe Val Arg Gln Val
               180                 185                 190
   tcc gcc gtg gtc atc tcg ctg gcc gcg cac ttc ggc gtg gcc gcg ctg      624
   Ser Ala Val Val Ile Ser Leu Ala Ala His Phe Gly Val Ala Ala Leu
           195                 200                 205
   tcc atc tac ctg agc ctg cag ttc ggc ttc aag acc atg gct atc tac      672
   Ser Ile Tyr Leu Ser Leu Gln Phe Gly Phe Lys Thr Met Ala Ile Tyr
       210                 215                 220
   tac tac ggg ccc gtg ttc gtg ttc ggc agc atg ctg gtc atc acc acc      720
   Tyr Tyr Gly Pro Val Phe Val Phe Gly Ser Met Leu Val Ile Thr Thr
   225                 230                 235                 240
   ttc ctg cac cac aac gac gag gag acc ccc tgg tac gcc gac tcg gag      768
   Phe Leu His His Asn Asp Glu Glu Thr Pro Trp Tyr Ala Asp Ser Glu
                   245                 250                 255
   tgg acc tac gtc aag ggc aac ctc tcg tcg gtc gac cgc tcc tac ggc      816
   Trp Thr Tyr Val Lys Gly Asn Leu Ser Ser Val Asp Arg Ser Tyr Gly
               260                 265                 270
   gcg ctc atc gac aac ctg agc cac aac atc ggc acg cac cag atc cac      864
   Ala Leu Ile Asp Asn Leu Ser His Asn Ile Gly Thr His Gln Ile His
           275                 280                 285
   cac ctc ttc ccc atc atc ccg cac tat aag ctc aag cgc gcc acc gag      912
   His Leu Phe Pro Ile Ile Pro His Tyr Lys Leu Lys Arg Ala Thr Glu
       290                 295                 300
   gcc ttc cac cag gcg ttc ccc gag ctc gtg cgc aag agc gac gag ccc      960
   Ala Phe His Gln Ala Phe Pro Glu Leu Val Arg Lys Ser Asp Glu Pro
   305                 310                 315                 320
   atc att aag gcc ttc ttc cgc gtc ggc cgc ctc tac gcc aac tac ggc      1008
   Ile Ile Lys Ala Phe Phe Arg Val Gly Arg Leu Tyr Ala Asn Tyr Gly
                   325                 330                 335
   gtc gtg gac tcg gac gcc aag ctc ttc acg ctc aag gag gcc aag gcc      1056
   Val Val Asp Ser Asp Ala Lys Leu Phe Thr Leu Lys Glu Ala Lys Ala
               340                 345                 350
   gtg tcc gag gcg gcg acc aag act aag gcc aac tga                      1092
   Val Ser Glu Ala Ala Thr Lys Thr Lys Ala Asn
           355                 360
<210> 24
   <211> 363
   <212> PRT
   <213> Phytophthora sojae
<400> 24
   Met Ala Ser Lys Gln Glu Gln Pro Tyr Gln Phe Pro Thr Leu Thr Glu
   1               5                   10                  15
   Ile Lys Arg Ser Leu Pro Ser Glu Cys Phe Glu Ala Ser Val Pro Leu
               20                  25                  30
   Ser Leu Tyr Tyr Thr Val Arg Cys Leu Val Ile Ala Val Ser Leu Ala
           35                  40                  45
   Phe Gly Leu His His Ala Arg Ser Leu Pro Val Val Glu Gly Leu Trp
       50                  55                  60
   Ala Leu Asp Ala Ala Leu Cys Thr Gly Tyr Val Leu Leu Gln Gly Ile
   65                  70                  75                  80
   Val Phe Trp Gly Phe Phe Thr Val Gly His Asp Ala Gly His Gly Ala
                   85                  90                  95
   Phe Ser Arg Tyr His Leu Leu Asn Phe Val Ile Gly Thr Phe Ile His
               100                 105                 110
   Ser Leu Ile Leu Thr Pro Phe Glu Ser Trp Lys Leu Thr His Arg His
           115                 120                 125
   His His Lys Asn Thr Gly Asn Ile Asp Arg Asp Glu Ile Phe Tyr Pro
       130                 135                 140
   Gln Arg Lys Ala Asp Asp His Pro Leu Ser Arg Asn Leu Ile Leu Ala
   145                 150                 155                 160
   Leu Gly Ala Ala Trp Phe Ala Tyr Leu Val Glu Gly Phe Pro Pro Arg
                   165                 170                 175
   Lys Val Asn His Phe Asn Pro Phe Glu Pro Leu Phe Val Arg Gln Val
               180                 185                 190
   Ser Ala Val Val Ile Ser Leu Ala Ala His Phe Gly Val Ala Ala Leu
           195                 200                 205
   Ser Ile Tyr Leu Ser Leu Gln Phe Gly Phe Lys Thr Met Ala Ile Tyr
       210                 215                 220
   Tyr Tyr Gly Pro Val Phe Val Phe Gly Ser Met Leu Val Ile Thr Thr
   225                 230                 235                 240
   Phe Leu His His Asn Asp Glu Glu Thr Pro Trp Tyr Ala Asp Ser Glu
                   245                 250                 255
   Trp Thr Tyr Val Lys Gly Asn Leu Ser Ser Val Asp Arg Ser Tyr Gly
               260                 265                 270
   Ala Leu Ile Asp Asn Leu Ser His Asn Ile Gly Thr His Gln Ile His
           275                 280                 285
   His Leu Phe Pro Ile Ile Pro His Tyr Lys Leu Lys Arg Ala Thr Glu
       290                 295                 300
   Ala Phe His Gln Ala Phe Pro Glu Leu Val Arg Lys Ser Asp Glu Pro
   305                 310                 315                 320
   Ile Ile Lys Ala Phe Phe Arg Val Gly Arg Leu Tyr Ala Asn Tyr Gly
                   325                 330                 335
   Val Val Asp Ser Asp Ala Lys Leu Phe Thr Leu Lys Glu Ala Lys Ala
               340                 345                 350
   Val Ser Glu Ala Ala Thr Lys Thr Lys Ala Asn
           355                 360
<210> 25
   <211> 1197
   <212> DNA
   <213> Phytophthora sojae
<220>
   <221> CDS
   <222> (1)..(1197)
   <223> Delta-12-Desaturase
<400> 25
   atg gcg atc ctg aac ccg gag gcc gac tcg gcc gcc aat ctg gcc acc      48
   Met Ala Ile Leu Asn Pro Glu Ala Asp Ser Ala Ala Asn Leu Ala Thr
   1               5                   10                  15
   gac agc gag gcc aag cag cgc cag ctc gcg gag gcc ggc tac acg cac      96
   Asp Ser Glu Ala Lys Gln Arg Gln Leu Ala Glu Ala Gly Tyr Thr His
               20                  25                  30
   gtg gag ggc gcg ccg gcg cca ctg ccg ctg gag ctg ccg cac ttc tcg     144
   Val Glu Gly Ala Pro Ala Pro Leu Pro Leu Glu Leu Pro His Phe Ser
           35                  40                  45
   ctg cgc gac ctg cgc gcc gcc atc ccc aag cac tgc ttc gag cgc tcg     192
   Leu Arg Asp Leu Arg Ala Ala Ile Pro Lys His Cys Phe Glu Arg Ser
       50                  55                  60
   ttc gtc acg tcc acg tac tac atg atc aag aac gtg ctc acg tgc gcc     240
   Phe Val Thr Ser Thr Tyr Tyr Met Ile Lys Asn Val Leu Thr Cys Ala
   65                  70                  75                  80
   gcg ctc ttc tac gcg gcc acc ttc atc gac cgc gcg ggc gcc gcc gcc     288
   Ala Leu Phe Tyr Ala Ala Thr Phe Ile Asp Arg Ala Gly Ala Ala Ala
                   85                  90                  95
   tac gtg ctg tgg ccc gtg tac tgg ttc ttc cag ggc agc tac ctc acg     336
   Tyr Val Leu Trp Pro Val Tyr Trp Phe Phe Gln Gly Ser Tyr Leu Thr
               100                 105                 110
   ggc gtc tgg gtc atc gcg cac gag tgt ggc cac cag gcc tac tgc tcg     384
   Gly Val Trp Val Ile Ala His Glu Cys Gly His Gln Ala Tyr Cys Ser
           115                 120                 125
   agc gag gtc gtc aac aac ctc atc ggc ctc gta ctg cac tcg gcg ctg     432
   Ser Glu Val Val Asn Asn Leu Ile Gly Leu Val Leu His Ser Ala Leu
       130                 135                 140
   ctt gtg ccg tac cac agc tgg cgc atc tcg cac cgc aag cac cac tcc     480
   Leu Val Pro Tyr His Ser Trp Arg Ile Ser His Arg Lys His His Ser
   145                 150                 155                 160
   aac acg ggc agc tgc gag aac gac gag gtg ttc gtg ccc gtg acc cgc     528
   Asn Thr Gly Ser Cys Glu Asn Asp Glu Val Phe Val Pro Val Thr Arg
                   165                 170                 175
   tcg gtg ctc gcc agc tcc tgg aac gag acg ctc gag gac tcg ccg ctc     576
   Ser Val Leu Ala Ser Ser Trp Asn Glu Thr Leu Glu Asp Ser Pro Leu
               180                 185                 190
   tac cag ctc tac cgc atc gtg tac atg ctg gtc gtg ggc tgg atg ccc     624
   Tyr Gln Leu Tyr Arg Ile Val Tyr Met Leu Val Val Gly Trp Met Pro
           195                 200                 205
   ggc tac ctc ttc ttc aac gcc acg ggc ccg acc aag tac tgg ggc aag      672
   Gly Tyr Leu Phe Phe Asn Ala Thr Gly Pro Thr Lys Tyr Trp Gly Lys
       210                 215                 220
   tcg cgc agc cac ttc aac ccg tac tcg gcc atc tac gcc gac cgc gag      720
   Ser Arg Ser His Phe Asn Pro Tyr Ser Ala Ile Tyr Ala Asp Arg Glu
   225                 230                 235                 240
   cgc tgg atg atc gtg ctg agc gac atc ttc ctc gtg gcc atg ctg gcc      768
   Arg Trp Met Ile Val Leu Ser Asp Ile Phe Leu Val Ala Met Leu Ala
                   245                 250                 255
   gtg ctg gcc gcg ctc gtg cac acc ttc tcc ttc aac acc atg gtc aag      816
   Val Leu Ala Ala Leu Val His Thr Phe Ser Phe Asn Thr Met Val Lys
               260                 265                 270
   ttc tac gtc gtg ccc tac ttc atc gtc aac gcc tac ctc gtg ctt atc      864
   Phe Tyr Val Val Pro Tyr Phe Ile Val Asn Ala Tyr Leu Val Leu Ile
           275                 280                 285
   acg tac ctg cag cac acg gac acg tac atc ccg cac ttc cgc gag ggc      912
   Thr Tyr Leu Gln His Thr Asp Thr Tyr Ile Pro His Phe Arg Glu Gly
       290                 295                 300
   gag tgg aac tgg ctg cgc ggc gcg ctt tgc acg gtg gac cgg tcg ttc      960
   Glu Trp Asn Trp Leu Arg Gly Ala Leu Cys Thr Val Asp Arg Ser Phe
   305                 310                 315                 320
   ggc ccg ttc ctc gac tcg gtg gtg cac cgc atc gtg gac acg cac gtg     1008
   Gly Pro Phe Leu Asp Ser Val Val His Arg Ile Val Asp Thr His Val
                   325                 330                 335
   tgc cac cac atc ttc tcc aag atg ccg ttc tac cac tgc gag gag gcc     1056
   Cys His His Ile Phe Ser Lys Met Pro Phe Tyr His Cys Glu Glu Ala
               340                 345                 350
   acg aac gcc atc aag ccg ctg ctg ggc aag ttc tac ctc aag gac acg     1104
   Thr Asn Ala Ile Lys Pro Leu Leu Gly Lys Phe Tyr Leu Lys Asp Thr
           355                 360                 365
   acg ccc gtg ccc gtc gcg ctc tgg cgg tcc tac acg cac tgc aag ttc     1152
   Thr Pro Val Pro Val Ala Leu Trp Arg Ser Tyr Thr His Cys Lys Phe
       370                 375                 380
   gtc gag gac gac ggc aag gtc gtc ttc tac aag aac aag ctc taa         1197
   Val Glu Asp Asp Gly Lys Val Val Phe Tyr Lys Asn Lys Leu
   385                 390                 395
<210> 26
   <211> 398
   <212> PRT
   <213> Phytophthora sojae
<400> 26
   Met Ala Ile Leu Asn Pro Glu Ala Asp Ser Ala Ala Asn Leu Ala Thr
   1               5                   10                  15
   Asp Ser Glu Ala Lys Gln Arg Gln Leu Ala Glu Ala Gly Tyr Thr His
               20                  25                  30
   Val Glu Gly Ala Pro Ala Pro Leu Pro Leu Glu Leu Pro His Phe Ser
           35                  40                  45
   Leu Arg Asp Leu Arg Ala Ala Ile Pro Lys His Cys Phe Glu Arg Ser
       50                  55                  60
   Phe Val Thr Ser Thr Tyr Tyr Met Ile Lys Asn Val Leu Thr Cys Ala
   65                  70                  75                  80
   Ala Leu Phe Tyr Ala Ala Thr Phe Ile Asp Arg Ala Gly Ala Ala Ala
                   85                  90                  95
   Tyr Val Leu Trp Pro Val Tyr Trp Phe Phe Gln Gly Ser Tyr Leu Thr
               100                 105                 110
   Gly Val Trp Val Ile Ala His Glu Cys Gly His Gln Ala Tyr Cys Ser
           115                 120                 125
   Ser Glu Val Val Asn Asn Leu Ile Gly Leu Val Leu His Ser Ala Leu
       130                 135                 140
   Leu Val Pro Tyr His Ser Trp Arg Ile Ser His Arg Lys His His Ser
   145                 150                 155                 160
   Asn Thr Gly Ser Cys Glu Asn Asp Glu Val Phe Val Pro Val Thr Arg
                   165                 170                 175
   Ser Val Leu Ala Ser Ser Trp Asn Glu Thr Leu Glu Asp Ser Pro Leu
               180                 185                 190
   Tyr Gln Leu Tyr Arg Ile Val Tyr Met Leu Val Val Gly Trp Met Pro
           195                 200                 205
   Gly Tyr Leu Phe Phe Asn Ala Thr Gly Pro Thr Lys Tyr Trp Gly Lys
       210                 215                 220
   Ser Arg Ser His Phe Asn Pro Tyr Ser Ala Ile Tyr Ala Asp Arg Glu
   225                 230                 235                 240
   Arg Trp Met Ile Val Leu Ser Asp Ile Phe Leu Val Ala Met Leu Ala
                   245                 250                 255
   Val Leu Ala Ala Leu Val His Thr Phe Ser Phe Asn Thr Met Val Lys
               260                 265                 270
   Phe Tyr Val Val Pro Tyr Phe Ile Val Asn Ala Tyr Leu Val Leu Ile
           275                 280                 285
   Thr Tyr Leu Gln His Thr Asp Thr Tyr Ile Pro His Phe Arg Glu Gly
       290                 295                 300
   Glu Trp Asn Trp Leu Arg Gly Ala Leu Cys Thr Val Asp Arg Ser Phe
   305                 310                 315                 320
   Gly Pro Phe Leu Asp Ser Val Val His Arg Ile Val Asp Thr His Val
                   325                 330                 335
   Cys His His Ile Phe Ser Lys Met Pro Phe Tyr His Cys Glu Glu Ala
               340                 345                 350
   Thr Asn Ala Ile Lys Pro Leu Leu Gly Lys Phe Tyr Leu Lys Asp Thr
           355                 360                 365
   Thr Pro Val Pro Val Ala Leu Trp Arg Ser Tyr Thr His Cys Lys Phe
       370                 375                 380
   Val Glu Asp Asp Gly Lys Val Val Phe Tyr Lys Asn Lys Leu
   385                 390                 395
<210> 27
   <211> 24
   <212> DNA
   <213> unknown
<220>
   <223> artificial sequence
<220>
   <221> misc_feature
   <222> (1)..(24)
   <223> primer
<400> 27
   gccatggccc ccatcgagac cgac 24
<210> 28
   <211> 20
   <212> DNA
   <213> unknown
<220>
   <223> artificial sequence
<400> 28
   ttagcccatg tggacggaca 20
<210> 29
   <211> 25
   <212> DNA
   <213> unknown
<220>
   <223> artificial sequence
<220>
   <221> misc_feature
   <222> (1)..(25)
   <223> primer
<400> 29
   accatggtgg atggccccaa gacca 25
<210> 30
   <211> 25
   <212> DNA
   <213> unknown
<220>
   <223> artificial sequence
<220>
   <221> misc_feature
   <222> (1)..(25)
   <223> primer
<400> 30
   ttacatggcc gggaactcga gcagg 25
<210> 31
   <211> 22
   <212> DNA
   <213> unknown
<220>
   <223> artificial sequence
<220>
   <221> misc_feature
   <222> (1)..(22)
   <223> primer
<400> 31
   gccatggcga tcctgaaccc gg 22
<210> 32
   <211> 19
   <212> DNA
   <213> unknown
<220>
   <223> artificial sequence
<220>
   <221> misc_feature
   <222> (1)..(19)
   <223> primer
<400> 32
   tagagcttgt tcttgtaga 19
<210> 33
   <211> 23
   <212> DNA
   <213> unknown
<220>
   <223> artificial sequence
<220>
   <221> misc_feature
   <222> (1)..(23)
   <223> primer
<400> 33
   gccatggcgt ccaagcagga gca 23
<210> 34
   <211> 25
   <212> DNA
   <213> unknown
<220>
   <223> artificial sequence
<220>
   <221> misc_feature
   <222> (1)..(25)
   <223> primer
<400> 34
   tcagttggcc ttagtcttgg tcgcc 25
<210> 35
   <211> 24
   <212> DNA
   <213> unknown
<220>
   <223> artificial sequence
<220>
   <221> misc_feature
   <222> (1)..(24)
   <223> primer
<400> 35
   aagatggaga cgaccttcgc gcgc 24
<210> 36
   <211> 28
   <212> DNA
   <213> unknown
<220>
   <223> artificial sequence
<220>
   <221> misc_feature
   <222> (1)..(28)
   <223> primer
<400> 36
   ttactgcgtc ttcttggcga ccgcagcg 28
<210> 37
   <211> 23
   <212> DNA
   <213> unknown
<220>
   <223> artificial sequence
<220>
   <221> misc_feature
   <222> (1)..(23)
   <223> primer
<400> 37
   gccatggcgt cggagctgct gca 23
<210> 38
   <211> 21
   <212> DNA
   <213> unknown
<220>
   <223> artificial sequence
<220>
   <221> misc_feature
   <222> (1)..(21)
   <223> primer
<400> 38
   ttagaggttc ttcttggccg g 21
<210> 39
   <211> 22
   <212> DNA
   <213> unknown
<220>
   <223> artificial sequence
<220>
   <221> misc_feature
   <222> (1)..(22)
   <223> primer
<400> 39
   accatgtcgg ccgacctgct gc 22
<210> 40
   <211> 18
   <212> DNA
   <213> unknown
<220>
   <223> artificial sequence
<220>
   <221> misc_feature
   <222> (1)..(18)
   <223> primer
<400> 40
   ttagagcttc ttcttggc 18
<210> 41
   <211> 32
   <212> DNA
   <213> unknown
<220>
   <223> artificial sequence
<220>
   <221> misc_feature
   <222> (1)..(32)
   <223> primer
<400> 41
   gcggccgcgc catggccccc atcgagaccg ac 32
<210> 42
   <211> 28
   <212> DNA
   <213> unknown
<220>
   <223> artificial sequence
<220>
   <221> misc_feature
   <222> (1)..(28)
   <223> primer
<400> 42
   gcggccgctt agcccatgtg gacggaca 28
<210> 43
   <211> 33
   <212> DNA
   <213> unknown
<220>
   <223> artificial sequence
<220>
   <221> misc_feature
   <222> (1)..(33)
   <223> primer
<400> 43
   gcggccgcac catggtggat ggccccaaga cca 33
<210> 44
   <211> 33
   <212> DNA
   <213> unknown
<220>
   <223> artificial sequence
<220>
   <221> misc_feature
   <222> (1)..(33)
   <223> primer
<400> 44
   gcggccgctt acatggccgg gaactcgagc agg 33
<210> 45
   <211> 30
   <212> DNA
   <213> unknown
<220>
   <223> artificial sequence
<220>
   <221> misc_feature
   <222> (1)..(30)
   <223> primer
<400> 45
   gcggccgcgc catggcgatc ctgaacccgg 30
<210> 46
   <211> 27
   <212> DNA
   <213> unknown
<220>
   <223> artificial sequence
<220>
   <221> misc_feature
   <222> (1)..(27)
   <223> primer
<400> 46
   gcggccgcta gagcttgttc ttgtaga 27
<210> 47
   <211> 31
   <212> DNA
   <213> unknown
<220>
   <223> artificial sequence
<220>
   <221> misc_feature
   <222> (1)..(31)
   <223> primer
<400> 47
   gcggccgcgc catggcgtcc aagcaggagc a 31
<210> 48
   <211> 33
   <212> DNA
   <213> unknown
<220>
   <223> artificial sequence
<220>
   <221> misc_feature
   <222> (1)..(33)
   <223> primer
<400> 48
   gcggccgctc agttggcctt agtcttggtc gcc 33
<210> 49
   <211> 32
   <212> DNA
   <213> unknown
<220>
   <223> artificial sequence
<220>
   <221> misc_feature
   <222> (1)..(32)
   <223> primer
<400> 49
   gcggccgcaa gatggagacg accttcgcgc gc 32
<210> 50
   <211> 36
   <212> DNA
   <213> unknown
<220>
   <223> artificial sequence
<220>
   <221> misc_feature
   <222> (1)..(36)
   <223> primer
<400> 50
   gcggccgctt actgcgtctt cttggcgacc gcagcg 36
<210> 51
   <211> 31
   <212> DNA
   <213> unknown
<220>
   <223> artificial sequence
<220>
   <221> misc_feature
   <222> (1)..(31)
   <223> primer
<400> 51
   gcggccgcgc catggcgtcg gagctgctgc a 31
<210> 52
   <211> 29
   <212> DNA
   <213> unknown
<220>
   <223> artificial sequence
<220>
   <221> misc_feature
   <222> (1)..(29)
   <223> primer
<400> 52
   gcggccgctt agaggttctt cttggccgg 29
<210> 53
   <211> 30
   <212> DNA
   <213> unknown
<220>
   <223> artificial sequence
<220>
   <221> misc_feature
   <222> (1)..(30)
   <223> primer
<400> 53
   gcggccgcac catgtcggcc gacctgctgc 30
<210> 54
   <211> 26
   <212> DNA
   <213> unknown
<220>
   <223> artificial sequence
<220>
   <221> misc_feature
   <222> (1)..(26)
   <223> primer
<400> 54
   gcggccgctt agagcttctt cttggc 26
<210> 55
   <211> 60
   <212> DNA
   <213> unknown
<220>
   <223> artificial sequence
<220>
   <221> misc_feature
   <222> (1)..(60)
   <223> primer
<400> 55
   gtcgacccgc ggactagtgg gccctctaga cccgggggat ccggatctgc tggctatgaa 60